# FASCICULE DE BREVET EUROPEEN

(11) **EP 3 161 492 B1**
(45) Date de publication et mention de la délivrance du brevet: **04.12.2019**
(21) Numéro de dépôt: 15733814.6
(22) Date de dépôt: 09.06.2015
(51) Int. Cl.: G01N 33/68, G16B 40/00

(54) **COMBINAISON SYNERGIQUE DE BIOMARQUEURS POUR LA DÉTECTION ET L'ÉVALUATION D'UNE FIBROSE HÉPATIQUE**
SYNERGISTISCHE KOMBINATION VON BIOMARKERN ZUR DETEKTION UND BEWERTUNG EINER LEBERFIBROSE
SYNERGISTIC COMBINATION OF BIOMARKERS FOR DETECTING AND ASSESSING HEPATIC FIBROSIS

(30) Priorité: 27.06.2014 FR 1456037
(43) Date de publication de la demande: 03.05.2017
(73) Titulaire: Bio-Rad Europe GmbH, 4051 Basel (CH); Assistance Publique Hôpitaux de Paris, 75004 Paris (FR); Centre National de la Recherche Scientifique, 75794 Paris Cedex 16 (FR); Université Paris Diderot - Paris 7, 75205 Paris Cedex 13 (FR)
(72) Inventeur: LAMBERT, Nadine, F-78400 Chatou (FR); WATELET, Bénédicte, F-34980 Saint Clement de Riviere (FR); BATXELLI, Isabelle Catherine, F-30670 Aigues-Vives (FR); ASSELAH, Tarik, F-75012 Paris (FR)
(74) Mandataire: dompatent von Kreisler Selting Werner - Partnerschaft von Patent- und Rechtsanwälten mbB
(86) Numéro de dépôt international: PCT/FR2015/051518
(87) Numéro de publication internationale: WO 2015/197934

(56) Documents cités:
- WO-A1-2012/107528
- WO-A2-2007/072290
- DARIELA MICHELOUD ET AL: "536: SERUM LEVELS OF FIBROSIS PROGRESSION BIOMARK- ERS MEASURED EARLY AFTER LT ARE ASSOCIATED TO SEVERE HCV RECURRENCE", HEPATOLOGY, vol. 46, no. 4, Suppl. S1, 19 septembre 2007 (2007-09-19), - 6 novembre 2007 (2007-11-06), page 476A, XP055174561, USA ISSN: 0270-9139, DOI: 10.1002/hep.22018
- PATEL KEYUR ET AL: "Multiplex protein analysis to determine fibrosis stage and progression in patients with chronic hepatitis C.", CLINICAL GASTROENTEROLOGY AND HEPATOLOGY : THE OFFICIAL CLINICAL PRACTICE JOURNAL OF THE AMERICAN GASTROENTEROLOGICAL ASSOCIATION DEC 2014, vol. 12, no. 12, 9 mai 2014 (2014-05-09), pages 2113-20.e1, XP008175348, ISSN: 1542-7714, DOI: http://www.sciencedirect.com/science/artic le/pii/S1542356514006727
- YI-MING WU ET AL: "A simple noninvasive index to predict significant liver fibrosis in patients with advanced schistosomiasis japonica", PARASITOLOGY INTERNATIONAL, vol. 62, no. 3, 1 juin 2013 (2013-06-01), pages 283-288, XP055174747, ISSN: 1383-5769, DOI: 10.1016/j.parint.2013.02.005
- EL RAZIKY M ET AL: "450 A NOVEL PREDICTION MODEL FOR LIVER FIBROSIS IN PATIENTS WITH CHRONIC HEPATITIS C VIRUS USING FIBROSCAN AND ROUTINE LABORATORY DATA", JOURNAL OF HEPATOLOGY, vol. 58, 1 avril 2013 (2013-04-01), XP028587807, ISSN: 0168-8278, DOI: 10.1016/S0168-8278(13)60452-3
- T. ABE ET AL: "CD44 Participates in IP-10 Induction in Cells in Which Hepatitis C Virus RNA Is Replicating, through an Interaction with Toll-Like Receptor 2 and Hyaluronan", JOURNAL OF VIROLOGY, vol. 86, no. 11, 1 juin 2012 (2012-06-01), pages 6159-6170, XP055174419, ISSN: 0022-538X, DOI: 10.1128/JVI.06872-11
- TAK-MAN MAK ET AL: "Liver Fibrosis Assessment Using Transient Elastography Guided with Real-Time B-mode Ultrasound Imaging: A Feasibility Study", ULTRASOUND IN MEDICINE & BIOLOGY, vol. 39, no. 6, 1 juin 2013 (2013-06-01), pages 956-966, XP055174892, ISSN: 0301-5629, DOI: 10.1016/j.ultrasmedbio.2013.01.009

## Description

### DOMAINE DE L'INVENTION

La demande est relative aux fibroses hépatiques, plus particulièrement aux fibroses hépatiques qui peuvent être présentes chez un sujet infecté par un ou plusieurs virus de l'hépatite et/ou qui est atteint d'hépatite, plus particulièrement d'hépatite chronique.

La demande fournit des méthodes pour déterminer le stade (ou degré) de fibrose hépatique d'un tel sujet. Plus particulièrement, les méthodes de la demande permettent de déterminer si le stade (ou degré) de fibrose hépatique de ce sujet a ou non dépassé le stade de la fibrose légère.

Les méthodes de l'invention mettent en œuvre une combinaison de biomarqueurs, tels que notamment la protéine CXCL10 et l'acide hyaluronique (HA).

### ARRIÈRE-PLAN DE L'INVENTION

Diverses pathologies causent, ou conduisent à, des lésions tissulaires du foie, connues sous le nom de fibrose hépatique. Une fibrose hépatique résulte notamment d'une accumulation excessive de composés moléculaires de la matrice extracellulaire altérée dans le parenchyme hépatique.

Le stade d'atteinte tissulaire du foie, plus particulièrement la nature et l'étendue des lésions tissulaires hépatiques, est évalué par un score de fibrose hépatique, notamment par le système de score F Metavir, qui comprend 5 stades de F0 à F4 (*cf.* tableau 25 ci-dessous). La détermination du score de fibrose hépatique revêt un caractère primordial pour le praticien, puisqu'il s'agit d'un score pronostique.

En effet, c'est la base de cette détermination qui permet au praticien de parvenir à la décision d'administrer ou de ne pas administrer de traitement en vue de traiter ces lésions, ou à tout le moins en vue de pallier leurs effets. C'est également sur cette base que le praticien décide d'initier un traitement. Notamment, lorsque le score de fibrose Metavir est d'au plus F1 (fibrose légère), le praticien décide généralement de ne pas administrer de traitement, alors que, lorsque le score de fibrose Metavir est d'au moins F2, il est préconisé d'administrer un traitement, et cela quel que soit le degré d'activité nécrotico-inflammatoire. Or, les traitements susceptibles d'être administrés entraînent des effets secondaires majeurs pour le patient (traitements incluant l'interféron) ou leur coût est très élevé (nouveaux agents antiviraux).

Dans ce contexte, pouvoir déterminer de manière fiable si un patient a ou non dépassé le stade de la fibrose légère est d'une importance cruciale pour le patient.

Or, s'il est relativement aisé d'identifier le stade de la cirrhose (score de fibrose Metavir F4), par exemple par élastographie impulsionnelle (FIBROSCAN™), il est par contre beaucoup plus difficile de distinguer de manière non invasive mais pourtant cliniquement fiable :
- les stades pour lesquels la fibrose est tout au plus légère (score de fibrose Metavir F0 ou F1)
- des stades de fibrose avancée (score de fibrose Metavir F2 ou F3).

Il existe différents tests non invasifs qui peuvent être appliqués en clinique à un patient pour tenter de déterminer le stade de fibrose hépatique. Ils mettent en œuvre différents biomarqueurs.

Le biomarqueur HA est par exemple mis en œuvre dans les tests cliniques ELF™ (US 7 141 380 B2), HEPASCORE™ (Adams *et al.* 2005 ; US 2007/0225919 A1), FIBROSPECT™ (US 6 986 995 B2), FIBROMETER™ (US 8 489 335 B2) et SHASTA™ (Kelleher *et al.* 2005).

Ces tests ne mettent toutefois pas en œuvre la protéine CXCL10.

En effet, la protéine CXCL10 est essentiellement connue comme étant un biomarqueur de la réponse à la thérapie anti-VHC par interféron, et n'a que peu été décrit dans le cadre de la fibrose hépatique.

HA a la capacité d'induire l'expression de la protéine CXCL10. Ainsi, une augmentation de HA induit une augmentation de CXCL10. Les concentrations des deux molécules évoluent toutes deux dans le même sens (l'augmentation de la concentration de HA entraîne l'augmentation de la concentration en CXCL10). Il n'était donc *a priori* pas attendu que la combinaison de ces deux molécules à titre de biomarqueurs conduise à un résultat supplémentaire par rapport à celui qui serait obtenu avec seulement l'une des deux molécules. Or, la présente demande décrit l'obtention d'un effet synergique allant au-delà de la simple juxtaposition des deux molécules à titre de biomarqueurs.

En outre, l'art antérieur décrit des essais qui cherchent à associer la protéine CXCL10 à d'autres biomarqueurs pour tenter de discriminer de manière fiable les scores de fibrose Metavir F0-F1 des scores de fibrose Metavir F2-F4, et rapporte que ces essais sont infructueux (Zeremski et al. 2009, page 179, colonne de gauche, première phrase : « The combination of multiple parameters did not improve the ability to identify patients with minimal fibrosis »). L'objet de la présente demande a donc surmonté un préjugé technique.

### RÉSUMÉ DE L'INVENTION

La demande est relative à des méthodes et moyens qui permettent de déterminer le stade (ou degré) de fibrose hépatique d'un sujet, plus particulièrement de déterminer si le stade (ou degré) de fibrose hépatique dudit sujet a ou non dépassé le stade de la fibrose légère telles que définies dans les revendications.

Les inventeurs ont identifié des biomarqueurs particuliers à cet effet, tels que CXCL10 (ligand 10 à chémokine (motif CXC)) et HA (acide hyaluronique ou hyaluronan). Les inventeurs ont plus particulièrement identifié des combinaisons particulières de biomarqueurs qui, telle la combinaison de CXCL10 à HA, conduisent à un effet synergique.

En effet, les inventeurs démontrent notamment que la combinaison du marqueur CXCL10 au marqueur HA conduit à des performances qui vont au-delà de la simple juxtaposition de leurs performances individuelles respectives (performances d'AUC et/ou Taux de bonne classification et/ou sensibilité et/ou VPN et/ou spécificité et/ou VPP). Des démonstrations expérimentales sont présentées dans les exemples ci-dessous, notamment :
- en exemple 1 (*cf.* Tableau 3),
- en exemple 3 (*cf.* Tableaux 8 et 10), et
- en exemple 7 (*cf.* Tableaux 20 et 21).

Les inventeurs démontrent que l'effet synergique observé avec la combinaison de CXCL10 et HA n'est pas dépendant du fait de mettre en œuvre une fonction mROC pour la classification du sujet ; *cf.* les exemples ci-dessous, notamment :
- l'exemple 7, plus particulièrement les Tableaux 20, 21 et 23,
- l'exemple 8, plus particulièrement le Tableau 24.

Les inventeurs démontrent que les performances de la combinaison de CXCL10 à HA sont particulièrement robustes (performances d'AUC et/ou Taux de bonne classification et/ou sensibilité et/ou VPN et/ou spécificité et/ou VPP): *cf.* exemple 9 ci-dessous.

Des comparaisons avec des tests non invasifs de l'art antérieur sont par ailleurs présentées en exemples 2 et 4 ci-dessous.

Selon un mode de réalisation avantageux, la détection de HA et celle de CXCL10 sont faites en multiplex : *cf.* exemple 10 ci-dessous. Certains marqueurs de l'art antérieur, tels que A2M, ont une concentration sérique très élevée et ne peuvent donc pas être mis en œuvre en multiplex avec des marqueurs dont la concentration sérique est beaucoup plus basse (tels que HA et CXCL10).

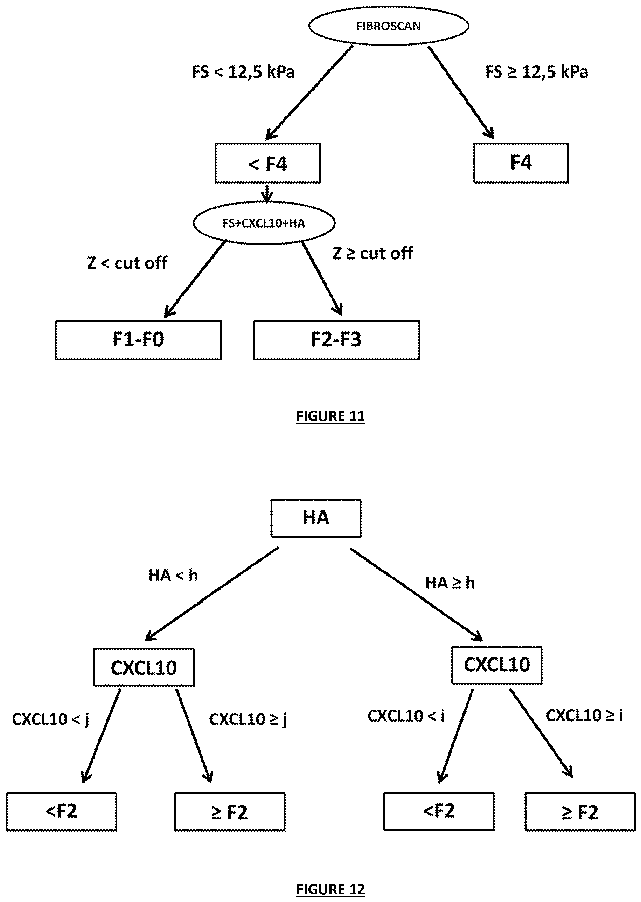

Les moyens de l'invention comprennent notamment :
- une méthode in vitro pour déterminer si le stade de fibrose hépatique d'un sujet infecté par un ou plusieurs virus de l'hépatite a ou non dépassé celui des stades de fibrose hépatique qui, selon le système de scores de fibrose Metavir, est de score F1, ladite méthode comprenant les étapes suivantes :
   i) sélectionner différents marqueurs biologiques, lesdits différents marqueurs biologiques sélectionnés consistant en :
      a) l'acide hyaluronique ainsi que la protéine CXCL10, et
      b) zéro, un, deux, trois ou quatre marqueur(s) additionnel(s) choisi(s) parmi la liste de marqueurs constituée par l'âge, l'Indice de Masse Corporelle, la charge virale et la dureté du foie,
   ii) quantifier les différents marqueurs biologiques sélectionnés à l'étape i)
      en mesurant in vitro la concentration d'acide hyaluronique et la concentration de protéine CXCL10 dans un échantillon de fluide biologique préalablement obtenu à partir dudit sujet, et
      lorsqu'un, deux, trois ou quatre marqueurs additionnel(s) est(sont) choisi(s) parmi ladite liste de l'étape i)b) ci-dessus, et lorsque ce (ou ces) marqueur(s) additionnel(s) est(sont) ou comprennent un ou plusieurs marqueur(s) parmi l'âge, l'Indice de Masse Corporelle et la dureté du foie : en collectant la valeur de quantification de ce ou chacun de ces marqueur(s) additionnel(s) qui a été préalablement déterminée pour ou sur ledit sujet,
      lorsqu'un, deux, trois ou quatre marqueurs additionnel(s) est(sont) choisi(s) parmi ladite liste de l'étape i)b) ci-dessus, et lorsque ce (ou ces) marqueur(s) additionnel(s) est(sont) ou comprennent la charge virale : en mesurant cette charge virale in vitro dans un échantillon de fluide biologique préalablement obtenu à
      partir dudit sujet, ou en collectant la valeur de cette charge virale qui a été préalablement déterminée pour ledit sujet, et
   iii) comparer les valeurs de quantification obtenues à l'étape ii) à leurs valeurs, ou à la distribution de leurs valeurs, dans des cohortes de référence préétablies selon le stade de fibrose hépatique, pour classer ledit sujet dans celle de ces cohortes de référence vis-à-vis de laquelle il a la plus forte probabilité d'appartenance, lesdites cohortes de référence étant :
      une première cohorte de référence dans laquelle le stade de fibrose hépatique des individus ne dépasse pas celui des stades de fibrose hépatique qui, selon le système de scores de fibrose Metavir, est de score F1, et
      une deuxième cohorte de référence dans laquelle le stade de fibrose hépatique des individus dépasse celui des stades de fibrose hépatique qui, selon le système de scores de fibrose Metavir, est de score F1,
      le classement dans ladite première cohorte indiquant que le stade de fibrose hépatique dudit sujet n'a pas dépassé celui des stades de fibrose hépatique qui, selon le système de scores de fibrose Metavir, est de score F1,
      le classement dans ladite deuxième cohorte indiquant que le stade de fibrose hépatique dudit sujet a dépassé celui des stades de fibrose hépatique qui, selon le système de scores de fibrose Metavir, est de score F1,
      dans laquelle ladite comparaison de l'étape iii) est faite :
         par apprentissage automatique, ou par régression logistique, ou par mROC, et
         dans laquelle ladite comparaison de l'étape iii) est faite :
            par apprentissage automatique en suivant l'arbre décisionnel de la Figure 12 avec 40 ≤ h ≤ 80, 150 ≤ i ≤ 300, et 400≤ j ≤620, ou
            par régression logistique à l'aide de la fonction LOGIT1, ladite fonction LOGIT1 étant: $LOGIT = Intercept + k \left(CXCL10\right) + l \left(HA\right) ,$ avec
               -5 ≤ Intercept ≤ -1, 0,001 ≤ k ≤ 0,010, et 0,010 ≤ l ≤ 0,050, ou
            par mROC à l'aide de la fonction Z13, ladite fonction Z13 étant $Z = a \left(CXCL10t\right) + b \left(HAt\right) + c \left(IMCt\right) + d \left(Âget\right) + e \left(CVt\right) + f \left(FSt\right)$ avec :
               a et b étant chacun, indépendamment l'un de l'autre, un nombre réel positif allant de +0,1 à +6,0 mais différent de zéro,
               c, d, e, et f étant chacun, indépendamment les uns des autres, un nombre réel allant de - 10,0 à +10,0,
               l'exposant t indiqué indiquant que la valeur à appliquer dans la fonction est la transformée Box-Cox de la valeur mesurée pour le marqueur considéré (BMQ), afin de normaliser cette valeur mesurée selon la formule suivante : BMQt = (BMQλ, - 1) / λ, et la valeur de λ pour chacun des marqueurs CXCL10 (λCXCL10), HA (λHA), IMC (λIMC) Âge (λÂge), CV (λCV) et FS (λFS) étant chacun, indépendamment les uns des autres, un nombre réel allant de -6,0 à 1,2 mais différent de zéro et dans laquelle la comparaison de l'étape iii) est faite en combinant les valeurs de quantification obtenues pour ledit sujet dans un modèle de classification préalablement construit comme suit :
                  α) pour une population d'individus qui sont de la même espèce que ledit sujet, et qui sont infectés par le ou les mêmes virus de l'hépatite que ledit sujet, déterminer le stade de fibrose hépatique de chacun desdits individus de la population, et les classer en sous-populations selon leur stade de fibrose hépatique, constituant ainsi des cohortes de référence établies selon leur stade de fibrose hépatique, lesdites cohortes de référence comprenant ou étant :
                     une première cohorte de référence dans laquelle le stade de fibrose hépatique des individus ne dépasse pas celui des stades de fibrose hépatique qui, selon le système de scores de fibrose Metavir, est de score F1, et
                     une deuxième cohorte de référence dans laquelle le stade de fibrose hépatique des individus dépasse celui des stades de fibrose hépatique qui, selon le système de scores de fibrose Metavir, est de score F1 ;
                  β) pour chacun desdits individus, quantifier les différents marqueurs biologiques sélectionnés à l'étape i), et
                  γ) faire une comparaison inter-cohorte des valeurs de quantification obtenues à l'étape β), ou de la distribution de ces valeurs, pour construire un modèle de classification, qui, à partir de valeurs de quantification desdits marqueurs biologiques sélectionnés, induit un classement dans l'une desdites cohortes de référence ;
- l'utilisation d'un article manufacturé pour effectuer cette méthode, dans laquelle ledit article manufacturé est adapté à la détection en multiplex de molécules contenues sous forme acellulaire dans un échantillon de fluide biologique, et qui comprend un support solide sur lequel sont fixés des ligands desdites molécules, dans lequel les ligands desdites molécules sont constitués par :
   une première protéine, qui est une protéine qui se lie de manière spécifique à HA et qui est un anticorps ou la protéine humaine recombinante aggrécane, et
   une deuxième protéine, qui est une protéine qui se lie de manière spécifique à la protéine CXCL10 et qui est un anticorps;
- l'utilisation d'une composition pour effectuer cette méthode, dans laquelle ladite composition est adaptée à la détection en multiplex de molécules contenues sous forme acellulaire dans un échantillon de fluide biologique, et comprend en mélange des ligands desdites molécules, dans laquelle les ligands desdites molécules sont constitués par:
   une première protéine, qui est une protéine qui se lie de manière spécifique à HA, qui est un anticorps ou la protéine humaine recombinante aggrécane, et qui porte un premier marqueur de détection, et
   une deuxième protéine, qui est une protéine qui se lie de manière spécifique à la protéine CXCL10, qui est un anticorps et qui porte un deuxième marqueur de détection,
   dans laquelle ledit premier marqueur de détection est différent dudit deuxième marqueur de détection;
- l'utilisation d'un kit pour effectuer cette méthode, dans laquelle ledit kit comprend des acides nucléiques qui se lient de manière spécifique à un ou des virus de l'hépatite, et qui comprend en outre des ligands qui se lient à des molécules contenues sous forme acellulaire dans un fluide biologique, lesdits ligands étant contenus dans le kit en préparation combinée pour une utilisation simultanée, lesdits ligands étant constitués par :
   une première protéine qui se lie de manière spécifique à HA, qui est un anticorps ou la protéine humaine recombinante aggrécane, et qui porte un premier marqueur de détection, et
   une deuxième protéine qui se lie de manière spécifique à CXCL10, qui est un anticorps et qui porte un deuxième marqueur de détection,
   dans lequel ledit deuxième marqueur de détection est différent dudit premier marqueur de détection; ainsi que
- l'utilisation d'un produit programme d'ordinateur pour effectuer cette méthode, dans laquelle ledit produit programme d'ordinatuer est destiné à être stocké dans une mémoire d'une unité de traitement, ou sur un support mémoire amovible destiné à coopérer avec un lecteur de ladite unité de traitement, caractérisé en ce qu'il comprend des instructions pour la mise en œuvre de cette méthode.

### BRÈVE DESCRIPTION DES FIGURES

La **Figure 1** présente la distribution de la concentration sérique en protéine CXCL10 selon le score de fibrose hépatique établi par Ponction Biopsie Hépatique (PBH) (score Metavir F1 ou F2) pour une population de 118 patients (*cf.* exemple 1 ci-dessous).
La **Figure 2** présente la distribution de la concentration sérique en acide hyaluronique (HA) selon le score de fibrose hépatique établi par PBH (score F1 ou F2) pour une population de 118 patients (*cf.* exemple 1 ci-dessous).

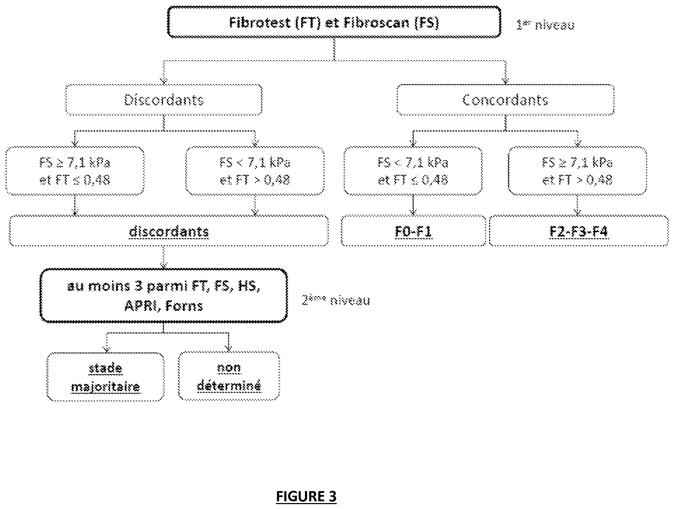

La **Figure 3** présente un modèle ou algorithme de classification qui permet de déterminer le stade de fibrose hépatique sans avoir recours à la PBH (score Metavir de fibrose hépatique < F2 ou ≥ F2). Ce modèle ou algorithme a été mis au point par modification de l'algorithme décrit par Castera *et al.* (Castera *et al.* 2010 et Castera *et al.* 2014). En sus du premier niveau d'analyse (FIBROTEST™ et FIBROSCAN™), ce modèle ou algorithme modifié comprend un deuxième niveau d'analyse, pour le traitement (sans PBH) de ceux des échantillons qui donnent des résultats discordants au premier niveau d'analyse (*cf.* exemple 3 ci-dessous).
La **Figure 4** présente la distribution de la concentration sérique en protéine CXCL10 selon le degré de fibrose hépatique (score Metavir < F2 ou ≥ F2) pour une population de 310 patients (*cf.* exemple 3 ci-dessous).
La **Figure 5** présente la distribution de la concentration sérique en acide hyaluronique (HA) selon le degré de fibrose hépatique (score Metavir < F2 ou ≥ F2) pour une population de 310 patients [*cf.* exemple 3 ci-dessous].

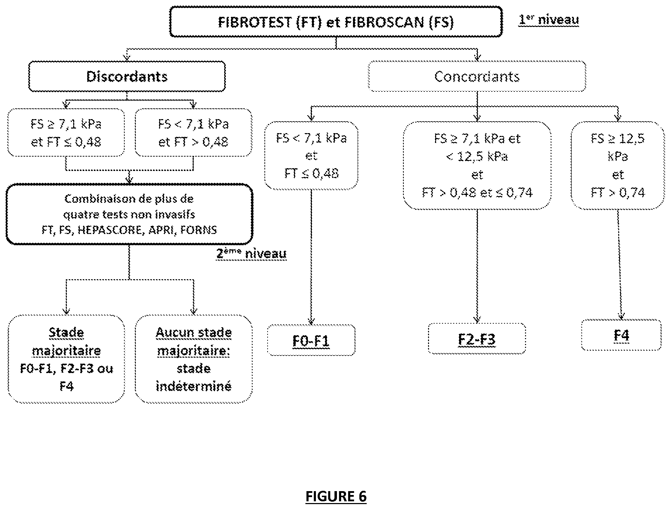

La **Figure 6** présente un modèle ou algorithme de classification qui permet de déterminer de manière fine le stade de fibrose hépatique sans avoir recours à la PBH (score Metavir de fibrose hépatique F0-F1 ou F2-F3 ou F4). Ce modèle ou algorithme a été mis au point par modification de l'algorithme décrit par Boursier *et al.* 2012. En sus du premier niveau d'analyse (FIBROTEST™ et FIBROSCAN™), ce modèle ou algorithme modifié comprend un deuxième niveau d'analyse, pour le traitement (sans PBH) de ceux des échantillons qui donnent des résultats discordants au premier niveau d'analyse (*cf.* exemple 6 ci-dessous).
La **Figure 7** présente la distribution de la concentration sérique en protéine CXCL10 selon le degré de fibrose hépatique (score Metavir F0-F1 ou F2-F3 ou F4) pour une population de 310 patients (*cf.* exemple 6 ci-dessous).
La **Figure 8** présente la distribution de la concentration sérique en acide hyaluronique (HA) selon le degré de fibrose hépatique (score Metavir F0-F1 ou F2-F3 ou F4) pour une population de 310 patients (*cf.* exemple 6 ci-dessous).
Les **Figures 9, 10** **et** **11** présentent chacune un modèle ou algorithme de classification comprenant la combinaison du marqueur HA et du marqueur CXCL10, pour la classification de patients en fonction de leur stade de fibrose hépatique (score Metavir de fibrose hépatique F0-F1 ou F2-F3).
   Ce modèle ou algorithme peut en outre comprendre une étape préalable permettant de classer les patients selon que leur score Metavir de fibrose hépatique est inférieur à F4, ou est égal à F4, par exemple par mesure de la dureté du foie, par exemple par FIBROSCAN™.

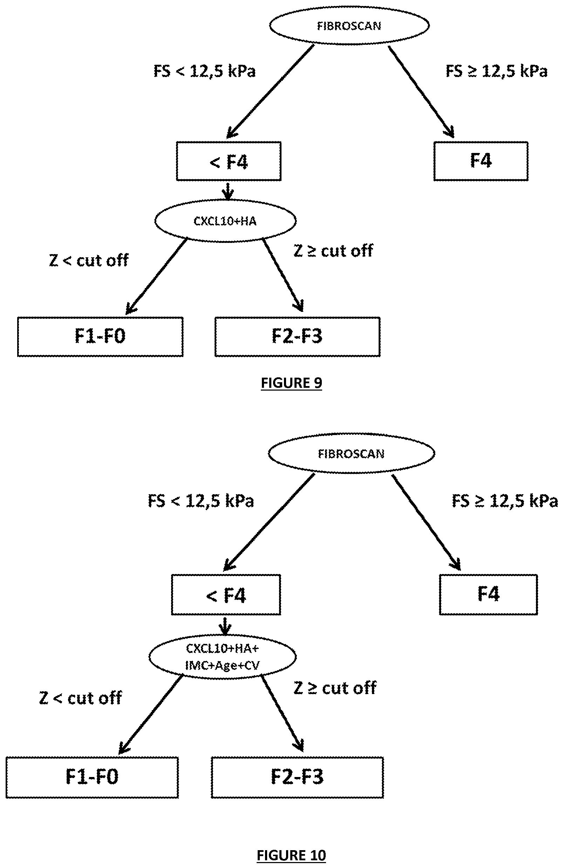

   Figure 9 : combinaison de HA et CXCL10.
   Figures 10 et 11 : combinaison de HA, CXCL10 et d'un ou plusieurs autres marqueurs additionnels.
   Figure 10 : combinaison de HA, CXCL10 et d'un ou plusieurs autres marqueurs additionnels [marqueur(s) clinique(s) ou anatomique(s) et/ou marqueur(s) virologique(s)], en l'occurrence : Indice de Masse Corporelle (IMC), âge à la date du prélèvement (Âge), Charge Virale à la date du prélèvement (CV).
   Figure 11 : combinaison de HA, CXCL10 et de la mesure de la dureté du foie, mesurée par exemple par FIBROSCAN™ (FS).
La **Figure 12** présente un modèle ou algorithme de classification qui n'est pas basé sur une fonction linéaire : ce modèle ou algorithme est basé sur un modèle de classification CART (*Classification And Regression Tree*), et comprend la combinaison du marqueur HA et du marqueur CXCL10 (*cf.* exemple 7 ci-dessous).

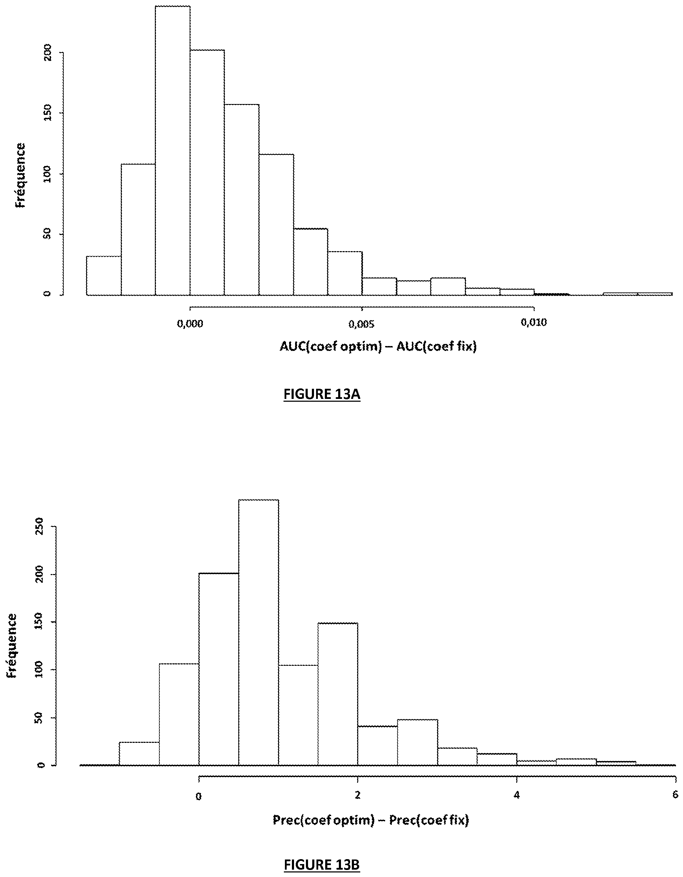

Les **Figures 13A à 16B** illustrent les performances de la combinaison HA+CXCL10 à l'aide de la méthode connue sous le nom de *Bootstrap* (*cf.* exemple 3 ci-dessous ; 1000 sous-populations de même taille tirées aléatoirement au sein d'une population de 310 patients ; tirage avec remise).
   Les valeurs d'AUC et de taux de bonne classification ont été mesurées :
   - d'une part, lorsque la règle décisionnelle mROC appliquée a pour coefficients ceux initialement établis sur la population de 310 patients (« coefficients fixes » ou « coef fix »), et
   - d'autre part, lorsque la règle décisionnelle mROC appliquée a pour coefficients ceux établis pour chacune des 1000 sous-populations (« coefficients optimisés » ou « coef optim »).
      AUC = aire sous la courbe ROC
      Taux de bonne classification = pourcentage de patients bien classés
Les **Figures 13A et 13B** présentent sous forme d'histogrammes les valeurs des différences d'AUC et de précision qui ont été mesurées entre d'une part la règle décisionnelle mROC à « coefficients fixes » (ou « coef fix ») et d'autre part la règle décisionnelle mROC à « coefficients optimisés » (« coef optim »).
   En Figures 13A et 13B, la règle décisionnelle mROC mettait en œuvre la combinaison HA+CXCL10.
La Figure 13A présente l'histogramme des différences d'AUC avec les coefficients fixes ou optimisés pour la combinaison HA+CXCL10 en *Bootstrap* (B = 1 000) [axe des abscisses : 0,000 ; 0,005 ; 0,010].
La Figure 13B présente l'histogramme des différences de taux de bonne classification (pourcentage de patients bien classés) avec les coefficients fixes ou optimisés pour la combinaison HA+CXCL10 en *Bootstrap* (B = 1 000).

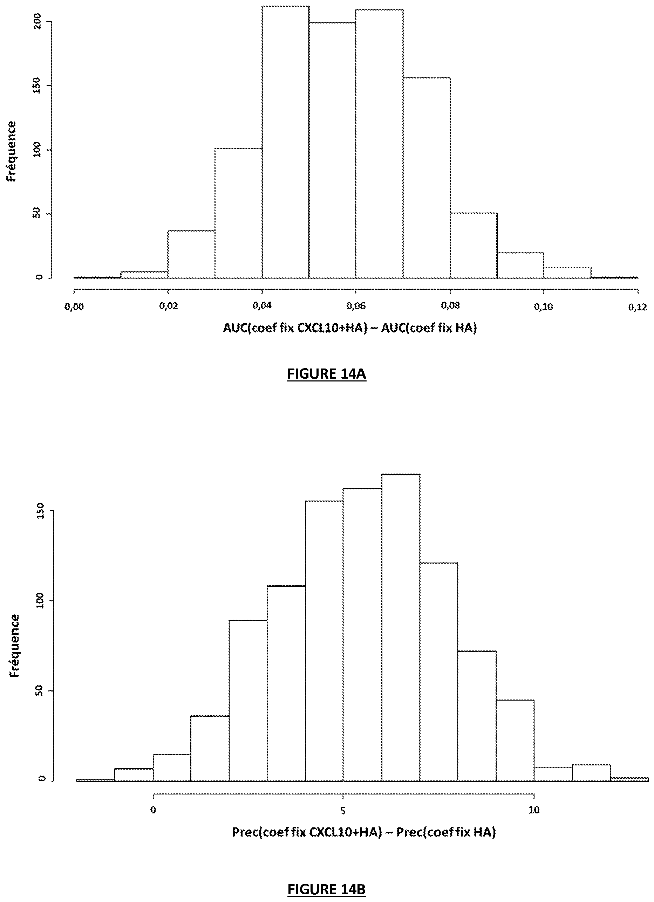

Les **Figures 14A et 14B** présentent sous forme d'histogrammes les valeurs des différences d'AUC et de taux de bonne classification qui ont été mesurées pour la règle décisionnelle mROC à « coefficients fixes » (« coef fix ») entre d'une part la combinaison HA+CXCL10 et d'autre part le marqueur HA seul.
La Figure 14A présente l'histogramme des différences d'AUC entre la combinaison HA+CXCL10 d'une part et le marqueur HA seul d'autre part, en *Bootstrap* (B = 1 000) avec les coefficients fixes (« coef fix ») [axe des abscisses : 0,02 ; 0,04 ; 0,06 ; 0,08 ; 0,10 ; 0,12].
La Figure 14B présente l'histogramme des différences de taux de bonne classification entre la combinaison HA+CXCL10 d'une part et le marqueur HA seul d'autre part, en *Bootstrap* (B = 1 000) avec les coefficients fixes (« coef fix »).

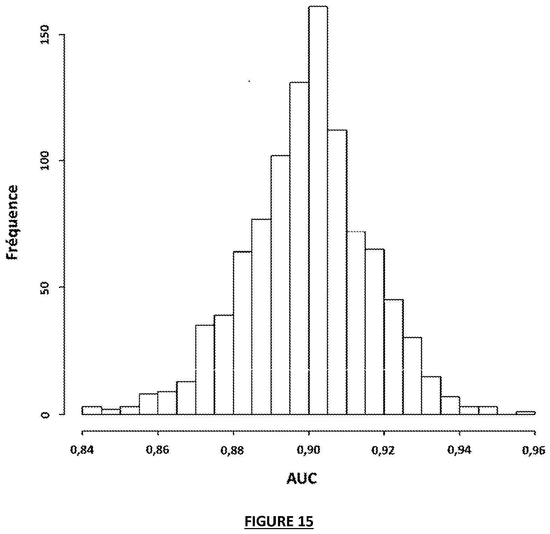

La **Figure 15** présente l'histogramme des AUC pour la combinaison HA+CXCL10, en *Bootstrap* (B = 1 000) avec les coefficients fixes (« coef fixes ») [axe des abscisses : 0,84 ; 0,86 ; 0,88 ; 0,90 ; 0,92 ; 0,94 ; 0,96] ; *cf.* exemple 9 ci-dessous.

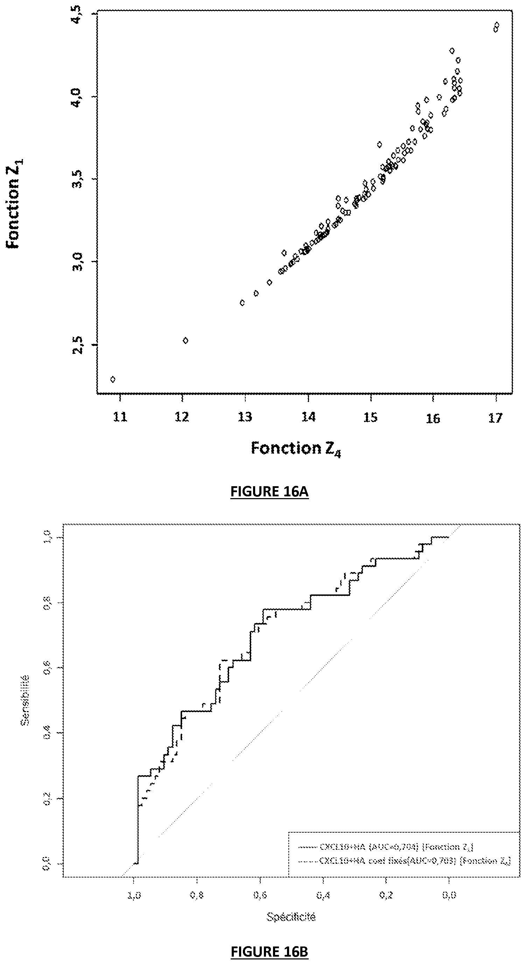

Les **Figures 16A et 16B** comparent les performances de la fonction mROC obtenue sur la population de 118 patients de l'exemple 1 (marqueurs HA+CXCL10 ; fonction Z1 ; *cf.* Tableau 2 ci-dessous) à celles de la fonction mROC obtenue sur la population de 310 patients de l'exemple 3 (marqueurs HA+CXCL10 ; fonction Z4 ; *cf.* Tableau 7 ci-dessous), lorsqu'elles sont toutes deux appliquées à la population de 118 patients; *cf.* exemple 9 ci-dessous.
Figure 16A : en ordonnée, fonction Z1 de l'exemple 1 [Z = (0,3686) x CXCL10^{t} + (0,3064) x HA^{t}, avec λCXCL10 = -0,013 et λHA = 0,099] ; en abscisse, fonction Z4 de l'exemple 3 [Z = (1,999) x CXCL10^{t} + (2,852) x HA^{t}, avec λCXCL10 = -0,116 et λHA = -0,288].
Figure 16B : en ordonnée, sensibilité ; en abscisse, spécificité ; courbes de Z1 et Z4 (les deux courbes se confondent).

### DESCRIPTION DÉTAILLÉE DE L'INVENTION

La demande est relative aux objets définis dans les revendications telles que déposées, aux objets décrits ci-dessous et aux objets illustrés en partie « exemples ».

Dans la demande, à moins que cela ne soit autrement spécifié, ou que le contexte n'en dicte autrement, tous les termes ont leur sens habituel dans le(s) domaine(s) concerné(s).

La demande est relative à une méthode pour déterminer, plus particulièrement pour déterminer avec une forte probabilité, le stade (ou degré) de fibrose du foie d'un sujet qui est infecté par un ou plusieurs virus de l'hépatite et/ou qui est atteint d'hépatite, plus particulièrement d'hépatite chronique.

Le ou les virus de l'hépatite peuvent par exemple être un ou des virus de l'hépatite C et/ou de l'hépatite B et/ou de l'hépatite D, plus particulièrement de l'hépatite C.

Ledit virus de l'hépatite C peut être de n'importe quel génotype. Par exemple, le génotype dudit virus de l'hépatite C peut être le génotype 1, 2, 3, 4, 5, 6 ou 7, plus particulièrement le génotype 1 ou 4.

Ledit sujet est un mammifère, plus particulièrement un humain.

La méthode de la demande met en œuvre la quantification de différents marqueurs, qui peuvent tous être mesurés ou déterminés de manière substantiellement non invasive (le degré d'intervention invasive sur le corps du sujet ne va pas au-delà du simple prélèvement de liquide biologique). La méthode de la demande peut être mise en œuvre *in vitro.*

Plus particulièrement, la méthode de la demande est une méthode pour déterminer, plus particulièrement pour déterminer avec une forte probabilité, si le stade (ou degré) de fibrose hépatique dudit sujet a ou non dépassé le stade de la fibrose légère.

Par « stade (ou degré) de fibrose hépatique qui n'a pas dépassé le stade de la fibrose légère », on entend un stade qui est inférieur ou égal à celui de la fibrose légère.

Par « stade (ou degré) de fibrose hépatique qui a dépassé le stade de la fibrose légère », on entend un stade qui est supérieur à celui de la fibrose légère.

Les différents stades de la fibrose hépatique sont les suivants (dans l'ordre du faible degré au plus fort degré) :
- absence de fibrose,
- fibrose portale sans septa,
- fibrose portale avec septas (c'est-à-dire avec au moins un ou plusieurs septa(s)),
- fibrose septale sans cirrhose, et
- cirrhose.

Ledit stade de fibrose légère est celui de la fibrose portale sans septa.

Lorsque le stade de fibrose hépatique dudit sujet n'a pas dépassé le stade de la fibrose légère, le foie de ce sujet est donc soit dépourvu de fibrose (absence de fibrose), soit atteint d'une fibrose portale sans septa.

Lorsque le stade de fibrose hépatique dudit sujet a dépassé le stade de la fibrose légère, le foie de ce sujet est donc atteint d'une fibrose portale avec septas, ou d'une fibrose septale sans cirrhose, ou d'une cirrhose.

Il existe différents systèmes de scores de fibrose hépatique. Le plus courant est le système de scores Metavir.

**Tableau 25 : correspondance entre les stades de fibrose et les scores de fibrose Metavir**

| Stade de fibrose | Score de fibrose Metavir |
|---|---|
| Absence de fibrose | F0 |
| **Fibrose portale sans septa** | **F1** |
| **Fibrose portale avec septas** | **F2** |
| Fibrose septale sans cirrhose | F3 |
| Cirrhose | F4 |

Ledit stade de fibrose légère est donc un degré de fibrose hépatique qui, selon le système de scores Metavir, a un score de F1.

Un score de fibrose hépatique indiquant que le foie dudit sujet n'a pas dépassé ledit stade de fibrose légère est donc un score d'au plus F1, c'est-à-dire un score de F0 ou F1, selon le système de scores Metavir.

Un score de fibrose hépatique indiquant que le foie dudit sujet a dépassé ledit stade de fibrose légère est donc un score d'au moins F2, c'est-à-dire un score de F2, F3 ou F4, selon le système de scores Metavir.

Un autre système de scores de fibrose hépatique est le système d'Ishak (Goodman 2007).

**Tableau 26 : correspondance entre les stades de fibrose et les scores de fibrose Ishak**

| Stade de fibrose | Score de fibrose Ishak |
|---|---|
| Absence de fibrose | F0 |
| **Fibrose portale sans septa** | **F1/F2** |
| **Fibrose portale avec septas** | **F3** |
| Fibrose septale sans cirrhose | F4 |
| Cirrhose | F5/F6 |

Ledit stade de fibrose légère est donc un degré de fibrose hépatique qui, selon le système de scores Ishak, a un score de F1/F2.

Un score de fibrose hépatique indiquant que le foie dudit sujet n'a pas dépassé ledit stade de fibrose légère est donc un score d'au plus F2, c'est-à-dire un score de F0, F1 ou F2, selon le système de scores Ishak.

Un score de fibrose hépatique indiquant que le foie dudit sujet a dépassé ledit stade de fibrose légère est donc un score d'au moins F3, c'est-à-dire un score de F3, F4, F5 ou F6, selon le système de scores Ishak.

Le fait de déterminer si le stade (ou degré) de fibrose hépatique d'un sujet infecté par un ou plusieurs virus de l'hépatite et/ou atteint d'hépatite, plus particulièrement d'hépatite chronique, a ou non dépassé le stade de la fibrose légère revêt une importance particulière pour le praticien.

En effet, la plupart des traitements (qu'il s'agisse de traitement anti-hépatite, anti-VHC ou anti-fibrose hépatique), et plus particulièrement ceux qui comprennent l'administration d'interféron, présentent des effets secondaires extrêmement lourds. Ces traitements ne sont donc généralement administrés que lorsque le foie dudit sujet dépasse le stade de la fibrose légère (score Metavir d'au moins F2).

Ainsi, la méthode de la demande peut donc être utilisée non seulement dans le diagnostic d'une fibrose hépatique ou du stade de fibrose hépatique, mais également dans le traitement d'une hépatopathie, notamment pour déterminer le moment auquel un traitement doit être administré au sujet. La méthode de l'invention peut donc ainsi être impliquée dans une méthode pour la thérapie d'un sujet, qui comprend le fait de n'administrer un traitement anti-hépatite et/ou anti-VHC et/ou anti-fibrose hépatique que lorsque, à l'aide des moyens de la demande, il a été déterminé, que le sujet a dépassé le stade de la fibrose légère, ou bien encore une méthode pour la thérapie d'un sujet, qui comprend le fait d'ajuster un traitement anti-hépatite et/ou anti-VHC et/ou anti-fibrose hépatique en fonction de la réponse du tissu hépatique du patient, telle que déterminée à l'aide des moyens de la demande. Ledit traitement peut notamment être un traitement visant à bloquer ou ralentir la progression de la fibrose hépatique, en éliminant le virus (notamment, dans le cas de l'hépatite C) et/ou en bloquant la réplication du virus (notamment dans le cas de l'hépatite B).

Avantageusement, la méthode de la demande est applicable quel que soit le stade de fibrose hépatique dudit sujet : ce stade peut être celui de l'absence de fibrose hépatique jusqu'à celui de la cirrhose.

Le score de fibrose hépatique dudit sujet peut donc être, selon le système de scores de fibrose Metavir, de F0, F1, F2, F3 ou F4, plus particulièrement de F0, F1, F2 ou F3, plus particulièrement de F0, F1 ou F2, plus particulièrement de F1, F2 ou F3, plus particulièrement de F1 ou F2.

Ledit sujet peut être un sujet dont la dureté du foie a été préalablement mesurée, notamment par une méthode non invasive telle que l'élastographie impulsionnelle (par exemple par FIBROSCAN™).

La méthode de la demande met en œuvre la quantification, plus particulièrement la détection et la quantification, de marqueurs (ou variables) biologiques.

Les marqueurs biologiques (ou biomarqueurs) sélectionnés pour la mise en œuvre de la méthode comprennent plusieurs molécules circulantes différentes. Ils peuvent en outre comprendre zéro, un ou plusieurs marqueur(s) additionnel(s), qui ne sont pas des molécules circulantes.

### Molécules circulantes

Le terme « molécules circulantes » est entendu conformément à sa signification usuelle dans le domaine. D'une manière générale, les molécules circulantes sont des molécules qui sont présentes dans le sang d'un mammifère, plus particulièrement d'un être humain, sous forme acellulaire : elles ne sont contenues ni dans une cellule circulante ni dans une cellule tissulaire. D'une manière générale, les molécules circulantes dans le sang sont également circulantes dans le plasma. D'une manière générale, les molécules circulantes dans le sang sont également circulantes dans le sérum. Les molécules circulantes peuvent par exemple être des protéines, des glycoprotéines, des enzymes, des polysaccharides, des lipides, des glycérides, des hormones. Les molécules circulantes peuvent par exemple être des molécules produites, ou des métabolites produits par les cellules de l'organisme dudit sujet. Plus particulièrement, les molécules circulantes peuvent par exemple être des molécules (ou métabolites) qui sont produits par les cellules de l'organisme dudit sujet, mais qui ne sont pas produits par un virus, plus particulièrement un virus de l'hépatite. Les éléments figurés du sang, tels que les plaquettes, ainsi que les virus, tel que les virus de l'hépatite, sont circulants, mais ne sont pas des molécules : ils ne font donc pas partie des molécules circulantes au sens de la demande. Lorsque ledit sujet est un humain, ces molécules circulantes sont des molécules circulantes humaines. Des exemples de molécules circulantes comprennent notamment :
- la protéine CXCL10 (forme complète et formes clivées),
- l'acide hyaluronique ou hyaluronan (HA),
- la gamma glutamyl transpeptidase (GGT),
- l'aspartate aminotransférase (AST),
- l'alanine aminotransférase (ALT),
- l'apolipoprotéine A1 (ApoA1),
- l'alpha 2 macroglobuline (A2M),
- l'inhibiteur 1 de métalloprotéinase (TIMP1),
- la vimentine (VIM),
- la phosphoprotéine 1 sécrétée (SPP1),
- le transducteur de signal interleukin-6 (IL6ST),
- l'inhibiteur de kinase 2A cycline dépendent (p14ARF),
- la métallopeptidase 9 de matrice (MMP9),
- l'angiopoïétine 2 (ANGPT2),
- le ligand 11 à chémokine, motif CXC (CXCL11),
- la métallopeptidase 2 de matrice (MMP2),
- la métalloprotéinase 7 de matrice (MMP7),
- la protéine A4 liant le calcium S100 (S100A4),
- l'inhibiteur 1 de métalloprotéinase (TIMP1),
- la protéine 1 de type chitinase-3 (CHI3L1),
- la chaîne alpha-1(I) du collagène (COL1A1),
- la chimiokine 1 de la protéine alpha régulant la croissance, motif CXC (CXCL1)
- le ligand 6 à chémokine, motif CXC (CXCL6),
- la protéine « *Indian Hedgehog* » (IHH),
- le facteur de transcription 3G stimulé par interféron (IRF9),
- la métalloprotéinase 1 de matrice (MMP1).

Pour quantifier, plus particulièrement pour détecter et quantifier, une ou des molécules circulantes, un prélèvement d'un échantillon biologique, plus particulièrement un échantillon de fluide biologique, doit être ou avoir été fait sur ledit sujet.

Conformément à la demande, les molécules circulantes sélectionnées pour la mise en œuvre de la méthode comprennent (ou consistent en) au moins deux molécules circulantes différentes.

Plus particulièrement, les molécules circulantes sélectionnées comprennent (ou consistent en) au moins l'acide hyaluronique (HA) et la protéine CXCL10 (CXCL10).

Les inventeurs démontrent que la combinaison du marqueur CXCL10 au marqueur HA conduit à un effet synergique, à savoir à des performances qui vont au-delà de la simple juxtaposition de leurs performances individuelles respectives (performances d'AUC et/ou Taux de bonne classification et/ou sensibilité et/ou VPN et/ou spécificité et/ou VPP). Des démonstrations expérimentales sont présentées dans les exemples ci-dessous, notamment :
- en exemple 1 (*cf.* Tableau 3),
- en exemple 3 (*cf.* Tableaux 8 et 10), et
- en exemple 7 (*cf.* Tableaux 20 et 21).

Des comparaisons avec des tests non invasifs de l'art antérieur sont par ailleurs présentées en exemples 2 et 4 ci-dessous.

L'acide hyaluronique (HA) et la protéine CXCL10 (CXCL10) sont bien connus de la personne du métier.

HA est un des principaux composants de la matrice extracellulaire. C'est un glycosaminoglycane de formule brute C₁₄H₂₃NO₁₂(C₁₄H₂₁NO₁₁)n (numéro CAS = 9004-61-9). Sa masse molaire est d'environ 776,647 g/mol. HA est un polymère d'acide hyalobiuronique ; sa formule développée est la suivante :

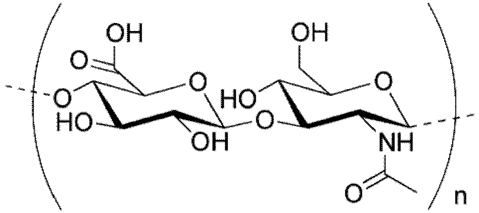

CXCL10 est le ligand 10 à chémokine (motif CXC) [*C-X-C motif chemokine 10*], également connu sous la désignation de C7 ; IFI10 ; INP10 ; IP-10 ; SCYB10 ; crg-2 ; gIP-10 ou mob-1. Sa séquence humaine est décrite dans les bases de données sous le numéro d'accès NM_001565.

La forme précurseur de la protéine CXCL10 (humaine) est une protéine constituée de 98 acides aminés, dont la séquence est : 
MNQTAILICCLIFLTLSGIQGVPLSRTVRCTCISISNQPVNPRSLEKLEIIPASQFCPRV
EIIATMKKKGEKRCLNPESKAIKNLLKAVSKERSKRSP [SEQ ID NO : 1]
 ou 
MNQTAILICCLIFLTLSGIQGVPLSRTVRCTCISISNQPVNPRSLEKLEIIPASQFCPRV
EIIATMKKKGEKRCLNPESKAIKNLLKAVSKEMSKRSP [SEQ ID NO : 2],
 ces deux séquences ne différant que d'un acide aminé, qui est situé en partie C-terminale (ERS en partie C-terminale de SEQ ID NO : 1 *versus* EMS en partie C-terminale de SEQ ID NO : 2).
Le peptide signal de la forme précurseur de CXCL10 est un peptide constitué de 21 acides aminés, qui est égal au fragment 1-21 de la séquence de SEQ ID NO : 1 ou 2, à savoir : MNQTAILICC LIFLTLSGIQG [**SEQ ID NO : 3**].

Il existe au moins trois formes circulantes de la protéine CXCL10, à savoir :
- une forme dite agoniste, qui est constituée de 77 acides aminés,
- une forme dite antagoniste, qui est constituée de 75 acides aminés, et
- une forme qui résulte d'un clivage après sécrétion (clivage protéolytique par les kératinocytes), qui est constituée de 73 acides aminés.

La forme agoniste de CXCL10 est une protéine de 77 acides aminés qui a la séquence suivante : 
VPLSRTVRCTCISISNQPVNPRSLEKLEIIPASQFCPRVEIIATMKKKGEKRCLNPES
KAIKNLLKAVSKERSKRSP [SEQ ID NO : 4],
 ou 
VPLSRTVRCTCISISNQPVNPRSLEKLEIIPASQFCPRVEIIATMKKKGEKRCLNPES
KAIKNLLKAVSKEMSKRSP [SEQ ID NO: 5].
 La structure de cette protéine de 77 acides aminés a été déterminée par RMN (identité PDB = 1LV9, publiée le 18 septembre 2002 ; *cf.* Booth *et al.* 2002), et par rayons X (identité PDB à 3 Å = 107Y ; identité PDB à 2 Å = 1080 ; identité PDB à 1,92 Å = 107Z ; Swaminathan *et al.* 2003).

La forme antagoniste de CXCL10 est une protéine de 75 acides aminés dont la séquence est celle de la forme agoniste tronquée des 2 acides aminés N-terminaux. La forme antagoniste de CXCL10 a donc la séquence suivante : 
LSRTVRCTCISISNQPVNPRSLEKLEIIPASQFCPRVEIIATMKKKGEKRCLNPESKAI
KNLLKAVSKERSKRSP [SEQ ID NO : 6]
 ou

LSRTVRCTCISISNQPVNPRSLEKLEIIPASQFCPRVEIIATMKKKGEKRCLNPESKAI
KNLLKAVSKEMSKRSP [SEQ ID NO : 7].

La forme de CXCL10 qui résulte d'un clivage après sécrétion est une protéine de 73 acides aminés, dont la séquence est celle de la forme agoniste tronquée des 4 acides aminés C-terminaux. La forme de CXCL10 qui résulte d'un clivage après sécrétion a donc la séquence suivante : 
VPLSRTVRCTCISISNQPVNPRSLEKLEIIPASQFCPRVEIIATMKKKGEKRCLNPES
KAIKNLLKAVSKERS [SEQ ID NO : 8]
 ou

VPLSRTVRCTCISISNQPVNPRSLEKLEIIPASQFCPRVEIIATMKKKGEKRCLNPES
KAIKNLLKAVSKEMS [SEQ ID NO : 9].

Les formes circulantes de la protéine CXCL10 (humaine) comprennent donc les protéines de SEQ ID NO : 4 à 9, plus particulièrement :
- SEQ ID NO : 4 et/ou NO : 5,
- SEQ ID NO : 6 et/ou NO : 7, et
- SEQ ID NO : 8 et/ou NO : 9.

Conformément à la demande, la quantification, plus particulièrement la détection et la quantification de CXCL10, comprend la quantification, ou la détection et la quantification, d'au moins une, plus particulièrement de plusieurs, plus particulièrement de toutes les formes circulantes de la protéine CXCL10.

Conformément à la demande, la quantification, plus particulièrement la détection et la quantification de CXCL10, comprend la quantification, ou la détection et la quantification, d'au moins une, plus particulièrement de plusieurs, plus particulièrement de toutes les formes circulantes les protéines de SEQ ID NO : 4 à 9.

Les molécules circulantes sélectionnées pour la mise en œuvre de la méthode peuvent être constituées par la protéine CXCL10 et par HA. Dans ce cas, CXCL10 et HA sont les deux seules molécules circulantes qui sont sélectionnées ou utilisées comme marqueurs biologiques.

Alternativement, une ou plusieurs autres protéines circulantes (différentes) peuvent être sélectionnées en sus de HA et CXCL10.

Selon un mode de réalisation alternatif ou complémentaire, ces autres molécules circulantes sélectionnées ne comprennent pas l'alpha 2 macroglobuline (A2M). En effet, contrairement à HA et à la protéine CXCL10, la protéine A2M est présente en forte quantité dans le sang des sujets humains. De ce fait, il n'est pas possible, ou à tout le moins très difficile en routine, de mesurer la concentration de A2M en multiplex avec celles de HA et de la protéine CXCL10.

Selon un mode de réalisation alternatif ou complémentaire, ces autres molécules circulantes sélectionnées ne comprennent pas le « *Granulocyte-Macrophage Colony-Stimulating Factor* » (GMCSF).

Selon un mode de réalisation alternatif ou complémentaire, ces autres molécules circulantes sélectionnées ne comprennent pas l'interleukine 12 (IL12).

Selon un mode de réalisation alternatif ou complémentaire, ces autres molécules circulantes sélectionnées ne comprennent pas l'interleukine 2 (IL2).

Selon un mode de réalisation alternatif ou complémentaire, ces autres molécules circulantes sélectionnées ne comprennent pas la métallopeptidase 13 de matrice (MMP13).

Selon un mode de réalisation alternatif ou complémentaire, ces autres molécules circulantes sélectionnées ne comprennent pas l'alanine aminotransférase (ALT).

Selon un mode de réalisation alternatif ou complémentaire, ces autres molécules circulantes sélectionnées ne comprennent pas la gamma glutamyl transpeptidase (GGT).

Selon un mode de réalisation alternatif ou complémentaire, ces autres molécules circulantes sélectionnées ne comprennent pas la protéine « *InterCellular Adhesion Molecule* » (ICAM1).

Selon un mode de réalisation alternatif ou complémentaire, ces autres molécules circulantes sélectionnées ne comprennent pas l'interleukine 4 (IL4).

Selon un mode de réalisation alternatif ou complémentaire, ces autres molécules circulantes sélectionnées ne comprennent pas le ligand 6 à chémokine motif CXC (CXCL9).

Selon un mode de réalisation alternatif ou complémentaire, ces autres molécules circulantes sélectionnées ne comprennent pas la protéine « *Vascular Cell Adhesion Molecule 1* » (VCAM1).

Selon un mode de réalisation alternatif ou complémentaire, ces autres molécules circulantes sélectionnées ne comprennent pas la « *Retinol Binding Protein* 4 » (RBP4).

Selon un mode de réalisation alternatif ou complémentaire, ces autres molécules circulantes sélectionnées ne comprennent pas l'inhibiteur 1 de métalloprotéinase (TIMP1).

Selon un mode de réalisation alternatif ou complémentaire, ces autres molécules circulantes sélectionnées ne comprennent pas la vimentine (VIM).

Selon un mode de réalisation alternatif ou complémentaire, ces autres molécules circulantes sélectionnées ne comprennent pas la phosphoprotéine 1 sécrétée (SPP1).

Selon un mode de réalisation alternatif ou complémentaire, ces autres molécules circulantes sélectionnées ne comprennent pas l'aspartate aminotransférase (AST).

Selon un mode de réalisation alternatif ou complémentaire, ces autres molécules circulantes sélectionnées ne comprennent pas l'apolipoprotéine A1 (ApoA1).
Selon un mode de réalisation alternatif ou complémentaire, ces autres molécules circulantes sélectionnées ne comprennent pas le transducteur de signal interleukin-6 (IL6ST).
Selon un mode de réalisation alternatif ou complémentaire, ces autres molécules circulantes sélectionnées ne comprennent pas l'inhibiteur de kinase 2A cycline dépendent (p14ARF).

Selon un mode de réalisation alternatif ou complémentaire, ces autres molécules circulantes sélectionnées ne comprennent pas la métallopeptidase 9 de matrice (MMP9). Selon un mode de réalisation alternatif ou complémentaire, ces autres molécules circulantes sélectionnées ne comprennent pas l'angiopoïétine 2 (ANGPT2).

Selon un mode de réalisation alternatif ou complémentaire, ces autres molécules circulantes sélectionnées ne comprennent pas le ligand 11 à chémokine motif CXC (CXCL11).

Selon un mode de réalisation alternatif ou complémentaire, ces autres molécules circulantes sélectionnées ne comprennent pas la métallopeptidase 2 de matrice (MMP2).

Selon un mode de réalisation alternatif ou complémentaire, ces autres molécules circulantes sélectionnées ne comprennent pas la métallopeptidase 7 de matrice (MMP7).

Selon un mode de réalisation alternatif ou complémentaire, ces autres molécules circulantes sélectionnées ne comprennent pas la protéine A4 liant le calcium S100 (S100A4).

Selon un mode de réalisation alternatif ou complémentaire, ces autres molécules circulantes sélectionnées ne comprennent pas l'inhibiteur 1 de métalloprotéinase (TIMP1).

Selon un mode de réalisation alternatif ou complémentaire, ces autres molécules circulantes sélectionnées ne comprennent pas la protéine 1 de type chitinase-3 (CHI3L1).

Selon un mode de réalisation alternatif ou complémentaire, ces autres molécules circulantes sélectionnées ne comprennent pas la chaîne alpha-1(I) du collagène (COL1A1).

Selon un mode de réalisation alternatif ou complémentaire, ces autres molécules circulantes sélectionnées ne comprennent pas la chémokine 1 de la protéine alpha régulant la croissance motif CXC (CXCL1).

Selon un mode de réalisation alternatif ou complémentaire, ces autres molécules circulantes sélectionnées ne comprennent pas le ligand 6 à chémokine motif CXC (CXCL6).

Selon un mode de réalisation alternatif ou complémentaire, ces autres molécules circulantes sélectionnées ne comprennent pas la protéine « *Indian Hedgehog* » (IHH).

Selon un mode de réalisation alternatif ou complémentaire, ces autres molécules circulantes sélectionnées ne comprennent pas le facteur de transcription 3G stimulé par interféron (IRF9).

Selon un mode de réalisation alternatif ou complémentaire, ces autres molécules circulantes sélectionnées ne comprennent pas la métalloprotéinase 1 de matrice (MMP1).

Selon un mode de réalisation alternatif ou complémentaire, ces autres molécules circulantes sélectionnées ne comprennent aucune des protéines GMCSF, IL12, IL2 et MMP13.

Selon un mode de réalisation alternatif ou complémentaire, ces autres molécules circulantes sélectionnées ne comprennent aucune des protéines A2M, GMCSF, IL12, IL2 et MMP13.

Selon un mode de réalisation alternatif ou complémentaire, ces autres molécules circulantes sélectionnées ne comprennent aucune des protéines ALT, GGT, ICAM1, IL4, CXCL9, VCAM1 et RBP4.

Selon un mode de réalisation alternatif ou complémentaire, ces autres molécules circulantes sélectionnées ne comprennent aucune des protéines A2M, ALT, GGT, ICAM1, IL4, CXCL9, VCAM1 et RBP4.

Selon un mode de réalisation alternatif ou complémentaire, ces autres molécules circulantes sélectionnées ne comprennent aucune des protéines A2M, TIMP1, VIM et SPP1.

Selon un mode de réalisation alternatif ou complémentaire, ces autres molécules circulantes sélectionnées ne comprennent aucune des protéines A2M, AST, ApoA1, IL6ST, p14ARF, MMP9, ANGPT2, CXCL11, MMP2, MMP7, S100A4, TIMP1, CHI3L1, COL1A1, CXCL1, CXCL6, IHH, IRF9 et MMP1.

Selon un mode de réalisation alternatif ou complémentaire, ces autres molécules circulantes sélectionnées ne comprennent aucune des protéines A2M, GMCSF, IL12, IL2, MMP13, ALT, GGT, ICAM1, IL4, CXCL9, VCAM1, RBP4, TIMP1, VIM, SPP1, AST, ApoA1, IL6ST, p14ARF, MMP9, ANGPT2, CXCL11, MMP2, MMP7, S100A4, TIMP1, CHI3L1, COL1A1, CXCL1, CXCL6, IHH, IRF9 et MMP1.

Selon un mode de réalisation alternatif ou complémentaire, le nombre total de molécules circulantes (différentes) qui sont sélectionnées sont au nombre de six, cinq, quatre, trois ou deux, plus particulièrement au nombre de cinq, quatre, trois ou deux, plus particulièrement au nombre de quatre, trois ou deux, plus particulièrement au nombre de trois ou deux, plus particulièrement au nombre de deux.

### Éventuel(s) marqueur(s) additionnel(s) [qui ne sont pas des molécules circulantes]

En sus des molécules circulantes (et notamment en sus de HA et CXCL10), les marqueurs biologiques sélectionnés peuvent en outre comprendre zéro, un ou plusieurs marqueur(s) additionnel(s), qui ne sont pas des molécules circulantes, plus particulièrement qui ne sont pas des molécules circulantes humaines.

Le terme « marqueur(s) additionnel(s) » (ou « marqueur(s) additionnel(s) sélectionné(s) ») signifie ici celui(ceux) des marqueurs biologiques sélectionnés qui n'est pas (ne sont pas) une(des) molécule(s) circulante(s), plus particulièrement qui n'est pas (ne sont pas) une(des) molécule(s) circulante(s) humaine(s).

Par exemple, les marqueurs biologiques sélectionnés peuvent comprendre, en sus desdites molécules circulantes (et notamment en sus de HA et CXCL10), un ou plusieurs marqueur(s) additionnel(s), qui est(sont) choisi(s) parmi :
- les éléments figurés du sang (par exemple, les plaquettes),
- les caractéristiques (ou marqueurs) cliniques ou anatomiques dudit sujet, et
- les caractéristiques (ou marqueurs) virologiques dudit sujet.

Plus particulièrement, ledit(lesdits) marqueur(s) additionnel(s) est(sont) choisi(s) parmi :
- les caractéristiques (ou marqueurs) cliniques ou anatomiques dudit sujet, et
- les caractéristiques (ou marqueurs) virologiques dudit sujet.

Les caractéristiques (ou marqueurs) cliniques ou anatomiques sont avantageusement des caractéristiques dont la mesure ne requiert pas une détection ou une quantification dans un échantillon de fluide biologique dudit sujet, et plus généralement dont la mesure peut être faite sans prélèvement d'échantillon biologique dudit sujet. Par exemple, l'âge, l'Indice de Masse Corporelle (IMC), le sexe et la dureté du foie sont des caractéristiques (ou marqueurs) cliniques ou anatomiques qui peuvent être quantifiées par mesure extracorporelle, sans prélèvement de fluide biologique, et plus généralement sans prélèvement d'échantillon biologique dudit sujet (la dureté du foie peut être mesurée par élastographie impulsionnelle, notamment par FIBROSCAN™), et plus généralement sans aucune intervention invasive.

Des exemples de caractéristiques cliniques ou anatomiques dudit sujet comprennent notamment l'âge dudit sujet, l'IMC dudit sujet, le sexe dudit sujet, et la dureté du foie dudit sujet (FS), plus particulièrement l'âge dudit sujet, l'IMC dudit sujet, et la dureté du foie dudit sujet (FS).

Le marqueur « âge dudit sujet » comprend notamment :
- l'âge dudit sujet à la date à laquelle a été fait le prélèvement de fluide biologique requis pour la détection ou la quantification des molécules circulantes,
- l'âge dudit sujet à la date à laquelle le diagnostic d'une infection par virus de l'hépatite a été fait,
- l'âge dudit sujet à la date à laquelle il a reçu pour la première fois une thérapie anti-hépatite,
plus particulièrement l'âge dudit sujet à la date à laquelle a été fait le prélèvement de fluide biologique requis pour la détection ou la quantification des molécules circulantes.

Le marqueur « IMC dudit sujet » comprend notamment :
- l'IMC dudit sujet à la date à laquelle a été fait le prélèvement de fluide biologique requis pour la détection ou la quantification des molécules circulantes,
- l'IMC dudit sujet à la date à laquelle le diagnostic d'une infection par virus de l'hépatite a été fait,
- l'IMC dudit sujet à la date à laquelle il a reçu pour la première fois une thérapie anti-hépatite,
plus particulièrement l'IMC dudit sujet à la date à laquelle a été fait le prélèvement de fluide biologique requis pour la détection ou la quantification des molécules circulantes.

Des exemples de caractéristiques cliniques ou anatomiques dudit sujet comprennent donc plus particulièrement :
- l'âge dudit sujet à la date à laquelle a été fait le prélèvement de fluide biologique requis pour la détection ou la quantification des molécules circulantes,
- l'IMC dudit sujet à la date à laquelle a été fait le prélèvement de fluide biologique requis pour la détection ou la quantification des molécules circulantes,
- le sexe dudit sujet (sexe masculin ou sexe féminin), et
- la dureté du foie dudit sujet (FS).
plus particulièrement :
- l'âge dudit sujet à la date à laquelle a été fait le prélèvement de fluide biologique requis pour la détection ou la quantification des molécules circulantes,
- l'IMC dudit sujet à la date à laquelle a été fait le prélèvement de fluide biologique requis pour la détection ou la quantification des molécules circulantes, et
- la dureté du foie dudit sujet (FS).

Selon un mode de réalisation alternatif ou complémentaire, le nombre total de (différents) marqueur(s) clinique(s) ou anatomique(s) qui sont sélectionné(s) à titre de marqueur(s) additionnel(s) est de zéro, un, deux ou trois, plus particulièrement de zéro, un ou deux, plus particulièrement de zéro ou un, par exemple zéro, par exemple un, par exemple deux, par exemple un, deux ou trois, par exemple un ou deux.

Par exemple, le(les) marqueur(s) clinique(s) ou anatomique(s) sélectionné(s) est(sont) un, deux ou trois marqueur(s) (différents), plus particulièrement un ou deux marqueur(s) (différents), par exemple un marqueur, par exemple deux marqueurs différents, choisi(s) parmi :
- l'âge dudit sujet (plus particulièrement l'âge dudit sujet à la date à laquelle a été fait le prélèvement de fluide biologique requis pour la détection ou la quantification des molécules circulantes),
- l'IMC dudit sujet (plus particulièrement l'IMC dudit sujet à la date à laquelle a été fait le prélèvement de fluide biologique requis pour la détection ou la quantification des molécules circulantes), et
- la dureté du foie dudit sujet (FS).

Les caractéristiques (ou marqueurs) virologiques dudit sujet sont quant à elles des caractéristiques qui nécessitent une détection ou une quantification dans un échantillon de fluide biologique dudit sujet. Avantageusement, cet échantillon de fluide biologique est le même, ou à tout le moins est de même nature, que celui mis en œuvre pour détecter ou quantifier lesdites molécules circulantes (telles que CXCL10 et HA).

Des exemples de caractéristiques virologiques dudit sujet comprennent notamment :
- la nature du(des) virus contenu(s) dans le sang dudit sujet,
- la charge virale dudit sujet (CV), plus particulièrement sa charge virale en virus de l'hépatite,
- le ou les génotype(s) du (ou des) virus de l'hépatite dont est infecté ledit sujet.

Le marqueur « nature du(des) virus contenu(s) dans le sang dudit sujet » comprend notamment la nature du(des) virus de l'hépatite contenu(s) dans le sang dudit sujet : par exemple, VHC et/ou VHB et/ou VHD, plus particulièrement VHC et/ou VHB, plus particulièrement VHC.

Le marqueur « charge virale dudit sujet » (CV) comprend notamment la charge virale dudit sujet en virus de l'hépatite par exemple, sa charge virale en VHC et/ou en VHB et/ou en VHD, plus particulièrement en VHC et/ou VHB, plus particulièrement en VHC.

Le marqueur « génotype(s) du (ou des) virus de l'hépatite dont est infecté ledit sujet » comprend notamment le ou les génotype(s) du (ou des) virus de l'hépatite dont est infecté ledit sujet, par exemple, génotype(s) 1 et/ou 2 et/ou 3 et/ou 4 et/ou 5 et/ou 6 et/ou 7.

Des exemples de caractéristiques virologiques dudit sujet comprennent plus particulièrement la charge virale dudit sujet en virus de l'hépatite, plus particulièrement la charge virale dudit sujet en VHC et/ou en VHB et/ou en VHD, plus particulièrement en VHC et/ou VHB, plus particulièrement en VHC.

Selon un mode alternatif ou complémentaire, le nombre total de (différents) marqueur(s) virologique(s) qui est(sont) sélectionné(s) à titre de marqueur(s) additionnel(s) est de zéro, un ou deux, plus particulièrement de zéro ou un, plus particulièrement de un ou deux, plus particulièrement de un, plus particulièrement de zéro.

Par exemple, aucun marqueur virologique n'est sélectionné, ou bien seulement un seul marqueur virologique est sélectionné et est la charge virale dudit sujet (plus particulièrement sa charge virale en virus de l'hépatite, plus particulièrement sa charge en VHC et/ou en VHB et/ou en VHD, plus particulièrement en VHC et/ou VHB, plus particulièrement en VHC).

Selon un mode de réalisation alternatif ou complémentaire, le nombre total de marqueur(s) additionnel(s) différents sélectionné(s) en sus desdites molécules circulantes (et notamment en sus de HA et CXCL10), est de zéro, un, deux, trois, quatre, cinq, ou plus de cinq.

Plus particulièrement, le nombre total de marqueur(s) additionnel(s) différents tel(s) que ci-dessus décrit(s) est de zéro, un, deux, trois, quatre ou cinq, plus particulièrement de zéro, un, deux, trois ou quatre, plus particulièrement de zéro, un, deux ou trois, plus particulièrement de zéro, un ou deux, plus particulièrement de zéro ou un, plus particulièrement de zéro, plus particulièrement de un, deux, trois, quatre ou cinq, ou plus de cinq, plus particulièrement de un, deux, trois, quatre ou cinq, plus particulièrement de un, deux, trois ou quatre, plus particulièrement de un, deux ou trois, plus particulièrement de un ou deux, plus particulièrement de un, plus particulièrement de deux, plus particulièrement de trois, plus particulièrement de quatre, plus particulièrement de cinq.

Ainsi, en sus desdites molécules circulantes (et notamment en sus de HA et CXCL10), ledit(lesdits) marqueur(s) additionnel(s) peut(peuvent) par exemple comprendre (ou consister en) :
- zéro, un, deux ou trois (différents) marqueur(s) clinique(s) ou anatomique(s) comme ci-dessus décrits, et
- zéro ou un marqueur virologique comme ci-dessus décrit.

Ainsi, en sus desdites molécules circulantes (et notamment en sus de HA et CXCL10), ledit(lesdits) marqueur(s) additionnel(s) peut(peuvent) par exemple comprendre (ou consister en) :
- zéro marqueur clinique ou anatomique comme ci-dessus décrit, ou bien un, deux ou trois (différents) marqueurs choisis parmi l'âge, l'IMC et la dureté du foie, et
- zéro marqueur virologique comme ci-dessus décrit, ou bien le marqueur virologique charge virale.

### Marqueurs biologiques sélectionnés :

Ainsi, les marqueurs biologiques sélectionnés peuvent comprendre (ou consister en) :
- six, cinq, quatre, trois ou deux molécules circulantes différentes telles que ci-dessus décrites, plus particulièrement cinq, quatre, trois ou deux molécules circulantes telles que ci-dessus décrites, plus particulièrement quatre, trois ou deux molécules circulantes telles que ci-dessus décrites, plus particulièrement trois ou deux molécules circulantes telles que ci-dessus décrites, plus particulièrement deux molécules circulantes telles que ci-dessus décrites, lesdites molécules circulantes différentes comprenant au moins HA et CXCL10,
   et
- zéro, un, deux, trois, quatre ou cinq, ou plus de cinq marqueur(s) additionnel(s) différents choisi(s) parmi :
   - l'âge dudit sujet (plus particulièrement l'âge dudit sujet à la date à laquelle a été fait le prélèvement de fluide biologique requis pour la détection ou la quantification des molécules circulantes),
   - l'IMC dudit sujet (plus particulièrement l'IMC dudit sujet à la date à laquelle a été fait le prélèvement de fluide biologique requis pour la détection ou la quantification des molécules circulantes),
   - la dureté du foie dudit sujet (FS), et
   - la charge virale dudit sujet (CV), plus particulièrement, la charge virale dudit sujet en virus de l'hépatite, par exemple, sa charge en VHC et/ou en VHB et/ou en VHD, plus particulièrement en VHC et/ou VHB, plus particulièrement en VHC.
Le nombre total de marqueur(s) additionnel(s) différents choisi(s) ci-dessus est plus particulièrement de zéro, un, deux, trois, quatre ou cinq, plus particulièrement de zéro, un, deux, trois ou quatre, plus particulièrement de zéro, un, deux ou trois, plus particulièrement de zéro, un ou deux, plus particulièrement de zéro ou un, plus particulièrement de zéro, plus particulièrement de un, deux, trois, quatre, cinq, ou plus de cinq, plus particulièrement de un, deux, trois, quatre ou cinq, plus particulièrement de un, deux, trois ou quatre, plus particulièrement de un, deux ou trois, plus particulièrement de un ou deux, plus particulièrement de un, plus particulièrement de deux, plus particulièrement de trois, plus particulièrement de quatre, plus particulièrement de cinq.

Ainsi, les marqueurs biologiques sélectionnés peuvent comprendre (ou consister en) :
- deux molécules circulantes différentes telles que ci-dessus décrites, ces deux molécules circulantes différentes étant constituées par HA et CXCL10,
   et
- zéro, un, deux, trois, quatre, cinq, ou plus de cinq marqueur(s) additionnel(s) différents choisi(s) parmi :
   - l'âge dudit sujet (plus particulièrement l'âge dudit sujet à la date à laquelle a été fait le prélèvement de fluide biologique requis pour la détection ou la quantification des molécules circulantes),
   - l'IMC dudit sujet (plus particulièrement l'IMC dudit sujet à la date à laquelle a été fait le prélèvement de fluide biologique requis pour la détection ou la quantification des molécules circulantes),
   - la dureté du foie dudit sujet (FS), et
   - la charge virale dudit sujet (CV), plus particulièrement, la charge virale dudit sujet en virus de l'hépatite, par exemple, sa charge en VHC et/ou en VHB et/ou en VHD, plus particulièrement en VHC et/ou VHB, plus particulièrement en VHC.
Le nombre total de marqueur(s) additionnel(s) différents choisi(s) ci-dessus est plus particulièrement de zéro, un, deux, trois, quatre ou cinq, plus particulièrement de zéro, un, deux, trois ou quatre, plus particulièrement de zéro, un, deux ou trois, plus particulièrement de zéro, un ou deux, plus particulièrement de zéro ou un, plus particulièrement de zéro, plus particulièrement de un, deux, trois, quatre, cinq, ou plus de cinq, plus particulièrement de un, deux, trois, quatre ou cinq, plus particulièrement de un, deux, trois ou quatre, plus particulièrement de un, deux ou trois, plus particulièrement de un ou deux, plus particulièrement de un, plus particulièrement de deux, plus particulièrement de trois, plus particulièrement de quatre, plus particulièrement de cinq.

Ainsi, les marqueurs biologiques sélectionnés peuvent comprendre (ou consister en) :
- deux molécules circulantes différentes telles que ci-dessus décrites, ces deux molécules circulantes différentes étant constituées par HA et CXCL10,
   et
- zéro, un, deux ou trois ou quatre marqueur(s) additionnel(s) différents choisi(s) parmi :
   - l'âge dudit sujet (plus particulièrement l'âge dudit sujet à la date à laquelle a été fait le prélèvement de fluide biologique requis pour la détection ou la quantification des molécules circulantes),
   - l'IMC dudit sujet (plus particulièrement l'IMC dudit sujet à la date à laquelle a été fait le prélèvement de fluide biologique requis pour la détection ou la quantification des molécules circulantes),
   - la dureté du foie dudit sujet (FS), et
   - la charge virale dudit sujet (CV), plus particulièrement, la charge virale dudit sujet en virus de l'hépatite, par exemple, sa charge en VHC et/ou en VHB et/ou en VHD, plus particulièrement en VHC et/ou VHB, plus particulièrement en VHC.
Le nombre total de marqueur(s) additionnel(s) différents choisi(s) ci-dessus est plus particulièrement de zéro, un, deux ou trois, plus particulièrement de un, deux ou trois, plus particulièrement de deux ou trois, plus particulièrement de zéro, un ou deux, plus particulièrement de zéro ou un, plus particulièrement de zéro, plus particulièrement de un, deux ou trois, plus particulièrement de un ou deux, plus particulièrement de un, plus particulièrement de deux, plus particulièrement de trois, plus particulièrement de quatre.

Par exemple, les marqueurs biologiques sélectionnés peuvent comprendre (ou consister en) HA et CXCL10.

Par exemple, les marqueurs biologiques sélectionnés peuvent comprendre (ou consister en) HA, CXCL10, et l'âge dudit sujet (plus particulièrement l'âge dudit sujet à la date à laquelle a été fait le prélèvement de fluide biologique requis pour la détection ou la quantification des molécules circulantes).

Par exemple, les marqueurs biologiques sélectionnés peuvent comprendre (ou consister en) HA, CXCL10, et l'IMC dudit sujet (plus particulièrement l'IMC dudit sujet à la date à laquelle a été fait le prélèvement de fluide biologique requis pour la détection ou la quantification des molécules circulantes).

Par exemple, les marqueurs biologiques sélectionnés peuvent comprendre (ou consister en) HA, CXCL10 et la dureté du foie dudit sujet (FS).

Par exemple, les marqueurs biologiques sélectionnés peuvent comprendre (ou consister en) : HA, CXCL10, et la charge virale (CV) dudit sujet, plus particulièrement, la charge virale dudit sujet en virus de l'hépatite, par exemple, sa charge en VHC et/ou en VHB et/ou en VHD, plus particulièrement en VHC et/ou VHB, plus particulièrement en VHC.

Par exemple, les marqueurs biologiques sélectionnés peuvent comprendre (ou consister en) :
- HA,
- CXCL10,
- l'âge dudit sujet (plus particulièrement l'âge dudit sujet à la date à laquelle a été fait le prélèvement de fluide biologique requis pour la détection ou la quantification des molécules circulantes), et
- l'IMC dudit sujet (plus particulièrement l'IMC dudit sujet à la date à laquelle a été fait le prélèvement de fluide biologique requis pour la détection ou la quantification des molécules circulantes).

Par exemple, les marqueurs biologiques sélectionnés peuvent comprendre (ou consister en) :
- HA,
- CXCL10,
- l'âge dudit sujet (plus particulièrement l'âge dudit sujet à la date à laquelle a été fait le prélèvement de fluide biologique requis pour la détection ou la quantification des molécules circulantes) et
- la charge virale (CV) dudit sujet, plus particulièrement, la charge virale dudit sujet en virus de l'hépatite, par exemple, sa charge en VHC et/ou en VHB et/ou en VHD, plus particulièrement en VHC et/ou VHB, plus particulièrement en VHC.

Par exemple, les marqueurs biologiques sélectionnés peuvent comprendre (ou consister en) :
- HA,
- CXCL10,
- l'âge dudit sujet (plus particulièrement l'âge dudit sujet à la date à laquelle a été fait le prélèvement de fluide biologique requis pour la détection ou la quantification des molécules circulantes), et
- la dureté du foie dudit sujet (FS).

Par exemple, les marqueurs biologiques sélectionnés peuvent comprendre (ou consister en) :
- HA,
- CXCL10,
- l'IMC dudit sujet (plus particulièrement l'IMC dudit sujet à la date à laquelle a été fait le prélèvement de fluide biologique requis pour la détection ou la quantification des molécules circulantes), et
- la charge virale (CV) dudit sujet, plus particulièrement, la charge virale dudit sujet en virus de l'hépatite, par exemple, sa charge en VHC et/ou en VHB et/ou en VHD, plus particulièrement en VHC et/ou VHB, plus particulièrement en VHC.

Par exemple, les marqueurs biologiques sélectionnés peuvent comprendre (ou consister en) :
- HA,
- CXCL10,
- l'IMC dudit sujet (plus particulièrement l'IMC dudit sujet à la date à laquelle a été fait le prélèvement de fluide biologique requis pour la détection ou la quantification des molécules circulantes), et
- la dureté du foie (FS) dudit sujet.

Par exemple, les marqueurs biologiques sélectionnés peuvent comprendre (ou consister en) :
- HA,
- CXCL10,
- la charge virale (CV) dudit sujet, plus particulièrement, la charge virale dudit sujet en virus de l'hépatite, par exemple, sa charge en VHC et/ou en VHB et/ou en VHD, plus particulièrement en VHC et/ou VHB, plus particulièrement en VHC, et
- la dureté du foie (FS) dudit sujet.

Par exemple, les marqueurs biologiques sélectionnés peuvent comprendre (ou consister en) :
- HA,
- CXCL10,
- l'âge (plus particulièrement l'âge dudit sujet à la date à laquelle a été fait le prélèvement de fluide biologique requis pour la détection ou la quantification des molécules circulantes),
- l'IMC dudit sujet (plus particulièrement l'IMC dudit sujet à la date à laquelle a été fait le prélèvement de fluide biologique requis pour la détection ou la quantification des molécules circulantes), et
- la charge virale (CV) dudit sujet, plus particulièrement, la charge virale dudit sujet en virus de l'hépatite, par exemple, sa charge en VHC et/ou en VHB et/ou en VHD, plus particulièrement en VHC et/ou VHB, plus particulièrement en VHC.

Par exemple, les marqueurs biologiques sélectionnés peuvent comprendre (ou consister en) :
- HA,
- CXCL10,
- l'âge dudit sujet (plus particulièrement l'âge dudit sujet à la date à laquelle a été fait le prélèvement de fluide biologique requis pour la détection ou la quantification des molécules circulantes),
- l'IMC dudit sujet (plus particulièrement l'IMC dudit sujet à la date à laquelle a été fait le prélèvement de fluide biologique requis pour la détection ou la quantification des molécules circulantes), et
- la dureté du foie dudit sujet (FS).

Par exemple, les marqueurs biologiques sélectionnés peuvent comprendre (ou consister en) :
- HA,
- CXCL10,
- l'IMC dudit sujet (plus particulièrement l'IMC dudit sujet à la date à laquelle a été fait le prélèvement de fluide biologique requis pour la détection ou la quantification des molécules circulantes),
- la charge virale (CV) dudit sujet, plus particulièrement, la charge virale dudit sujet en virus de l'hépatite, par exemple, sa charge en VHC et/ou en VHB et/ou en VHD, plus particulièrement en VHC et/ou VHB, plus particulièrement en VHC, et
- la dureté du foie (FS) dudit sujet.

Dans les fonctions LOGIT, mROC et CART ici décrites (LOGIT₁ à LOGIT₄, Z₁ à Z₁₃, CART₁ à CART₄ ; *cf.* ci-dessous), les biomarqueurs sont plus particulièrement comme suit :
- le biomarqueur CXCL10 est la valeur de quantification des formes circulantes de CXCL10 dans ledit patient, plus particulièrement la concentration sérique en CXCL10, exprimée par exemple en mg/mL, en µg/mL, ng/mL ou pg/mL, plus particulièrement en pg/mL,
- le biomarqueur HA est la valeur de quantification de HA dans ledit patient, plus particulièrement peut être la concentration sérique en HA, exprimée par exemple en mg/mL, en µg/mL, ng/mL ou pg/mL, plus particulièrement en ng/mL,
- le biomarqueur IMC est l'IMC dudit sujet, plus particulièrement l'IMC calculée comme étant la masse dudit sujet en kg divisée par (la taille dudit sujet en m)², plus particulièrement l'IMC dudit sujet à la date à laquelle a été fait le prélèvement de fluide biologique requis pour la détection ou la quantification des molécules circulantes CXCL10 et HA,
- le biomarqueur Âge est l'âge dudit sujet, par exemple exprimé en nombre d'années (nombre entier ou avec décimale(s)), plus particulièrement l'âge dudit sujet à la date à laquelle a été fait le prélèvement de fluide biologique requis pour la détection ou la quantification des molécules circulantes CXCL10 et HA,
- le biomarqueur CV est la charge virale dudit sujet en virus de l'hépatite, plus particulièrement sa charge en VHC et/ou en VHB et/ou en VHD, plus particulièrement en VHC et/ou VHB, plus particulièrement en VHC, par exemple exprimée en copies/mL ou en UI/mL ou en multiple de l'une de ces unités tel que en 10³ copies par mL, plus particulièrement cette charge à la date à laquelle a été fait le prélèvement de fluide biologique requis pour la détection ou la quantification des molécules circulantes CXCL10 et HA,
- le biomarqueur FS est la dureté du foie dudit sujet, exprimée par exemple en kPA (cette mesure peut être faite de manière non invasive par élastographie impulsionnelle, notamment par FIBROSCAN™).

### Détection et Quantification des marqueurs biologiques

Pour déterminer si le foie d'un sujet infecté par un ou plusieurs virus de l'hépatite et/ou atteint d'une hépatite, plus particulièrement d'une hépatite chronique, n'a pas dépassé le stade de la fibrose portale sans septa, ou s'il a au contraire dépassé ce stade, la méthode de la demande comprend l'étape de quantifier, plus particulièrement de détecter et quantifier, pour ledit sujet les marqueurs biologiques sélectionnés (*cf.* ci-dessus).

Les molécules circulantes sélectionnées (qui comprennent au moins HA et CXCL10) sont quantifiées, plus particulièrement détectées et quantifiées, dans un échantillon de fluide biologique dudit sujet.

Une méthode de la demande peut donc comprendre l'étape de prélever un échantillon de fluide biologique dudit sujet. Toutefois, si la méthode est une méthode *in vitro,* cette étape de prélèvement est une étape préalable à la méthode.

Un échantillon de fluide biologique dudit sujet est par exemple un échantillon de sang, de sérum, de plasma ou d'urine, plus particulièrement un échantillon de sang, de sérum ou de plasma, plus particulièrement un échantillon de sérum ou de plasma, plus particulièrement un échantillon de sérum. Cet échantillon de fluide biologique peut avoir été transformé après collecte. Il peut par exemple avoir subi un traitement de purification et/ou concentration et/ou extraction, tel que par exemple par purification et/ou concentration et/ou extraction de ou des protéines et/ou polypeptides et/ou peptides sériques, ou par purification et/ou extraction et/ou concentration d'une fraction protéique. La purification et/ou concentration peut par exemple être faite par filtration et/ou par gradient de densité.

Les valeurs de quantification des molécules circulantes, plus particulièrement de HA et CXCL10, sont des valeurs de concentration ou de proportion, plus particulièrement des valeurs de concentration. Elles peuvent être par exprimées en mg/mL, en µg/mL, ng/mL ou pg/mL. Par exemple, la valeur de quantification de HA est une valeur de concentration exprimée en ng/mL. Par exemple, la valeur de quantification de CXCL10 est une valeur de concentration exprimée en pg/mL.

Ces valeurs de concentration ou de proportion, plus particulièrement ces valeurs de concentration, peuvent être celles mesurées dans ledit échantillon de fluide biologique ou dans un extrait ou filtrat de cet échantillon. Par exemple, si l'échantillon de fluide biologique prélevé subit un traitement de purification par filtration avant mesure desdites concentrations ou proportions, les valeurs de quantification peuvent être celles mesurées dans le filtrat de purification dudit échantillon biologique. Par exemple, si l'échantillon de fluide biologique prélevé est du sang, et si le sérum est séparé de ce sang, les valeurs de quantification peuvent être celles mesurées dans le sérum.

Ainsi, chacune des valeurs de quantification desdites molécules circulantes, plus particulièrement de HA et CXCL10, peut être une valeur de concentration (ou proportion) sanguine, plasmique ou sérique, plus particulièrement sérique.

Avantageusement, les valeurs de quantification de toutes les molécules circulantes sélectionnées, et plus particulièrement de HA et CXCL10, sont mesurées dans le même échantillon, ou à tout le moins dans un échantillon de nature identique. Par exemple, les valeurs de quantification de chacune des molécules circulantes sélectionnées, et plus particulièrement de HA et CXCL10, sont toutes mesurées dans un échantillon de sérum qui n'a subi aucun traitement après collecte, ou bien sont toutes mesurées dans un échantillon de sérum qui a subi le même traitement après collecte (par exemple, le même traitement de purification).

Avantageusement, les valeurs de quantification de toutes les molécules circulantes sélectionnées, et plus particulièrement de HA et CXCL10, peuvent être mesurées dans un échantillon de sérum non dilué.

Avantageusement, les valeurs de quantification de toutes les molécules circulantes sélectionnées, et plus particulièrement de HA et CXCL10, peuvent être mesurées dans un seul et même échantillon biologique, plus particulièrement dans un seul et même échantillon de sérum, plus particulièrement dans un seul et même échantillon de sérum non dilué.

Dans la méthode de la demande, ladite étape de quantification peut notamment comprendre la détection *in vitro* de chacune des molécules circulantes sélectionnées comme marqueurs biologiques, telles que HA et CXCL10.

Selon un mode avantageux de réalisation, la détection de chacune des molécules circulantes sélectionnées comme marqueurs biologiques, plus particulièrement de HA et de CXCL10 (aux fins de leur quantification), sont faites en multiplex (détection simultanée dans un seul et même échantillon de fluide biologique) ; *cf.* exemple 10 ci-dessous. Certains marqueurs de l'art antérieur, tels que A2M, ont une concentration sérique très élevée : leur quantification ne peut donc être faite en multiplex avec des marqueurs dont la concentration sérique est beaucoup plus basse, tels que HA et CXCL10. Les combinaisons de biomarqueurs décrites dans la demande permettent quant à elles une mise en œuvre en multiplex.

Pour la quantification, plus particulièrement pour la détection et quantification, de chacune des molécules circulantes sélectionnées comme marqueurs biologiques, telles que HA et CXCL10, un ligand spécifique de cette molécule circulante peut être utilisé. Ce ligand peut directement porter un marqueur de détection (tel qu'un marqueur chimio-luminescent ou un fluorophore). Ce ligand peut être utilisé à titre de ligand de capture et/ou de ligand de détection.

Ce ligand peut par exemple être une molécule ou un complexe organique, ou une molécule ou un complexe inorganique. Par exemple, ce ligand peut être une protéine (plus particulièrement un anticorps, plus particulièrement un anticorps monoclonal ou un anticorps polyclonal), un polysaccharide, un lipide, ou un complexe de protéine(s) et/ou de polysaccharide(s) et/ou de lipide(s).

Pour la quantification, plus particulièrement pour la détection et quantification de HA dans le fluide biologique, des protéines qui se lient de manière spécifique à HA, telles qu'un anticorps (polyclonal ou monoclonal) anti-HA, ou telles que la protéine humaine recombinante aggrécane [protéine G1-IGD-G2 commercialisée par la société R&D SYSTEMS, Inc. (614 McKinley Place NE ; Minneapolis, MN 55413 ; U.S.A.), sous la référence catalogue 1220-PG-025], peuvent être utilisées à titre de ligands.

Pour la quantification, plus particulièrement pour la détection et la quantification de CXCL10 dans le fluide biologique, c'est-à-dire des formes circulantes de CXCL10, un ou des anticorps (polyclonaux ou monoclonaux) peuvent être utilisés à titre de ligands. Ce (ou ces) anticorps peuvent se lier de manière spécifique à une, à plusieurs ou à toutes les formes circulantes de CXCL10. Par exemple, peut être mis en œuvre un anticorps (polyclonal ou monoclonal) qui se lie de manière spécifique à une, à plusieurs ou à toutes les protéines de SEQ ID NO : 4 à 9, plus particulièrement à toutes les protéines de SEQ ID NO : 4 à 9 [par exemple, l'anticorps monoclonal de souris anti-CXCL10 humaine, commercialisé par R&D SYSTEMS, Inc. (614 McKinley Place NE ; Minneapolis, MN 55413 ; U.S.A.), sous la référence catalogue MAB266 (clone 33036, classe IgG1)].

Si les marqueurs biologiques comprennent en outre un ou plusieurs marqueur(s) additionnel(s), qui ne sont pas des molécules circulantes, plus particulièrement qui ne sont pas des molécules circulantes humaines (*cf.* ci-dessus), ce ou ces marqueur(s) additionnel(s) sont également quantifiés.

La valeur de quantification de ce, ou de chacun de ces, marqueur(s) additionnel(s) peut(peuvent) être déterminée en la mesurant ou en la collectant pour ou sur ledit sujet, par exemple en collectant la valeur de quantification de ce ou de chacun de ces marqueur(s) additionnel(s) préalablement mesurée pour ou sur ledit sujet.

Par exemple, si ce(ces) marqueur(s) additionnel(s) est(comprennent) le marqueur âge, la valeur de quantification de ce marqueur peut être déterminée (ou préalablement déterminée) en collectant la valeur de l'âge dudit sujet [par exemple, une valeur exprimée en nombre d'années (nombre entier ou avec décimale(s))].

Par exemple, si ce(ces) marqueur(s) additionnel(s) est(comprennent) le marqueur sexe, la valeur de quantification de ce marqueur peut être déterminée (ou préalablement déterminée) en affectant une valeur de quantification à la nature (féminine ou masculine) du sexe dudit sujet [par exemple, valeur de 0 pour un sexe féminin, et valeur de 1 pour un sexe masculin].

Par exemple, si ce(ces) marqueur(s) additionnel(s) est(comprennent) le marqueur Indice de Masse Corporelle, la valeur de quantification de ce marqueur peut être déterminée (ou préalablement déterminée) sur ledit sujet par mesure de la masse et de la taille dudit sujet (par exemple par mesure de la taille en m et de la masse en kg), pour calculer le rapport de la masse sur la taille au carré [IMC = masse divisée par (taille)², plus particulièrement masse en kg divisée par (taille en m)²].

Par exemple, si ce(ces) marqueur(s) additionnel(s) est(comprennent) le marqueur charge virale, la valeur de quantification de ce marqueur peut être déterminée (ou préalablement déterminée) en mesurant *in vitro* la valeur de la charge ou concentration en virus de l'hépatite (plus particulièrement, en VHC et/ou VHC et/ou VHD, plus particulièrement VHC) dans un échantillon de fluide biologique préalablement obtenu à partir dudit sujet (humain), plus particulièrement en mesurant *in vitro* la valeur de la quantité en virus de l'hépatite dans un échantillon de fluide biologique préalablement obtenu à partir dudit sujet (humain) et en déterminant la valeur de cette charge ou concentration virale. La valeur de cette charge en virus de l'hépatite peut par exemple être exprimée en copies/mL ou en UI/mL, ou en multiple de l'une de ces unités tel que en 10³ copies par mL.

La valeur de ce ou de chacun de ces marqueur(s) additionnelle(s) est avantageusement la valeur de ce marqueur à la date à laquelle ledit fluide biologique a été prélevé pour mesurer *in vitro* les concentrations de chacune desdites molécules circulantes (humaines), plus particulièrement à la date à laquelle ledit fluide biologique a été prélevé pour mesurer *in vitro* les concentrations de HA et de la protéine CXCL10.

### Détermination du stade de fibrose hépatique à partir des valeurs de quantification des marqueurs biologiques

Les valeurs de quantification des marqueurs biologiques sélectionnés (et notamment celles de HA et CXCL10) sont comparées à leurs valeurs, ou à la distribution de leurs valeurs, dans des cohortes de référence préétablies selon le stade de fibrose hépatique, pour classer ledit sujet dans celle de ces cohortes de référence vis-à-vis de laquelle il a la plus forte probabilité d'appartenance.

Lesdites cohortes de référence comprennent ou sont :
- une première cohorte de référence dans laquelle le stade de fibrose hépatique des individus ne dépasse pas ledit stade de fibrose légère (c'est-à-dire une première cohorte de référence dans laquelle le stade de fibrose hépatique des individus ne dépasse pas celui des stades de fibrose hépatique qui, selon le système de scores de fibrose Metavir, est de score F1), et
- une deuxième cohorte de référence dans laquelle le stade de fibrose hépatique des individus dépasse ledit stade de fibrose légère (c'est-à-dire une deuxième cohorte de référence dans laquelle le stade de fibrose hépatique des individus dépasse celui des stades de fibrose hépatique qui, selon le système de scores de fibrose Metavir, est de score F1).

En d'autres termes, les valeurs de quantification des marqueurs biologiques sélectionnés (et notamment celles de HA et CXCL10) sont comparées à une valeur de référence (« *cut-off* » ou « *threshold* »), prédéterminée pour la classification dudit sujet dans soit la première cohorte soit la deuxième cohorte.

Le classement dans la première cohorte détermine ou indique que le stade de fibrose hépatique dudit sujet n'a pas dépassé le stade de la fibrose portale sans septa.

Le classement dans la deuxième cohorte détermine ou indique que le stade de fibrose hépatique dudit sujet a dépassé le stade de la fibrose portale sans septa.

Selon un aspect de la demande, les individus desdites cohortes de référence sont des individus de la même espèce que ledit sujet [par exemple des humains, si ledit sujet est un humain], et sont infectés par un ou des virus de l'hépatite qui appartiennent à différents génotypes.

Par exemple, les individus desdites cohortes de référence sont des humains infectés par VHC, et les souches de VHC de ces individus appartiennent à au moins deux génotypes différents, plus particulièrement au moins trois, plus particulièrement au moins quatre, plus particulièrement au moins cinq, plus particulièrement au moins six, plus particulièrement au moins sept génotypes différents.

Par exemple, les individus desdites cohortes de référence sont des humains infectés par VHC, et les souches de VHC de ces individus appartiennent à au moins quatre génotypes différents, qui comprennent les génotypes 1, 2, 3 et 4.

Par exemple, les individus desdites cohortes de référence sont des humains infectés par VHC, et les souches de VHC de ces individus appartiennent à au moins cinq génotypes différents, qui comprennent les génotypes 1, 2, 3, 4 et 5.

Par exemple, les individus desdites cohortes de référence sont des humains infectés par VHC, et les souches de VHC de ces individus appartiennent à au moins six génotypes différents, qui comprennent les génotypes 1, 2, 3, 4, 5 et 6.

Les individus de ladite première cohorte de référence comprennent au moins des individus dont le stade de fibrose hépatique est celui de la fibrose sans septa (score F1 selon le système de scores de fibrose Metavir).

Les individus de ladite première cohorte de référence peuvent donc être constitués d'individus dont le stade de fibrose hépatique est celui de la fibrose portale sans septa [score F1 selon le système de scores de fibrose Metavir].

Les individus de ladite première cohorte de référence peuvent donc être constitués d'individus dont le stade de fibrose hépatique est celui de la fibrose portale sans septa et d'individus qui sont infectés par un ou plusieurs virus de l'hépatite et/ou atteint d'hépatite (plus particulièrement d'hépatite chronique), mais qui n'ont pas de fibrose hépatique [scores F0 et F1 selon le système de scores de fibrose Metavir].

Les individus de ladite deuxième cohorte de référence comprennent au moins des individus dont le stade de fibrose hépatique est celui de la fibrose portale avec septa(s) (score F2 selon le système de scores de fibrose Metavir).

Les individus de ladite deuxième cohorte de référence peuvent être constitués d'individus dont le stade de fibrose hépatique est celui de la fibrose portale sans septa [score F2 selon le système de scores de fibrose Metavir].

Les individus de ladite deuxième cohorte de référence peuvent être constitués d'individus dont le stade de fibrose hépatique est celui de la fibrose portale sans septa et d'individus dont le stade de fibrose hépatique est celui de la fibrose septale sans cirrhose [scores F2 et F3 selon le système de scores de fibrose Metavir].

Les individus de ladite deuxième cohorte de référence peuvent être constitués d'individus dont le stade de fibrose hépatique est celui de la fibrose portale sans septa, d'individus dont le stade de fibrose hépatique est celui de la fibrose septale sans cirrhose et d'individus dont le stade de fibrose hépatique est celui de la cirrhose [scores F2, F3 et F4 selon le système de scores de fibrose Metavir].

Le nombre total d'individus, qui forment ladite première cohorte de référence et ladite deuxième cohorte de référence, peut être d'au moins 100, plus particulièrement d'au moins 200, avantageusement d'au moins 300.

Chacune de ces deux cohortes est constituée d'une pluralité d'individus et représente 30% à 70% de la population totale des individus (le total des individus de ladite première cohorte de référence et de ladite deuxième cohorte de référence étant de 100%). Par exemple, le nombre des individus qui constituent ladite première cohorte de référence et ladite deuxième cohorte de référence est au total de 100, ladite première cohorte de référence est constituée de 30 individus et ladite deuxième cohorte de référence est constituée de 70 individus.

Plus particulièrement, chacune de ces deux cohortes est constituée d'une pluralité d'individus et représente 40% à 60% de la population totale des individus, plus particulièrement 40% à 55%, plus particulièrement 45% à 55%, plus particulièrement 45% à 50% de la population totale des individus (le total des individus de ladite première cohorte de référence et de ladite deuxième cohorte de référence étant de 100%). Par exemple, le nombre des individus qui constituent ladite première cohorte de référence et ladite deuxième cohorte de référence est au total de plus de 300, et ladite première cohorte de référence est constituée d'un nombre d'individus représentant 40% à 60% de cette population totale de plus de 300 individus (ladite deuxième cohorte de référence est donc alors constituée du nombre d'individus qui représente le pourcentage complémentaire pour arriver à un total de 100%).

Une méthode de la demande est donc une méthode (*in vitro*) pour déterminer (plus particulièrement pour déterminer avec une forte probabilité) si le stade de fibrose hépatique d'un sujet (humain) infecté par un ou plusieurs virus de l'hépatite et/ou atteint d'hépatite, plus particulièrement d'hépatite chronique, n'a pas dépassé le stade de la fibrose légère, ou s'il a au contraire dépassé ce stade, ledit stade de fibrose légère étant celui de la fibrose portale sans septa (selon le score Metavir de fibrose hépatique, le stade de fibrose portale sans septa est le score F1), ladite méthode comprenant les étapes suivantes :
i) dans un échantillon de fluide biologique préalablement obtenu à partir dudit sujet (humain), mesurer les quantités de plusieurs molécules circulantes (humaines) dans ledit échantillon, pour obtenir la valeur de la concentration de chacune desdites molécules circulantes, lesdites molécules circulantes (humaines) comprenant ou consistant en l'acide hyaluronique (HA) et la protéine CXCL10 (*cf.* «Molécules circulantes », «Marqueurs biologiques sélectionnés » et « Détection et quantification des marqueurs biologiques » ci-dessus),
ii) comparer les valeurs de concentrations ainsi obtenues pour chacune desdites molécules circulantes, à leurs valeurs, ou à la distribution de leurs valeurs, dans des cohortes de référence préétablies selon le stade (ou degré) de fibrose hépatique, pour classer ledit sujet dans celle de ces cohortes de référence vis-à-vis de laquelle il a la plus forte probabilité d'appartenance, lesdites cohortes de référence comprenant ou étant :
   une première cohorte de référence dans laquelle le stade de fibrose hépatique des individus ne dépasse pas ledit stade de fibrose légère, et
   une deuxième cohorte de référence dans laquelle le stade de fibrose hépatique des individus dépasse ledit stade de fibrose légère,
   le classement dans ladite première cohorte indiquant que le stade de fibrose hépatique dudit sujet n'a pas dépassé le stade de la fibrose portale sans septa,
   le classement dans ladite deuxième cohorte indiquant que le stade de fibrose hépatique dudit sujet a dépassé le stade de la fibrose portale sans septa.

Plus particulièrement, une méthode de la demande est une méthode (*in vitro*) pour déterminer (plus particulièrement pour déterminer avec une forte probabilité) si le stade de fibrose hépatique d'un sujet (humain) infecté par un ou plusieurs virus de l'hépatite et/ou atteint d'hépatite, plus particulièrement d'hépatite chronique, n'a pas dépassé le stade de la fibrose légère, ou s'il a au contraire dépassé ce stade, ledit stade de fibrose légère étant celui de la fibrose portale sans septa (selon le score Metavir de fibrose hépatique, le stade de fibrose portale sans septa est le score F1), ladite méthode comprenant les étapes suivantes :
i) sélectionner différents marqueurs (ou variables) biologiques, les différents marqueurs (ou variables) biologiques sélectionnés comprenant (ou consistant en) :
   a) différentes molécules circulantes (humaines), lesdites différentes molécules circulantes (humaines) comprenant ou consistant en HA et CXCL10 (*cf.* « Molécules circulantes », « Marqueurs biologiques sélectionnés » et « Détection et quantification des marqueurs biologiques » ci-dessus), et
   b) zéro, un, deux, trois ou quatre marqueur(s) additionnel(s) parmi la liste de marqueurs constituée par l'âge, l'Indice de Masse Corporelle (IMC), la charge virale (CV), et la dureté du foie (FS) (*cf.* « Éventuel(s) marqueur(s) additionnel(s) », « Marqueurs biologiques sélectionnés » et « Détection et quantification des marqueurs biologiques » ci-dessus),
ii) quantifier les différents marqueurs (ou variables) biologiques sélectionnés à l'étape i)
   en mesurant *in vitro* les concentrations de chacune desdites molécules circulantes (humaines) de l'étape i)a) ci-dessus, dans un échantillon de fluide biologique préalablement obtenu à partir dudit sujet, plus particulièrement en mesurant *in vitro* les quantités de chacune desdites molécules circulantes (humaines) de l'étape i)a) ci-dessus, dans un échantillon de fluide biologique préalablement obtenu à partir dudit sujet (humain) pour déterminer la valeur de la concentration de chacune desdites molécules circulantes [dans ledit échantillon et/ou dans ledit sujet], et
   lorsqu'un, deux, trois, quatre ou cinq marqueurs additionnel(s) est(sont) choisi(s) parmi ladite liste de l'étape i)b) ci-dessus, et lorsque ce (ou ces) marqueur(s) additionnel(s) est(sont) ou comprennent un ou plusieurs marqueur(s) parmi l'âge, l'Indice de Masse Corporelle et la dureté du foie : en collectant la valeur de quantification de ce ou chacun de ces marqueur(s) additionnel(s) qui a été préalablement déterminée pour ou sur ledit sujet,
   lorsqu'un, deux, trois, quatre ou cinq marqueurs additionnel(s) est(sont) choisi(s) parmi ladite liste de l'étape i)b) ci-dessus, et lorsque ce (ou ces) marqueur(s) additionnel(s) est(sont) ou comprennent la charge virale : en mesurant cette charge virale *in vitro* dans un échantillon de fluide biologique préalablement obtenu à partir dudit sujet, ou en collectant la valeur de cette charge virale qui a été préalablement déterminée pour ledit sujet,
iii) comparer les valeurs de quantification obtenues à l'étape ii) à leurs valeurs, ou à la distribution de leurs valeurs, dans des cohortes de référence préétablies selon le stade (ou degré) de fibrose hépatique, pour classer ledit sujet dans celle de ces cohortes de référence vis-à-vis de laquelle il a la plus forte probabilité d'appartenance, lesdites cohortes de référence comprenant ou étant :
   une première cohorte de référence dans laquelle le stade de fibrose hépatique des individus ne dépasse pas ledit stade de fibrose légère, et
   une deuxième cohorte de référence dans laquelle le stade de fibrose hépatique des individus dépasse ledit stade de fibrose légère,
   le classement dans ladite première cohorte indiquant que le stade de fibrose hépatique dudit sujet n'a pas dépassé le stade de la fibrose portale sans septa,
   le classement dans ladite deuxième cohorte indiquant que le stade de fibrose hépatique dudit sujet a dépassé le stade de la fibrose portale sans septa.

À ladite étape i)b), on sélectionne plus particulièrement zéro, un, deux ou trois marqueur(s) additionnel(s) parmi la liste de marqueurs constituée par l'âge, l'Indice de Masse Corporelle (IMC), la charge virale (CV) et la dureté du foie (FS), parmi cette liste (*cf.* « Éventuel(s) marqueur(s) additionnel(s) » ci-dessus).

Par exemple, on ne sélectionne aucun marqueur additionnel, ou l'on sélectionne le marqueur additionnel dureté du foie, ou les marqueurs additionnels âge et IMC, ou les marqueurs additionnels âge, IMC et charge virale.

Ainsi, les différents marqueurs biologiques sélectionnés à l'étape i) peuvent par exemple comprendre ou consister en :
- l'acide hyaluronique (HA) et la protéine CXCL10, ou
- l'acide hyaluronique (HA), la protéine CXCL10, l'âge et l'IMC, ou
- l'acide hyaluronique (HA), la protéine CXCL10, l'âge, l'IMC et la charge virale (CV), ou
- l'acide hyaluronique (HA), la protéine CXCL10 et la dureté du foie (FS) ;
*cf.* « Molécules circulantes », « Éventuel(s) marqueur(s) additionnel(s) », « Marqueurs biologiques sélectionnés » et « Détection et quantification des marqueurs biologiques » ci-dessus.

Ladite étape de comparaison des valeurs de quantification des marqueurs biologiques à leurs valeurs, ou à la distribution de leurs valeurs, dans des cohortes de référence préétablies selon le stade (ou degré) de fibrose hépatique, pour classer ledit sujet dans celle de ces cohortes de référence vis-à-vis de laquelle il a la plus forte probabilité d'appartenance, peut être réalisée par tout moyen que la personne du métier considère appropriée.

Cette comparaison peut notamment être faite par classification, plus particulièrement en combinant les valeurs de dosage (ou de mesure) obtenues pour ledit sujet dans un modèle de classification, plus particulièrement dans un modèle de classification multivariée.

Un tel modèle de classification compare (de manière combinée) les valeurs de dosage obtenues pour ledit sujet à leurs valeurs, ou à la distribution de leurs valeurs, dans des cohortes de référence pré-établies selon leur score de fibrose hépatique, pour classer ledit sujet dans celle de ces cohortes de référence vis-à-vis de laquelle il a la plus forte probabilité d'appartenance, par exemple en lui attribuant une valeur de sortie indicatrice du score de fibrose hépatique dudit sujet.

Un tel modèle de classification peut être construit, notamment préalablement construit, en faisant une comparaison inter-cohorte des valeurs de dosage obtenues pour lesdites cohortes de référence ou des distributions de ces valeurs de dosage.

Plus particulièrement, un tel modèle de classification peut être construit, notamment préalablement construit, en mesurant ou collectant les valeurs de quantification des marqueurs biologiques dans des cohortes de référence pré-établies selon leur score de fibrose hépatique, et en analysant ces valeurs de dosage, ou leur distribution, par une méthode statistique pour construire un modèle de classification, plus particulièrement un modèle de classification multivarié, qui induit ou détermine un score de fibrose hépatique à partir desdites valeurs de quantification.

Un modèle de classification peut par exemple être (préalablement) construit par :
- constitution d'au moins deux cohortes de référence préétablies selon le stade (ou degré ou score) de fibrose hépatique,
   une première cohorte de référence consistant en des individus, dont le stade de fibrose hépatique ne dépasse pas ledit stade de fibrose légère, et
   une deuxième cohorte de référence consistant en des individus, dont le stade de fibrose hépatique dépasse ledit stade de fibrose légère (*cf.* « Détermination du stade de fibrose hépatique à partir des valeurs de quantification des marqueurs biologiques » ci-dessus),
- quantification de chacun desdits différents marqueurs biologiques sélectionnés dans ladite première cohorte de référence et dans ladite deuxième cohorte de référence,
- comparaison (mathématique et/ou statistique) des valeurs de quantification que lesdits marqueurs prennent dans la première cohorte de référence à celles qu'ils prennent (respectivement) dans la deuxième cohorte de référence.

Plus particulièrement, ledit modèle de classification peut être (préalablement) construit comme suit :
α) pour une population d'individus qui sont de la même espèce que ledit sujet, et qui sont infectés par le ou les mêmes virus de l'hépatite que ledit sujet, déterminer le stade (ou degré ou score) de fibrose hépatique de chacun desdits individus de la population, et les classer en sous-populations selon leur stade (ou degré ou score) de fibrose hépatique, constituant ainsi des cohortes de référence établies selon leur stade (ou degré ou score) de fibrose hépatique, lesdites cohortes de référence comprenant ou étant :
   une première cohorte de référence dans laquelle le stade (ou degré ou score) de fibrose hépatique des individus ne dépasse pas le stade de la fibrose portale sans septa (c'est-à-dire ne dépassant pas le score de fibrose Metavir F1), et
   une deuxième cohorte de référence dans laquelle le stade (ou degré ou score) de fibrose hépatique des individus dépasse le stade de la fibrose portale sans septa (c'est-à-dire dépassant le score de fibrose Metavir F1) ;
β) pour chacun desdits individus, quantifier les différents marqueurs biologiques sélectionnés à l'étape i) de la revendication 1, et
γ) faire une comparaison inter-cohorte des valeurs de quantification obtenues à l'étape β), ou de la distribution de ces valeurs, pour construire un modèle de classification, qui, à partir de valeurs de quantification desdits marqueurs biologiques sélectionnés (plus particulièrement, à partir de la combinaison de ces valeurs), induit un classement dans l'une desdites cohortes de référence.

Ladite étape de comparaison peut être réalisée en classant les valeurs de quantification obtenues pour ledit sujet dans un modèle de classification, plus particulièrement un modèle de classification multivariée pour affecter audit sujet un score (Z) indiquant si le stade (ou degré) de fibrose hépatique dudit sujet a ou non dépassé ledit stade de fibrose légère.

Ledit modèle de classification peut être un modèle qui, à partir de la combinaison des valeurs de quantification desdits marqueurs biologiques, induit une valeur de score (Z) à partir de la combinaison des valeurs de quantification desdits marqueurs biologiques sélectionnés.

Plus particulièrement, ladite étape de comparaison peut être réalisée en appliquant aux valeurs de quantification obtenues pour ledit sujet une règle décisionnelle de classification préétablie pour classer ledit sujet dans celle desdites cohortes de référence vis-à-vis de laquelle il a la plus forte probabilité d'appartenance (plus particulièrement, pour classer ledit sujet dans ladite première cohorte de référence ou dans ladite deuxième cohorte de référence).

Cette règle décisionnelle peut être une règle qui compare les valeurs de quantification obtenues pour les marqueurs biologiques sélectionnés, à une ou des valeurs seuils prédéterminée(s) par classification, pour classer ledit sujet dans celle desdites cohortes de référence vis-à-vis de laquelle il a la plus forte probabilité d'appartenance (c'est-à-dire pour classer ledit sujet dans ladite première cohorte de référence ou dans ladite deuxième cohorte de référence).

Par exemple, un modèle peut être construit par une fonction mathématique, une technique non paramétrique, une procédure de classification heuristique ou encore une approche de prédiction probabiliste. Un exemple typique de classification fondée sur la quantification de marqueurs biologiques consiste en la discrimination de sujets "sains" *versus* "malades". La formalisation de ce problème consiste en *m* échantillons indépendants, décrits par *n* variables aléatoires. Chaque individu *i* (*i* =1,..., *m*) est caractérisé par un vecteur *xᵢ* décrivant les *n* valeurs caractéristiques: *xᵢⱼ*, *i* = 1,...*m j* = 1,...*n*. Ces valeurs caractéristiques peuvent par exemple représenter des valeurs de concentrations protéiques et/ou des données cliniques et/ou anatomiques et/ou des données virologiques. Chaque échantillon *xᵢ* est associé à une valeur discrète *yᵢ* représentant le statut clinique de l'individu *i.* A titre d'exemple, *yᵢ* = 0 si le patient *i* a un score de fibrose hépatique F1, *yᵢ* = 1 si le patient i a un score de fibrose hépatique F2. Un modèle offre une règle décisionnelle (par exemple, une fonction mathématique, un algorithme ou une procédure), qui utilise l'information disponible depuis *xᵢ* pour prédire *yⱼ* dans chaque échantillon observé. L'objectif est d'utiliser ce modèle afin de prédire le statut clinique du patient *p*, à savoir *yₚ,* à partir des valeurs biologiques et/ou cliniques disponibles, à savoir *xₚ.*

Différents modèles de classification sont connus de la personne du métier (*cf.* Hastie, Tibishirani et Friedman, 2009 ; Falissard, 2005 ; Theodoridis et Koutroumbos 2009). Les règles décisionnelles des modèles de classification multivariée peuvent par exemple être fondées sur une formule mathématique du type *y* = *f(x₁,x₂, ...xₙ) o*ù *f* est une fonction mathématique linéaire ou non linéaire (régression logistique, mROC, par exemple), ou sur un algorithme d'apprentissage automatique ou d'intelligence artificielle dont les caractéristiques consistent en une série de paramètres de réglage identifiés comme étant les plus efficaces pour la discrimination des sujets (par exemple, KNN, WKNN, SVM, RF). Tout modèle de classification que la personne du métier considère approprié peut être utilisé ou mis en œuvre.

Conformément à la demande, ladite règle décisionnelle de classification peut par exemple être :
- une méthode d'analyse statistique, plus particulièrement d'analyse statistique multivariée, par exemple :
   une méthode ROC (*Receiver Operating Characteristics*),
   une fonction mathématique linéaire ou non linéaire, notamment une fonction mathématique linéaire, tel qu'une fonction générée par la méthode mROC (méthode ROC multivariée), ou
   une méthode de régression, linéaire ou non linéaire, comme par exemple la régression logistique, plus particulièrement la régression logistique mettant en œuvre une fonction affine (LOGIT);
   une méthode PLS-DA (*Partial Least Squares* - *Discriminant Analysis*) ;
   une méthode LDA (*Linear Discriminant Analysis*) ;
- une méthode de classification par apprentissage ou intelligence artificielle, par exemple, un algorithme d'apprentissage ou d'intelligence artificielle, une méthode de classification non paramétrique, ou heuristique, ou de prédiction probabiliste, telle que :
   un arbre décisionnel, telle que la méthode CART (*Classification And Regression Tree*) ; ou
   une méthode de type *boosting*, fondée sur des classifieurs binaires (ex : *Adaboost*) ou une méthode liée au *boosting* (*bagging*) ; ou
   une méthode des k plus proches voisins (*k-nearest neighbors* ou KNN), ou plus généralement la méthode des k plus proches voisins pondérés (*weighed k-nearest neighbors* ou WKNN), ou
   une méthode (par exemple, un algorithme) Machines à Vecteurs de support (*Support Vector Machine* ou SVM) ; ou
   une forêt aléatoire (*Random Forest* ou RF); ou
   un réseau bayésien ; ou
   un réseau de neurones (*Neural Network*) ; ou
   un treillis de Galois (ou *Formal Concept Analysis*).

La méthode ROC multivariée (mROC) est une généralisation de la méthode ROC (*Receiver Operating Characteristic)* (*cf.* Reiser et Faraggi 1997 ; Su et Liu 1993, Shapiro, 1999). Elle calcule l'aire sous la courbe ROC (*Area Under the Curve* ou AUC) relative à une combinaison linéaire de biomarqueurs et/ou de transformations de biomarqueurs (dans le cas d'une normalisation), sous l'hypothèse d'une distribution normale multivariée. La méthode mROC a notamment été décrite dans Kramar *et al.* 1999 et Kramar *et al.* 2001. Le logiciel mROC version 1.0, disponible commercialement auprès des concepteurs (A. Kramar, A. Fortune, D. Farragi et B. Reiser) peut par exemple être utilisé pour construire un modèle mROC. Andrew Kramar et Antoine Fortune peuvent être contactés auprès de, ou *via,* l'Unité de Biostatistique du Centre Régional de Lutte contre le Cancer (CRLC) Val d'Aurelle - Paul Lamarque (208, rue des Apothicaires ; Parc Euromédecine ; 34298 Montpellier Cedex 5 ; France). David Faraggi et Benjamin Reiser peuvent être contactés auprès du, ou *via* le, Département de Statistique de l'Université de Haifa (Mount Carmel ; Haifa 31905 ; Israël). Un modèle mROC peut prendre la forme d'une fonction linéaire de type Z = a(BMQ₁) + b(BMQ₂) + ... ... + w(BMQn) avec BMQ = biomarqueur, n étant le nombre de biomarqueurs BMQ, BMQᵢ étant la valeur de quantification d'un des biomarqueurs sélectionnés (i allant de 1 à n) ou la valeur de la transformée Box-Cox de cette valeur de quantification [Box et Cox 1964 : pour un biomarqueur BMQ, la transformée Box-Cox de la valeur de quantification de BMQ est BMQ^{t}, qui est égal à (BMQ_{λ} - 1) / λ], et avec a, b, ... et w étant les paramètres de la fonction mROC.

La régression logistique (LR) mettant en œuvre une fonction affine, ou fonction LOGIT, est un modèle de régression binomiale (Berkson 1944 ; Berkson 1951). Un modèle de régression logistique peut prendre la forme d'une fonction affine de type LOGIT = Intercept + k(BMQ₁) + 1(BMQ₂) + ... + w(BMQn), avec BMQ = biomarqueur, n le nombre de biomarqueurs BMQ, BMQᵢ = la valeur de quantification d'un des biomarqueurs sélectionnés (i allant de 1 à n), et avec Intercept, k, 1, ... et z les paramètres de la fonction LOGIT.

La famille des méthodes d'intelligence artificielle ou d'apprentissage automatique est une famille d'algorithmes qui, au lieu de procéder à une généralisation explicite, comparent les exemples d'un nouveau problème avec les exemples considérés en apprentissage et qui ont été stockés en mémoire. Ces algorithmes construisent directement les hypothèses à partir des exemples d'apprentissage eux-mêmes.

Un exemple de ce type d'algorithme est l'algorithme CART dont l'acronyme signifie « Classification And Regression Trees » (Breiman 1984). Un modèle CART est un arbre de décision. Un exemple en est présenté en Figure 12. La valeur de quantification de chacun des biomarqueurs (BMQi) est comparée à une succession de valeurs seuils (paramètres h, i et j en Figure 12) qui, en suivant l'arbre de décision, permet de classer l'échantillon testé (en Figure 12 : score < F2, ou score ≥ F2).

Un exemple autre de ce type d'algorithme est l'algorithme des *k* plus proches voisins (*k-nearest neighbors* ou KNN), et une de ses extensions possibles connue sous le nom d'algorithme des *k* plus proches voisins pondérés (*weighted k nearest neighbors* ou WKNN) (Hechenbichler et Schliep, 2004). Dans le contexte de classification d'une nouvelle observation *x*, l'idée fondatrice simple est de faire voter les plus proches voisins de cette observation. La classe (ou statut clinique) de *x* est déterminée en fonction de la classe majoritaire parmi les *k* plus proches voisins de l'observation *x*. Des bibliothèques de fonctions spécifiques KKNN sont disponibles par exemple dans le logiciel R (http://www. R-project.org/). Le logiciel R a été initialement développé par John Chambers et les laboratoires Bell (*cf.* Chambers 2008). La version actuelle de cette suite logicielle est la version 2.11.1. Le code source est disponible librement sous les termes de la licence publique générale « Free Software Foundation's GNU », sur le site http://www. R-project.org/. Ce logiciel peut être utilisé pour construire un modèle WKNN.

Une Forêt Aléatoire (*Random Forest* ou RF) est constituée d'un ensemble d'arbres simples de prévision, chacun étant capable de produire une réponse lorsqu'on lui présente un sous-ensemble de prédicteurs (Breiman 2001 ; Liaw et Wiener 2002). Les calculs sont réalisés avec le logiciel R. Ce logiciel peut être utilisé pour construire des modèles RF.

Un réseau de neurones est constitué d'un graphe pondéré orienté dont les nœuds symbolisent les neurones. Le réseau est construit à partir d'exemples de chaque classe (par exemple, F2 versus F1), et est ensuite utilisé pour déterminer à quelle classe appartient un nouvel élément ; *cf.* Intrator et Intrator 1993, Riedmiller et Braun 1993, Riedmiller 1994, Anastasiadis *et. al.* 2005 ; *cf.* http://cran.r-project.org/web/packages/neuralnet/index.html. Le logiciel R librement disponible sur http://www.r-project.org/, (version 1.3 de *Neuralnet,* écrit par Stefan Fritsch et Frauke Guenther, suivant le travail de Marc Suling) peut par exemple être utilisé pour construire un réseau de neurones.

Conformément à la demande, la comparaison des valeurs de quantification des biomarqueurs sélectionnés, à leurs valeurs ou à la distribution de leurs valeurs dans des cohortes de référence préétablies selon le stade de fibrose hépatique, pour classer ledit sujet dans celle de ces cohortes de référence vis-à-vis de laquelle il a la plus forte probabilité d'appartenance, peut donc être notamment réalisée en suivant une méthode, et/ou en utilisant un algorithme ou un logiciel :
- qui est basé sur une fonction mathématique, tel que par exemple :
   - une fonction linéaire (par exemple, une fonction mROC) ou
   - une fonction non linéaire, telle que par exemple une fonction affine (par exemple, une régression logistique LOGIT), ou
- qui n'est pas basé sur une fonction mathématique, tel que par exemple une méthode, un logiciel, ou un algorithme par apprentissage ou intelligence artificielle (par exemple, un arbre de décision CART).

Plus particulièrement, ladite comparaison peut donc être notamment réalisée en suivant une méthode, et/ou en utilisant un algorithme ou un logiciel :
- mROC,
- de régression logistique (plus particulièrement de régression logistique mettant en œuvre une fonction affine LOGIT),
- KNN, WKNN (plus particulièrement WKNN),
- RF, ou
- NN, ou
- CART,
plus particulièrement mROC, régression logistique (plus particulièrement régression logistique mettant en œuvre une fonction affine LOGIT) ou CART.

Chacun de ces algorithmes, logiciels ou méthodes permet de construire un modèle de classification, à partir des valeurs de quantification de chacune desdites cohortes de référence, et de combiner les valeurs de quantification obtenues sur ledit sujet dans ce modèle pour en induire le classement ou le score de fibrose hépatique dudit sujet.

Les inventeurs démontrent que l'effet synergique observé avec la combinaison de CXCL10 et HA n'est pas dépendant du fait de mettre en œuvre une fonction mROC pour la classification du sujet ; *cf.* les exemples ci-dessous, notamment :
- l'exemple 7, plus particulièrement les Tableaux 20, 21 et 23,
- l'exemple 8, plus particulièrement le Tableau 24.

Selon un mode de réalisation, le modèle de classification mis en œuvre est un modèle de classification multivariée mettant en œuvre une fonction mathématique, tel que mROC ou la régression logistique (plus particulièrement régression logistique mettant en œuvre une fonction affine LOGIT).

Selon un mode de réalisation alternatif ou complémentaire, le modèle de classification mis en œuvre est un modèle par apprentissage automatique ou intelligence artificielle, tel que CART.

### Performances (sensibilité, VPN, spécificité, VPN, taux de bonne classification, AUC)

Les moyens de la demande, plus particulièrement la sélection de marqueurs biologiques de la demande, permettent d'atteindre de très bonnes performances de classement dudit sujet.

Ainsi, grâce aux moyens de la demande, ladite comparaison des valeurs de quantification dudit sujet à leurs valeurs, ou à la distribution de leurs valeurs, dans lesdites cohortes de référence est faite en classant ledit sujet dans celle desdites cohortes de référence vis-à-vis de laquelle il a la plus forte probabilité d'appartenance avec une sensibilité (Se) et/ou une Valeur de Prédiction Négative (VPN) et/ou une spécificité (Spé) et/ou une Valeur de Prédiction Positive (VPP) et/ou une aire sous la courbe ROC (AUC) et/ou un Taux de bonne classification (TauxBC) particulièrement élevé(s).

Les termes sensibilité (Se), spécificité (Spé), Valeur de Prédiction Positive (VPP), Valeur de Prédiction Négative (VPN), aire sous la courbe ROC (AUC) et Taux de bonne classification (TauxBC) sont entendus conformément à leur signification usuelle dans le domaine. Pour mémoire :
Se = VP / (VP + FN), avec VP = le nombre de vrais positifs et FN = le nombre de faux négatifs ;
Sp = VN / (VN + FP), avec VN = le nombre de vrais négatifs et FP = le nombre de faux positifs ;
VPP = VP / (VP + FP) avec VP = les Vrais Positifs et FP = Faux Positifs ;
VPN = VN / (VN+FN), avec VN = Vrais Négatifs et FN = Faux Négatifs ; avec
test positif = ledit stade de fibrose légère est dépassé et
test négatif = ledit stade de fibrose légère n'est pas dépassé.

VPP représente donc la probabilité que le sujet testé ait effectivement dépassé ledit stade de fibrose légère (score Metavir de fibrose hépatique d'au moins F2), sachant que les moyens de la demande indiquent qu'il a dépassé ledit stade de fibrose légère (score Metavir de fibrose hépatique d'au moins F2) [résultat positif du test].

VPN représente donc la probabilité que le sujet testé n'ait effectivement pas dépassé ledit stade de fibrose légère (score Metavir de fibrose hépatique d'au plus F1), sachant que les moyens de la demande indiquent qu'il n'a pas dépassé ledit stade de fibrose légère (score Metavir de fibrose hépatique d'au moins F2) [résultat négatif du test].

Le Taux de bonne classification est le pourcentage de patients correctement classés.

L'aire sous la courbe ROC (AUC) est l'aire sous la courbe ROC (*Area Under the Curve* ou AUC) relative à une combinaison linéaire de biomarqueurs et/ou de transformations de biomarqueurs (dans le cas d'une normalisation), sous l'hypothèse d'une distribution normale multivariée.

Ladite comparaison des valeurs de quantification dudit sujet à leurs valeurs, ou à la distribution de leurs valeurs, dans lesdites cohortes de référence peut être faite en suivant un modèle de classification tel que ci-dessus décrit.

Plus particulièrement, ladite comparaison peut être faite selon une fonction mROC, une fonction LOGIT ou un arbre CART, notamment une fonction mROC Z₁ à Z₁₃, une fonction LOGIT₁ à LOGIT₄, un arbre CART₁ à CART₄ telles que ci-dessous décrites.

Conformément à la demande, ladite comparaison est faite (par exemple par mROC, régression logistique ou arbre CART) en classant ledit sujet avec une sensibilité (Se) d'au moins 70%, d'au moins 75%, d'au moins 76%, d'au moins 77%, d'au moins 78%, d'au moins 80%, d'au moins 82% ou d'au moins 83%.

Plus particulièrement, ladite comparaison peut être faite (par exemple par mROC, régression logistique ou arbre CART) en classant ledit sujet avec une sensibilité (Se) d'au moins 76%, d'au moins 77%, d'au moins 78%, d'au moins 80%, d'au moins 82% ou d'au moins 83%.

Selon un mode alternatif ou complémentaire de réalisation, ladite comparaison est faite (par exemple par mROC, régression logistique ou arbre CART) en classant ledit sujet avec une Valeur de Prédiction Négative (VPN) d'au moins 70%, d'au moins 75%, d'au moins 76%, d'au moins 77%, d'au moins 78%, d'au moins 80%, d'au moins 81%, d'au moins 82% ou d'au moins 83%.

Plus particulièrement, ladite comparaison est faite (par exemple par mROC, régression logistique ou arbre CART) en classant ledit sujet avec une Valeur de Prédiction Négative (VPN) d'au moins 75%, d'au moins 76%, d'au moins 77%, d'au moins 78%, d'au moins 80%, d'au moins 81%, d'au moins 82% ou d'au moins 83%.

Selon un mode alternatif ou complémentaire de réalisation, ladite comparaison est faite (par exemple par mROC, régression logistique ou arbre CART) en classant ledit sujet avec une spécificité d'au moins 59%, d'au moins 70%, d'au moins 71%, d'au moins 80%, d'au moins 85%, d'au moins 86%, d'au moins 87%, d'au moins 89%, d'au moins 90% ou d'au moins 91%.

Plus particulièrement, ladite comparaison peut être faite (par exemple par mROC, régression logistique ou arbre CART) en classant ledit sujet avec une spécificité d'au moins 85%, d'au moins 86%, d'au moins 87%, d'au moins 89%, d'au moins 90% ou d'au moins 91%.

Selon un mode alternatif ou complémentaire de réalisation, ladite comparaison est faite (par exemple par mROC, régression logistique ou arbre CART) en classant ledit sujet avec une Valeur de Prédiction Positive (VPP) d'au moins 54%, d'au moins 61%, d'au moins 62%, d'au moins 85%, d'au moins 86%, d'au moins 87%, d'au moins 88%, d'au moins 90% ou d'au moins 91%.

Plus particulièrement, ladite comparaison peut être faite (par exemple par mROC, régression logistique ou arbre CART) en classant ledit sujet avec une Valeur de Prédiction Positive (VPP) d'au moins 85%, d'au moins 86%, d'au moins 87%, d'au moins 88%, d'au moins 90% ou d'au moins 91%.

Toutes les combinaisons de valeur minimale de sensibilité et/ou de VPN et/ou de spécificité et/ou de VPP sont explicitement inclues dans la description de la demande.

Ainsi, selon un mode alternatif ou complémentaire de réalisation, ladite comparaison est faite (par exemple par mROC, régression logistique ou arbre CART) en classant ledit sujet avec une sensibilité (Se) d'au moins 76% (comme ci-dessus décrit) et/ou avec une Valeur de Prédiction Négative (VPN) d'au moins 75% (comme ci-dessus décrit).

Selon un mode alternatif ou complémentaire de réalisation, ladite comparaison est faite (par exemple par mROC, régression logistique ou arbre CART) en classant ledit sujet avec une sensibilité (Se) d'au moins 76% (comme ci-dessus décrit) et/ou une spécificité (Spé) d'au moins 59% (comme ci-dessus décrit).

Selon un mode alternatif ou complémentaire de réalisation, ladite comparaison est faite (par exemple par mROC, régression logistique ou arbre CART) en classant ledit sujet avec une sensibilité (Se) d'au moins 76% (comme ci-dessus décrit) et/ou une spécificité (Spé) d'au moins 85%(comme ci-dessus décrit).

Selon un mode alternatif ou complémentaire de réalisation, ladite comparaison est faite (par exemple par mROC, régression logistique ou arbre CART) en classant ledit sujet avec une sensibilité (Se) d'au moins 76% (comme ci-dessus décrit) et/ou une VPP d'au moins 54% (comme ci-dessus décrit).

Selon un mode alternatif ou complémentaire de réalisation, ladite comparaison est faite (par exemple par mROC, régression logistique ou arbre CART) en classant ledit sujet avec une sensibilité (Se) d'au moins 76% (comme ci-dessus décrit) et/ou une VPP d'au moins 85% (comme ci-dessus décrit).

Selon un mode alternatif ou complémentaire de réalisation, ladite comparaison est faite (par exemple par mROC, régression logistique ou arbre CART) en classant ledit sujet avec une spécificité (Spé) d'au moins 54% (comme ci-dessus décrit) et/ou avec une VPP d'au moins 54% (comme ci-dessus décrit).

Selon un mode alternatif ou complémentaire de réalisation, ladite comparaison est faite (par exemple par mROC, régression logistique ou arbre CART) en classant ledit sujet avec une spécificité (Spé) d'au moins 54% (comme ci-dessus décrit) et/ou avec une VPP d'au moins 85% (comme ci-dessus décrit).

Selon un mode alternatif ou complémentaire de réalisation, ladite comparaison est faite (par exemple par mROC, régression logistique ou arbre CART) en classant ledit sujet avec une spécificité (Spé) d'au moins 85% (comme ci-dessus décrit) et/ou avec une VPP d'au moins 54% (comme ci-dessus décrit).

Selon un mode alternatif ou complémentaire de réalisation, ladite comparaison est faite (par exemple par mROC, régression logistique ou arbre CART) en classant ledit sujet avec une spécificité (Spé) d'au moins 85% (comme ci-dessus décrit) et/ou avec une VPP d'au moins 85% (comme ci-dessus décrit).

Selon un mode alternatif ou complémentaire de réalisation, ladite comparaison est faite (par exemple par mROC, régression logistique ou arbre CART) en classant ledit sujet avec une spécificité (Spé) d'au moins 54% (comme ci-dessus décrit) et/ou avec une VPN d'au moins 75% (comme ci-dessus décrit).

Selon un mode alternatif ou complémentaire de réalisation, ladite comparaison est faite (par exemple par mROC, régression logistique ou arbre CART) en classant ledit sujet avec une spécificité (Spé) d'au moins 85% (comme ci-dessus décrit) et/ou avec une VPN d'au moins 75% (comme ci-dessus décrit).

Selon un mode alternatif ou complémentaire de réalisation, ladite comparaison est faite (par exemple par mROC, régression logistique ou arbre CART) en classant ledit sujet avec une VPN d'au moins 75% (comme ci-dessus décrit) et/ou une VPP d'au moins 54% (comme ci-dessus décrit).

Selon un mode alternatif ou complémentaire de réalisation, ladite comparaison est faite (par exemple par mROC, régression logistique ou arbre CART) en classant ledit sujet avec une VPN d'au moins 75% (comme ci-dessus décrit) et/ou une VPP d'au moins 85% (comme ci-dessus décrit).

Selon un mode alternatif ou complémentaire de réalisation, ladite comparaison est faite (par exemple par mROC, régression logistique ou arbre CART) en classant ledit sujet avec une sensibilité (Se) d'au moins 76% (comme ci-dessus décrit) et avec une VPN d'au moins 75% (comme ci-dessus décrit), ainsi qu'avec :
- une spécificité (Spé) d'au moins 85%, et/ou
- une VPP d'au moins 85%.

Grâce aux moyens de la demande, ladite comparaison peut être faite (par exemple par mROC ou régression logistique) en classant ledit sujet avec une aire sous la courbe ROC (AUC) d'au moins 0,700, d'au moins 0,704, d'au moins 0,720, d'au moins 0,729, d'au moins 0,730, d'au moins 0,731, d'au moins 0,840, d'au moins 0,844, d'au moins 0,860, d'au moins 0,865, d'au moins 0,868, d'au moins 0,880, d'au moins 0,882, d'au moins 0,890, d'au moins 0,895, d'au moins 0,898, d'au moins 0,899, d'au moins 0,0900, d'au moins 0,910, d'au moins 0,920, d'au moins 0,921, d'au moins 0,930, d'au moins 0,931, ou d'au moins 0,933.

En effet, la combinaison des deux seuls marqueurs HA et CXCL10 suffit à atteindre une AUC d'au moins 0,700, plus particulièrement d'au moins 0,704, plus particulièrement d'au moins 0,865 (*cf.* les exemples ci-dessous).

Plus particulièrement, ladite comparaison peut être faite, par exemple par mROC ou régression logistique, en classant ledit sujet avec une AUC d'au moins 0,800, plus particulièrement d'au moins 0,865, d'au moins 0,868, d'au moins 0,880, d'au moins 0,882, d'au moins 0,890, d'au moins 0,895 ou d'au moins 0,898.

Grâce aux moyens de la demande, ladite comparaison peut être faite (par exemple par mROC, régression logistique ou arbre CART) en classant ledit sujet avec un Taux de bonne classification (TauxBC) d'au moins 60%, d'au moins 66%, d'au moins 70%, d'au moins 72%, d'au moins 73%, d'au moins 75%, d'au moins 77%, d'au moins 80%, d'au moins 81%, d'au moins 82% , d'au moins 83%, d'au moins 85%, d'au moins 86%, d'au moins 88% ou d'au moins 89%.

Plus particulièrement, ladite comparaison peut être faite (par exemple par mROC, régression logistique ou arbre CART) en classant ledit sujet avec un Taux de bonne classification (TauxBC) d'au moins 80%, d'au moins 81%, d'au moins 82% , d'au moins 83%, d'au moins 85%, d'au moins 86%, d'au moins 88% ou d'au moins 89%.

Toutes les combinaisons de valeur minimale de sensibilité et/ou de VPN et/ou de spécificité et/ou de VPP sont explicitement inclues dans la description de la demande.

Ainsi, selon un mode alternatif ou complémentaire de réalisation, ladite comparaison est faite (par exemple par mROC, régression logistique ou arbre CART) en classant ledit sujet avec un taux de bonne classification d'au moins 80% et/ou une AUC d'au moins 0,704.

Selon un mode alternatif ou complémentaire de réalisation, ladite comparaison est faite (par exemple par mROC, régression logistique ou arbre CART) en classant ledit sujet avec un taux de bonne classification d'au moins 70% et/ou une AUC d'au moins 0,800, plus particulièrement avec un taux de bonne classification d'au moins 80% et/ou une AUC d'au moins 0,800.

Toutes les combinaisons de valeurs minimales :
- sensibilité et spécificité, ou de
- sensibilité et VPP, ou de
- sensibilité et VPN, ou de
- sensibilité et AUC, ou de
- sensibilité et taux de bonne classification, ou de
- spécificité et VPP, ou de
- spécificité et VPN, ou de
- spécificité et AUC, ou de
- spécificité et taux de bonne classification, ou de
- VPP et VPN, ou de
- VPP et AUC, ou de
- VPP et taux de bonne classification, ou de
- VPN et AUC, ou de
- VPN et taux de bonne classification, ou de
- AUC et taux de bonne classification, ou de
- sensibilité, spécificité et VPP, ou de
- sensibilité, spécificité et VPN, ou de
- sensibilité, spécificité et AUC, ou de
- sensibilité, spécificité et taux de bonne classification, ou de
- sensibilité, VPP et VPN, ou de
- sensibilité, VPP et AUC, ou de
- sensibilité, VPP et taux de bonne classification, ou de
- sensibilité, VPN et AUC, ou de
- sensibilité, VPN et taux de bonne classification, ou de
- sensibilité, AUC et taux de bonne classification, ou de
- sensibilité, spécificité, VPP et VPN, ou de
- sensibilité, spécificité, VPP et AUC, ou de
- sensibilité, spécificité, VPP et taux de bonne classification, ou de
- sensibilité, spécificité, VPN et AUC, ou de
- sensibilité, spécificité, VPN et taux de bonne classification, ou de
- sensibilité, VPP, VPN et taux de bonne classification, ou de
- spécificité, VPP, VPN et AUC, ou de
- spécificité, VPP, VPN et taux de bonne classification, ou de
- sensibilité, spécificité, VPP, VPN et AUC, ou de
- sensibilité, spécificité, VPP, VPN et taux de bonne classification, ou de
- spécificité, VPP, VPN, AUC et taux de bonne classification, ou de
- sensibilité, spécificité, VPP, VPN, AUC et taux de bonne classification,
sont explicitement inclues dans la description de la demande.

Par exemple, ladite comparaison peut être faite (par exemple par mROC, régression logistique ou arbre CART) avec au moins l'une des deux performances 1/ et 2/ ci-dessous :
1/ une spécificité d'au moins 59% et/ou une Valeur de Prédiction Positive d'au moins 54%, et/ou
2/ un taux de bonne classification d'au moins 66% et/ou une aire sous la courbe ROC d'au moins 0,704.

Plus particulièrement, ladite comparaison peut être faite (par exemple par mROC, régression logistique ou arbre CART) avec au moins l'une des deux performances 1/ et 2/ ci-dessous :
1/ une spécificité d'au moins 85% et/ou une Valeur de Prédiction Positive d'au moins 85%, et/ou
2/ un taux de bonne classification d'au moins 80% et/ou une aire sous la courbe ROC d'au moins 0,800.

Une règle décisionnelle de la demande (qu'elle soit mROC, CART, LR ou autre, *cf.* ci-dessous), plus particulièrement une fonction mROC répondant à la formule de la fonction Z₁₀, Z₁₁, Z₁₂ ou Z₁₃ (*cf.* ci-dessous), a un taux de patients non classés de 0%.

En outre, une règle décisionnelle de la demande (qu'elle soit mROC, CART, LR ou autre), plus particulièrement une fonction mROC répondant à la formule de la fonction Z₁₀, Z₁₁, Z₁₂ ou Z₁₃, a au moins l'une de ses performances d'AUC, de Taux de bonne classification, de sensibilité, de VPN, de spécificité et de VPP, qui est supérieure :
- tant à celle obtenue dans les mêmes conditions mais sans le marqueur HA,
- qu'à celle obtenue dans les mêmes conditions mais sans le marqueur CXCL10.

Les inventeurs démontrent en effet que la combinaison du marqueur CXCL10 au marqueur HA conduit à un effet synergique, à savoir à des performances qui vont au-delà de la simple juxtaposition de leurs performances individuelles respectives (performances d'AUC et/ou Taux de bonne classification et/ou sensibilité et/ou VPN et/ou spécificité et/ou VPP). Des démonstrations expérimentales sont présentées dans les exemples ci-dessous, notamment :
- en exemple 1 (*cf.* Tableau 3),
- en exemple 3 (*cf.* Tableaux 8 et 10), et
- en exemple 7 (*cf.* Tableaux 20 et 21).

Pour réaliser ladite étape de comparaison des valeurs de quantification des marqueurs biologiques dudit sujet à leurs valeurs, ou à la distribution de leurs valeurs, dans des cohortes de référence préétablies selon le stade (ou degré) de fibrose hépatique, pour classer ledit sujet dans celle de ces cohortes de référence vis-à-vis de laquelle il a la plus forte probabilité d'appartenance, différents moyens de classification tels que mROC (fonction linéaire), régression logistique (fonction affine) ou arbre décisionnel (arbre CART par exemple) peuvent être mis en oeuvre (*cf.* ci-dessus).

Plus particulièrement, les inventeurs démontrent que l'effet synergique observé avec la combinaison de CXCL10 et HA n'est pas dépendant du fait de mettre en oeuvre une fonction mROC pour la classification du sujet ; *cf.* les exemples ci-dessous, notamment :
- l'exemple 7, plus particulièrement les Tableaux 20, 21 et 23,
- l'exemple 8, plus particulièrement le Tableau 24.

### Fonction mathématique (par exemple, mROC, régression logistique)

Une fonction mathématique peut être mise en oeuvre pour réaliser ladite étape de comparaison des valeurs de quantification des marqueurs biologiques dudit sujet à leurs valeurs, ou à la distribution de leurs valeurs, dans des cohortes de référence préétablies selon le stade (ou degré) de fibrose hépatique, pour classer ledit sujet dans celle de ces cohortes de référence vis-à-vis de laquelle il a la plus forte probabilité d'appartenance. La comparaison est alors faite en combinant les valeurs de quantification de chacun des marqueurs biologiques sélectionnés dans cette fonction mathématique. Cette fonction mathématique peut affecter audit sujet un score (Z), qui est alors comparé à une valeur seuil (δ ou *cut-off*) prédéterminée par classification multivariée, pour classer ledit sujet dans celle desdites cohortes de référence vis-à-vis de laquelle il a la plus forte probabilité d'appartenance (plus particulièrement, pour classer ledit sujet dans ladite première cohorte de référence ou dans ladite deuxième cohorte de référence).

Ladite fonction mathématique peut par exemple être une fonction linéaire telle qu'une fonction mROC ou une fonction non linéaire, par exemple une fonction affine telle qu'une fonction de régression logistique mettant en oeuvre une fonction affine LOGIT.

Une fonction mROC de la demande peut avoir la forme suivante Z = a(BMQ₁^{t}) + b(BMQ₂^{t}) + c(BMQ₃^{t}) + d(BMQ₄^{t}) + e(BMQ₅^{t}), dans laquelle BMQ₁, BMQ₂, BMQ₃, BMQ₄ et BMQ₅ sont cinq biomarqueurs différents (par exemple CXCL10, HA, IMC, âge et charge virale), dans laquelle a, b, c, d et e sont les valeurs numériques des constantes de la fonction mROC, et dans laquelle l'exposant t indique que la valeur à appliquer dans la fonction linéaire est la transformée Box-Cox (Box et Cox 1964) de la valeur de quantification mesurée pour le biomarqueur (BMQ), afin de normaliser cette valeur mesurée selon la formule suivante : BMQ^{t} = (BMQ^{λ}) - 1) / λ.

Conformément à la demande, une fonction mROC peut être formulée comme suit : $Z = a \left(CXCL{10}^{t}\right) + b \left({HA}^{t}\right) + c \left({IMC}^{t}\right) + d \left({Âge}^{t}\right) + e \left({CV}^{t}\right) + f \left({FS}^{t}\right)$ avec :
a et b étant chacun, indépendamment l'un de l'autre, un nombre réel positif allant de +0,1 à +6,0,
c, d, e, et f étant chacun, indépendamment les uns des autres, un nombre réel allant de - 10,0 à +10,0,
λ_{CXCL10}, λ_{HA}, λ_{IMC}, λ_{Âge}, λ_{CV} et λ_{FS} étant chacun, indépendamment les uns des autres, un nombre réel allant de -6,0 à 1,2 mais différent de zéro ;
*cf.* exemple 8.

Dans la fonction Z₁₃ ci-dessus, comme toutes les fonctions mROC ici indiquées, l'exposant t indique, conformément à l'usage en la matière, que la valeur à appliquer dans la fonction linéaire est la transformée Box-Cox (Box et Cox 1964) de la valeur de quantification mesurée pour le biomarqueur (BMQ), afin de normaliser cette valeur mesurée selon la formule suivante : BMQ^{t} = (BMQ^{λ}) - 1) / λ.

Les fonctions mROC de la demande, telles que la fonction Z₁₃ ci-dessus, ainsi que les fonctions Z₁₂ à Z₁ décrites ci-dessous, présentent les performances qui ont été décrites ci-dessus (performances d'AUC et/ou Taux de bonne classification et/ou sensibilité et/ou VPN et/ou spécificité et/ou VPP).

Les inventeurs démontrent en outre que les performances de la combinaison de CXCL10 à HA sont particulièrement robustes (performances d'AUC et/ou Taux de bonne classification et/ou sensibilité et/ou VPN et/ou spécificité et/ou VPP), dès lors que les paramètres de la fonction mROC sont choisis dans les plages de valeurs indiquées : *cf.* exemple 9 ci-dessous.

Un exemple de fonction Z₁₃ est le suivant : $Z = a \left(CXCL{10}^{t}\right) + b \left({HA}^{t}\right) + c \left({IMC}^{t}\right) + d \left({Âge}^{t}\right) + e \left({CV}^{t}\right) + f \left({FS}^{t}\right)$ avec :
a et b étant chacun, indépendamment l'un de l'autre, un nombre réel positif allant de +0,1 à +6,0, plus particulièrement de +0,3 à +5,5,
c étant un nombre réel allant de -10,0 à +4,0,
d étant un nombre réel allant de -0,8 à +0,2,
e étant un nombre réel allant de -0,003 à +0,002,
f étant un nombre réel allant de +0,0 à +10,0,
λ_{CXCL10}, λ_{HA}, λ_{IMC}, λ_{Âge}, λ_{CV} et λ_{FS} étant chacun, indépendamment les uns des autres, un nombre réel allant de -6,0 à 1,2 mais différent de zéro ;
*cf.* exemple 8 ci-dessous.

La valeur seuil de chacune des fonctions mROC de la demande, plus particulièrement de Z₁₃ et Z₁₂, peut par exemple être de -7 à 25, plus particulièrement de 3 à 25, plus particulièrement de 10 à 25.

Une fonction mROC de la demande, plus particulièrement Z₁₃ et Z₁₂, peut par exemple avoir un taux de bonne classification d'au moins 66% et/ou une valeur d'aire sous la courbe ROC (AUC) d'au moins 0,704, plus particulièrement un taux de bonne classification d'au moins 72% et/ou une valeur d'aire sous la courbe ROC (AUC) d'au moins 0,729, plus particulièrement un taux de bonne classification d'au moins 80% et une valeur d'AUC d'au moins 0,865, plus particulièrement un taux de bonne classification d'au moins 80% et/ou une valeur d'aire sous la courbe ROC (AUC) d'au moins 0,898.

Une fonction mROC de la demande, plus particulièrement Z₁₃ et Z₁₂, peut par exemple avoir plus une sensibilité d'au moins 70% et/ou une VPN d'au moins 70%, particulièrement une sensibilité d'au moins 75% et/ou une VPN d'au moins 75%, plus particulièrement une sensibilité d'au moins 76% et/ou une VPN d'au moins 75%.

Une fonction mROC de la demande, plus particulièrement Z₁₃ et Z₁₂, peut par exemple avoir une spécificité d'au moins 59% et/ou une VPP d'au moins 54%, plus particulièrement une spécificité d'au moins 70% et/ou une VPP d'au moins 61%, plus particulièrement une spécificité d'au moins 85% et/ou une VPP d'au moins 85%, plus particulièrement une spécificité d'au moins 86% et/ou une VPP d'au moins 86%.

Un exemple de fonction Z₁₃, qui est également un exemple de fonction Z₁₂, est le suivant : $Z = a \left(CXCL{10}^{t}\right) + b \left({HA}^{t}\right)$ avec $0,812 \leq a \leq 5,089$ $2,033 \leq b \leq 4,462$ $- 0,262 \leq {λ}_{CXCL 10} \leq 0,030$ $- 0,382 \leq {λ}_{HA} \leq - 0,219$ a, b, λ_{CXCL10}, et λ_{HA} étant différents de zéro.

La valeur seuil de Z₁₀ peut par exemple être de -7 à 25, plus particulièrement de 3 à 25, plus particulièrement de 10 à 25, plus particulièrement de 11,68 à 23,71.

Des illustrations de fonctions Z₁₀ sont présentées en exemples 3, 5 et 8 ci-dessous (fonctions Z₁₀ et Z₄).

Une fonction Z₁₀ peut par exemple avoir un taux de bonne classification d'au moins 66% et/ou une valeur d'AUC d'au moins 0,704, plus particulièrement un taux de bonne classification d'au moins 72% et une valeur d'AUC d'au moins 0,729, plus particulièrement un taux de bonne classification d'au moins 80% et une valeur d'AUC d'au moins 0,865, plus particulièrement un taux de bonne classification d'au moins 80% et une valeur d'AUC d'au moins 0,898, plus particulièrement un taux de bonne classification d'au moins 83% et une valeur d'AUC d'au moins 0,868 (*cf.* exemples 3, 5 et 8 ci-dessous).

Une fonction Z₁₀ peut par exemple avoir une sensibilité d'au moins 70% et/ou une VPN d'au moins 70%, particulièrement une sensibilité d'au moins 75% et/ou une VPN d'au moins 75%, plus particulièrement une sensibilité d'au moins 76% et/ou une VPN d'au moins 75%, plus particulièrement une sensibilité d'au moins 78% et/ou une VPN d'au moins 77% (*cf.* exemples 1, 3, 5 et 6 ci-dessous).

Une fonction Z₁₀ peut par exemple avoir une spécificité d'au moins 59% et/ou une VPP d'au moins 54%, plus particulièrement une spécificité d'au moins 70% et/ou une VPP d'au moins 61%, plus particulièrement une spécificité d'au moins 85% et/ou une VPP d'au moins 85%, plus particulièrement une spécificité d'au moins 86% et/ou une VPP d'au moins 86%, plus particulièrement une spécificité d'au moins 90% et/ou une VPP d'au moins 90% (*cf.* exemples 1, 3, 5 et 6 ci-dessous).

Un exemple de fonction Z₁₀ est la fonction : $Z = \left(1,999\right) \times CXCL {10}^{t} + \left(2,852\right) \times {HA}^{t}$ avec λ_{CXCL10} = -0,116 et avec λ_{HA} = -0,288.

La valeur seuil de Z₄ peut par exemple être de -7 à 25, plus particulièrement de 3 à 25, plus particulièrement de 10 à 25, plus particulièrement de 15,170.

Une fonction Z₄ peut par exemple avoir un taux de bonne classification d'au moins 66% et/ou une valeur d'AUC d'au moins 0,704, plus particulièrement un taux de bonne classification d'au moins 72% et une valeur d'AUC d'au moins 0,729, plus particulièrement un taux de bonne classification d'au moins 80% et une valeur d'AUC d'au moins 0,865, plus particulièrement un taux de bonne classification d'au moins 80% et une valeur d'AUC d'au moins 0,882, plus particulièrement un taux de bonne classification d'au moins 83% et une valeur d'AUC d'au moins 0,898 (*cf.* exemples 3, 5 et 8 ci-dessous).

Une fonction Z₄ peut par exemple avoir une sensibilité d'au moins 70% et/ou une VPN d'au moins 70%, particulièrement une sensibilité d'au moins 75% et/ou une VPN d'au moins 75%, plus particulièrement une sensibilité d'au moins 76% et/ou une VPN d'au moins 75%, plus particulièrement une sensibilité d'au moins 78% et/ou une VPN d'au moins 77% (*cf.* exemples 3 et 5 ci-dessous).

Une fonction Z₄ peut par exemple avoir une spécificité d'au moins 59% et/ou une VPP d'au moins 54%, plus particulièrement une spécificité d'au moins 70% et/ou une VPP d'au moins 61%, plus particulièrement une spécificité d'au moins 85% et/ou une VPP d'au moins 85%, plus particulièrement une spécificité d'au moins 86% et/ou une VPP d'au moins 86%, plus particulièrement une spécificité d'au moins 90% et/ou une VPP d'au moins 90% (*cf.* exemples 3 et 5 ci-dessous).

Un autre exemple de fonction Z₁₃ est la fonction : $Z = 0,386 \times CXCL {10}^{t} + 0,3064 \times {HA}^{t}$ avec λ_{CXCL10} = -0,013 et λ_{HA} = 0,099.

La valeur seuil de Z₁ peut par exemple être de -7 à 25, plus particulièrement de 3 à 25, plus particulièrement de 3 à 5, plus particulièrement de 3,382.

La fonction Z₁ peut par exemple avoir un taux de bonne de classification d'au moins 66% et/ou une AUC d'au moins 0,704 (*cf.* exemple 1 ci-dessous).

La fonction Z₁ peut par exemple avoir une sensibilité d'au moins 78% et/ou une VPN d'au moins 81% (*cf.* exemple 1 ci-dessous).

La fonction Z₁ peut par exemple avoir une spécificité d'au moins 59% et/ou une VPP d'au moins 54% (*cf.* exemple 1 ci-dessous).

Un autre exemple de fonction Z₁₃ est la fonction : $Z = \left(1,849\right) \times CXCL {10}^{t} + \left(2,368\right) \times {HA}^{t}$ avec λ_{CXCL10} = -0,116 et λ_{HA} = -0,27.

La valeur seuil de Z₇ peut par exemple être de -7 à 25, plus particulièrement de 3 à 25, plus particulièrement de 10 à 25, plus particulièrement de 13,5.

Une fonction Z₇ peut par exemple avoir un taux de bonne classification d'au moins 66% et/ou une valeur d'AUC d'au moins 0,704, plus particulièrement un taux de bonne classification d'au moins 72% et une valeur d'AUC d'au moins 0,729, plus particulièrement un taux de bonne classification d'au moins 80% et une valeur d'AUC d'au moins 0,865, plus particulièrement un taux de bonne classification d'au moins 82% et une valeur d'AUC d'au moins 0,865 (*cf.* exemple 6 ci-dessous).

Une fonction Z₇ peut par exemple avoir une sensibilité d'au moins 70% et/ou une VPN d'au moins 70%, particulièrement une sensibilité d'au moins 75% et/ou une VPN d'au moins 75%, plus particulièrement une sensibilité d'au moins 76% et/ou une VPN d'au moins 75%, plus particulièrement une sensibilité d'au moins 78% et/ou une VPN d'au moins 77%.

Une fonction Z₇ peut par exemple avoir une spécificité d'au moins 59% et/ou une VPP d'au moins 54%, plus particulièrement une spécificité d'au moins 70% et/ou une VPP d'au moins 61%, plus particulièrement une spécificité d'au moins 85% et/ou une VPP d'au moins 85%, plus particulièrement une spécificité d'au moins 86% et/ou une VPP d'au moins 86%, plus particulièrement une spécificité d'au moins 90% et/ou une VPP d'au moins 90%.

Un autre exemple de fonction Z₁₃, qui est également un exemple de fonction Z₁₂, est le suivant : $Z = a \left(CXCL{10}^{t}\right) + b \left({HA}^{t}\right) + c \left({IMC}^{t}\right) + d \left({Âge}^{t}\right) + e \left({CV}^{t}\right)$ avec $0,748 \leq a \leq 5,357$ $2,075 \leq b \leq 4,690$ $- 0,848 \leq c \leq 3,697$ $- 0,746 \leq d \leq 0,147$ $- 0,003 \leq e \leq 0,002$ $- 0,262 \leq {λ}_{CXCL 10} \leq 0,047$ $- 0,882 \leq {λ}_{HA} \leq - 0,219$ $- 5,545 \leq {λ}_{IMC} \leq 0,485$ $- 0,116 \leq {λ}_{âge} \leq 0,828$ $0,236 \leq {λ}_{CV} \leq 0,305$ a, b, c, d, e, λ_{CXCL10}, λ_{HA}, λ_{IMC}, λ_{CV} étant différent de zéro.

La valeur seuil de Z₁₁ peut par exemple être de -7 à 25, plus particulièrement de -7 à 17.

Des illustrations de fonctions Z₁₁ sont présentées en exemples 1, 3, 6, 7 et 8 ci-dessous (fonctions Z₃, Z₅ et Z₈).

Une fonction Z₁₁ peut par exemple avoir un taux de bonne classification d'au moins 66% et/ou une valeur d'AUC d'au moins 0,704, plus particulièrement un taux de bonne classification d'au moins 72% et une valeur d'AUC d'au moins 0,729, plus particulièrement un taux de bonne classification d'au moins 80% et une valeur d'AUC d'au moins 0,865, plus particulièrement un taux de bonne classification d'au moins 80% et une valeur d'AUC d'au moins 0,882, plus particulièrement un taux de bonne classification d'au moins 83% et une valeur d'AUC d'au moins 0,899 (*cf.* exemples 1, 3, 6, 7 et 8 ci-dessous).

Une fonction Z₁₁ peut par exemple avoir une sensibilité d'au moins 70% et/ou une VPN d'au moins 70%, particulièrement une sensibilité d'au moins 75% et/ou une VPN d'au moins 75%, plus particulièrement une sensibilité d'au moins 76% et/ou une VPN d'au moins 75%, plus particulièrement une sensibilité d'au moins 80% et/ou une VPN d'au moins 78% (*cf.* exemples 1, 3, 6 et 7 ci-dessous).

Une fonction Z₁₁ peut par exemple avoir une spécificité d'au moins 59% et/ou une VPP d'au moins 54%, plus particulièrement une spécificité d'au moins 70% et/ou une VPP d'au moins 61%, plus particulièrement une spécificité d'au moins 85% et/ou une VPP d'au moins 85%, plus particulièrement une spécificité d'au moins 86% et/ou une VPP d'au moins 86%, plus particulièrement une spécificité d'au moins 87% et/ou une VPP d'au moins 88% (*cf.* exemples 1, 3, 6 et 7 ci-dessous).

Un exemple de fonction Z₁₁ est la fonction : $Z = \left(0,2914\right) \times CXCL {10}^{t} + \left(0,2569\right) \times {HA}^{t} + \left(-9,3855\right) \times {IMC}^{t} + \left(0,01419\right) \times {Âge}^{t} + \left(0,0140\right) \times {CV}^{t}$ avec ${λ}_{CXCL 10} = - 0,013$ ${λ}_{HA} = 0,099$ ${λ}_{âge} = 1,086$ ${λ}_{IMC} = - 0,923$ ${λ}_{CV} = 0,159 .$

La valeur seuil de Z₃ peut par exemple être de -7 à 25, plus particulièrement de -7 à 17, plus particulièrement de -5,730.

La fonction Z₃ peut par exemple avoir un taux de bonne de classification d'au moins 66% et/ou une AUC d'au moins 0,704, plus particulièrement un taux de bonne classification d'au moins 72% et une valeur d'AUC d'au moins 0,729, plus particulièrement un taux de bonne de classification d'au moins 73% et une AUC d'au moins 0,731, plus particulièrement un taux de bonne classification d'au moins 80% et une valeur d'AUC d'au moins 0,868 (*cf.* exemples 1 et 8 ci-dessous).

La fonction Z₃ peut par exemple avoir une sensibilité d'au moins 70% et/ou une VPN d'au moins 70%, particulièrement une sensibilité d'au moins 75% et/ou une VPN d'au moins 75%, plus particulièrement une sensibilité d'au moins 76% et/ou une VPN d'au moins 75%, plus particulièrement une sensibilité d'au moins 76% et/ou une VPN d'au moins 83% (*cf.* exemple 1 ci-dessous).

La fonction Z₃ peut par exemple avoir une spécificité d'au moins 59% et/ou une VPP d'au moins 54%, plus particulièrement une spécificité d'au moins 70% et/ou une VPP d'au moins 61%, plus particulièrement une spécificité d'au moins 71% et/ou une VPP d'au moins 62% (*cf.* exemple 1 ci-dessous).

Un exemple de fonction Z₁₁ est la fonction : $Z = \left(1,999\right) \times CXCL {10}^{t} + \left(2,958\right) \times {HA}^{t} + \left(0,616\right) \times {IMC}^{t} + \left(-0,053\right) \times {Âge}^{t} + \left(-0,00024\right) \times {CV}^{t}$ avec ${λ}_{CXCL 10} = - 0,116$ ${λ}_{HA} = - 0,288$ ${λ}_{âge} = 0,433$ ${λ}_{IMC} = - 0,039$ ${λ}_{CV} = 0,279 .$

La valeur seuil de Z₅ peut par exemple être de -7 à 25, plus particulièrement de -7 à 17, plus particulièrement de 16,543.

Une fonction Z₅ peut par exemple avoir un taux de bonne classification d'au moins 66% et/ou une valeur d'AUC d'au moins 0,704, plus particulièrement un taux de bonne classification d'au moins 72% et une valeur d'AUC d'au moins 0,729, plus particulièrement un taux de bonne classification d'au moins 80% et une valeur d'AUC d'au moins 0,865, plus particulièrement un taux de bonne classification d'au moins 80% et une valeur d'AUC d'au moins 0,868, plus particulièrement un taux de bonne classification d'au moins 80% et une valeur d'AUC d'au moins 0,882, plus particulièrement un taux de bonne classification d'au moins 83% et une valeur d'AUC d'au moins 0,899 (*cf.* exemples 1, 3, 7 et 8 ci-dessous).

Une fonction Z₅ peut par exemple avoir une sensibilité d'au moins 70% et/ou une VPN d'au moins 70%, particulièrement une sensibilité d'au moins 75% et/ou une VPN d'au moins 75%, plus particulièrement une sensibilité d'au moins 76% et/ou une VPN d'au moins 75%, plus particulièrement une sensibilité d'au moins 80% et/ou une VPN d'au moins 78% (*cf.* exemples 1, 3 et 7 ci-dessous).

Une fonction Z₅ peut par exemple avoir une spécificité d'au moins 59% et/ou une VPP d'au moins 54%, plus particulièrement une spécificité d'au moins 70% et/ou une VPP d'au moins 61%, plus particulièrement une spécificité d'au moins 85% et/ou une VPP d'au moins 85%, plus particulièrement une spécificité d'au moins 86% et/ou une VPP d'au moins 86%, plus particulièrement une spécificité d'au moins 87% et/ou une VPP d'au moins 88% (*cf.* exemples 1, 3 et 7 ci-dessous).

Un exemple de fonction Z₁₁ est la fonction : $Z = \left(1,853\right) \times CXCL {10}^{t} + \left(2,511\right) \times {HA}^{t} + \left(0,4246\right) \times {IMC}^{t} + \left(-0,0343\right) \times {Âge}^{t} + \left(-0,00027\right) \times {CV}^{t}$ avec ${λ}_{CXCL 10} = - 0,116$ ${λ}_{HA} = - 0,27$ ${λ}_{âge} = 0,536$ ${λ}_{IMC} = - 0,0056$ ${λ}_{CV} = 0,288 .$

La valeur seuil de Z₈ peut par exemple être de -7 à 25, plus particulièrement de -7 à 17, plus particulièrement de 14,7.

Une fonction Z₈ peut par exemple avoir un taux de bonne classification d'au moins 66% et/ou une valeur d'AUC d'au moins 0,704, plus particulièrement un taux de bonne classification d'au moins 72% et une valeur d'AUC d'au moins 0,729, plus particulièrement un taux de bonne classification d'au moins 80% et une valeur d'AUC d'au moins 0,865, plus particulièrement un taux de bonne classification d'au moins 80% et une valeur d'AUC d'au moins 0,868, plus particulièrement un taux de bonne classification d'au moins 82% et une valeur d'AUC d'au moins 0,868 (*cf.* exemples 6 et 8 ci-dessous).

Une fonction Z₈ peut par exemple avoir une sensibilité d'au moins 70% et/ou une VPN d'au moins 70%, particulièrement une sensibilité d'au moins 75% et/ou une VPN d'au moins 75%, plus particulièrement une sensibilité d'au moins 76% et/ou une VPN d'au moins 75%, plus particulièrement une sensibilité d'au moins 80% et/ou une VPN d'au moins 78%.

Une fonction Z₈ peut par exemple avoir une spécificité d'au moins 59% et/ou une VPP d'au moins 54%, plus particulièrement une spécificité d'au moins 70% et/ou une VPP d'au moins 61%, plus particulièrement une spécificité d'au moins 85% et/ou une VPP d'au moins 85%, plus particulièrement une spécificité d'au moins 86% et/ou une VPP d'au moins 86%, plus particulièrement une spécificité d'au moins 87% et/ou une VPP d'au moins 88%.

Un autre exemple de fonction Z₁₃, qui est également un exemple de fonction Z₁₂, est le suivant : $Z = \left(0,3313\right) \times CXCL {10}^{t} + \left(0,25154\right) \times {HA}^{t} + \left(-9,8818\right) \times {IMC}^{t} + \left(0,0143\right) \times {Âge}^{t}$ avec ${λ}_{CXCL 10} = - 0,013$ ${λ}_{HA} = 0,099$ ${λ}_{âge} = 1,086$ ${λ}_{IMC} = - 0,923 .$

La valeur seuil de Z₂ peut par exemple être de -7 à 25, plus particulièrement de -7 à 17, plus particulièrement de 14,7.

Une fonction Z₂ peut par exemple avoir un taux de bonne classification d'au moins 66% et/ou une valeur d'AUC d'au moins 0,704, plus particulièrement un taux de bonne classification d'au moins 72% et une valeur d'AUC d'au moins 0,729 (*cf.* exemple 1 ci-dessous).

Une fonction Z₂ peut par exemple avoir une sensibilité d'au moins 70% et/ou une VPN d'au moins 70%, particulièrement une sensibilité d'au moins 75% et/ou une VPN d'au moins 75%, plus particulièrement une sensibilité d'au moins 76% et/ou une VPN d'au moins 75%, plus particulièrement une sensibilité d'au moins 76% et/ou une VPN d'au moins 82% (*cf.* exemple 1 ci-dessous).

Une fonction Z₂ peut par exemple avoir une spécificité d'au moins 59% et/ou une VPP d'au moins 54%, plus particulièrement une spécificité d'au moins 70% et/ou une VPP d'au moins 61% (*cf.* exemple 1 ci-dessous).

Un autre exemple de fonction Z₁₃, qui est également un exemple de fonction Z₁₂, est le suivant : $Z = \left(1,686\right) \times CXCL {10}^{t} + \left(2,216\right) \times {HA}^{t} + \left(6,947\right) \times FS$ FS étant la valeur de quantification de la dureté du foie, par exemple en kDa, avec ${λ}_{CXCL 10} = - 0,016$ ${λ}_{HA} = 0,2888$ ${λ}_{FS} = - 0,888 .$

La valeur seuil de Z₆ peut par exemple être de -7 à 25, plus particulièrement de -7 à 17, plus particulièrement de 14,7.

Une fonction Z₆ peut par exemple avoir un taux de bonne classification d'au moins 66% et/ou une valeur d'AUC d'au moins 0,704, plus particulièrement un taux de bonne classification d'au moins 72% et une valeur d'AUC d'au moins 0,729, plus particulièrement un taux de bonne classification d'au moins 80% et une valeur d'AUC d'au moins 0,865, plus particulièrement un taux de bonne classification d'au moins 80% et une valeur d'AUC d'au moins 0,868, plus particulièrement un taux de bonne classification d'au moins 86% et une valeur d'AUC d'au moins 0,931 (*cf.* exemple 5 ci-dessous).

Une fonction Z₆ peut par exemple avoir une sensibilité d'au moins 70% et/ou une VPN d'au moins 70%, particulièrement une sensibilité d'au moins 75% et/ou une VPN d'au moins 75%, plus particulièrement une sensibilité d'au moins 76% et/ou une VPN d'au moins 75%, plus particulièrement une sensibilité d'au moins 83% et/ou une VPN d'au moins 81% (*cf.* exemple 5 ci-dessous).

Une fonction Z₆ peut par exemple avoir une spécificité d'au moins 59% et/ou une VPP d'au moins 54%, plus particulièrement une spécificité d'au moins 70% et/ou une VPP d'au moins 61%, plus particulièrement une spécificité d'au moins 85% et/ou une VPP d'au moins 85%, plus particulièrement une spécificité d'au moins 86% et/ou une VPP d'au moins 86%, plus particulièrement une spécificité d'au moins 89% et/ou une VPP d'au moins 90% (*cf.* exemple 5 ci-dessous).

Un autre exemple de fonction Z₁₃, qui est également un exemple de fonction Z₁₂, est le suivant : ${Z}_{7} = \left(1,585\right) \times CXCL {10}^{t} + \left(2,181\right) \times {HA}^{t} + \left(2,910\right) \times FS$ FS étant la valeur de quantification de la dureté du foie, par exemple en kDa, avec ${λ}_{CXCL 10} = - 0,016$ ${λ}_{HA} = - 0,27$ ${λ}_{FS} = - 0,27 .$

La valeur seuil de Z₉ peut par exemple être de -7 à 25, plus particulièrement de -7 à 17, plus particulièrement de 14,7.

Une fonction Z₉ peut par exemple avoir un taux de bonne classification d'au moins 66% et/ou une valeur d'AUC d'au moins 0,704, plus particulièrement un taux de bonne classification d'au moins 72% et une valeur d'AUC d'au moins 0,729, plus particulièrement un taux de bonne classification d'au moins 80% et une valeur d'AUC d'au moins 0,865, plus particulièrement un taux de bonne classification d'au moins 85% et une valeur d'AUC d'au moins 0,868 (*cf.* exemple 6 ci-dessous).

Une fonction Z₉ peut par exemple avoir une sensibilité d'au moins 70% et/ou une VPN d'au moins 70%, particulièrement une sensibilité d'au moins 75% et/ou une VPN d'au moins 75%, plus particulièrement une sensibilité d'au moins 76% et/ou une VPN d'au moins 75% (*cf.* exemple 6 ci-dessous).

Une fonction Z₉ peut par exemple avoir une spécificité d'au moins 59% et/ou une VPP d'au moins 54%, plus particulièrement une spécificité d'au moins 70% et/ou une VPP d'au moins 61%, plus particulièrement une spécificité d'au moins 85% et/ou une VPP d'au moins 85%, plus particulièrement une spécificité d'au moins 86% et/ou une VPP d'au moins 86% (*cf.* exemple 6 ci-dessous).

Une fonction de régression logistique peut avoir la forme suivante : $LOGIT = Intercept + k \left({BMQ}_{1}\right) + l \left({BMQ}_{2}\right) ,$ dans laquelle BMQ₁ et BMQ₂ sont deux biomarqueurs différents (par exemple CXCL10 et HA), et dans laquelle Intercept, k et l sont les valeurs numériques des constantes de la fonction de régression logistique.

Par exemple : $LOGIT = Intercept + k \left(CXCL10\right) + l \left(AH\right)$ avec $- 5 \leq Intercept \leq - 1$ $0,001 \leq k \leq 0,010$ $0,010 \leq l \leq 0,050 .$

Plus particulièrement, une fonction de régression logistique peut être : $LOGIT = Intercept + k \left(CXCL10\right) + l \left(HA\right)$ avec $- 4,481 \leq Intercept \leq - 2,398$ $0,003 \leq k \leq 0,008$ $0,013 \leq l \leq 0,045 ,$ (*cf.* exemple 8, Tableau 24).

Des illustrations de fonctions de régression logistique LOGIT₁ et LOGIT₂ sont présentées en exemple 7 ci-dessous, à savoir : ${LOGIT}_{3} = Intercept + k \left(CXCL10\right) + l \left(HA\right)$ avec $- 3,57 \leq Intercept \leq - 2,67$ $0,003 \leq k \leq 0,007$ et 0,02 ≤ 1 ≤ 0,04 (*cf.* exemple 7, Tableau 19) ; $LOGIT = Intercept + k \left(CXCL10\right) + l \left(HA\right)$ avec
Intercept = -3,164
k= 0,005 et
1 = 0,024 (*cf.* exemple 7, Tableau 22).

La valeur seuil de chacune des fonctions LOGIT de la demande, plus particulièrement de LOGIT₁, LOGIT₂, LOGIT₃, LOGIT₄, peut par exemple être de 0,5.

Une fonction de régression logistique de la demande (mettant en oeuvre en fonction affine LOGIT), plus particulièrement LOGIT₁, LOGIT₂, LOGIT₃ et LOGIT₄, peut par exemple avoir un taux de bonne classification d'au moins 66% et/ou une valeur d'aire sous la courbe ROC (AUC) d'au moins 0,704, plus particulièrement un taux de bonne classification d'au moins 72% et/ou une valeur d'aire sous la courbe ROC (AUC) d'au moins 0,729, plus particulièrement un taux de bonne classification d'au moins 80% et une valeur d'AUC d'au moins 0,865, plus particulièrement un taux de bonne classification d'au moins 80% et/ou une valeur d'aire sous la courbe ROC (AUC) d'au moins 0,882.

Une fonction de régression logistique de la demande, plus particulièrement LOGIT₁, LOGIT₂, LOGIT₃ et LOGIT₄, peut par exemple avoir plus une sensibilité d'au moins 70% et/ou une VPN d'au moins 70%, particulièrement une sensibilité d'au moins 75% et/ou une VPN d'au moins 75%, plus particulièrement une sensibilité d'au moins 76% et/ou une VPN d'au moins 75%.

Une fonction de régression logistique de la demande, plus particulièrement LOGIT₁, LOGIT₂, LOGIT₃ et LOGIT₄, peut par exemple avoir une spécificité d'au moins 59% et/ou une VPP d'au moins 54%, plus particulièrement une spécificité d'au moins 70% et/ou une VPP d'au moins 61%, plus particulièrement une spécificité d'au moins 85% et/ou une VPP d'au moins 85%, plus particulièrement une spécificité d'au moins 86% et/ou une VPP d'au moins 86%.

### Apprentissage automatique (par exemple, CART)

Alternativement ou complémentairement, l'apprentissage automatique peut être mis en oeuvre pour comparer les valeurs de quantification des biomarqueurs à leurs valeurs, ou à la distribution de leurs valeurs, dans des cohortes de référence préétablies selon le stade de fibrose hépatique, pour classer ledit sujet dans celle de ces cohortes de référence vis-à-vis de laquelle il a la plus forte probabilité d'appartenance. Ledit apprentissage automatique peut être un apprentissage par arbre de décision, plus particulièrement par arbre de décision CART.

Par exemple, les valeurs de quantification obtenues pour ledit sujet sont chacune comparées à une valeur seuil qui est propre au, ou caractéristique du marqueur dont est la valeur, en suivant un arbre de décision, plus particulièrement un arbre de décision CART. Cet arbre de décision (plus particulièrement cet arbre de décision CART), plus particulièrement lesdites valeurs seuils de cet arbre, peuvent être [préalablement] déterminées par apprentissage automatique, plus particulièrement par établissement d'un arbre de décision (plus particulièrement par établissement d'un arbre CART), à partir des valeurs de quantification, ou de la distribution des valeurs de quantification, desdites cohortes de référence, plus particulièrement desdites première et deuxième cohortes de référence. Un arbre de décision, plus particulièrement un arbre de décision CART, peut donc être défini par une succession de valeurs seuils, qui s'ordonnent selon un arbre de décision.

Par exemple, un arbre de décision mettant en oeuvre les marqueurs HA et CXCL10 peut être défini par trois valeurs seuils :
- une première valeur seuil (par exemple, h) s'appliquant à la valeur de quantification de l'un des deux biomarqueurs (par exemple, la valeur de quantification de HA), et créant un embranchement à deux ramifications selon que la valeur de quantification est inférieure ou supérieure à la valeur seuil, puis
- deux autres valeurs seuils (par exemple, i et j) qui s'appliquent chacune à la valeur de quantification de l'autre des deux biomarqueurs (par exemple, la valeur de quantification de CXCL10), l'une de ces deux autres valeurs seuils s'appliquant sur l'une des deux ramifications et l'autre des deux autres valeurs seuils s'appliquant sur l'autre des deux ramifications.

Un tel arbre CART est représenté en Figure 12.

Conformément à la Figure 12, ledit apprentissage automatique peut être un arbre de décision CART, qui comprend un seuil de décision h pour le marqueur HA et des seuils de décision i et j pour le marqueur CXCL10, lesdits seuils h, i et j étant comme suit :
si la valeur de quantification de HA pour ledit sujet est inférieure à h, le seuil du marqueur CXCL10 est j, et:
   ledit sujet est classé dans ladite première cohorte (score de fibrose Metavir < F2) si la valeur de quantification de CXCL10 est inférieure à j,
   ledit sujet est classé dans ladite deuxième cohorte (score de fibrose Metavir ≥ F2) si la valeur de quantification de CXCL10 est supérieure ou égale à j,
   et
si la valeur de quantification de HA pour ledit sujet est supérieure ou égale à h, le seuil du marqueur CXCL10 est i, et
   ledit sujet est classé dans ladite première cohorte (score de fibrose Metavir < F2) si la valeur de quantification de CXCL10 est inférieure à i,
   ledit sujet est classé dans ladite deuxième cohorte (score de fibrose Metavir ≥ F2) si la valeur de quantification de CXCL10 est supérieure ou égale à i.

Par exemple, l'arbre CART est celui de la Figure 12, avec : $40 \leq h \leq 80$ $150 \leq i \leq 300$ $400 \leq j \leq 620$

### [arbre CART₁].

Par exemple, l'arbre CART est celui de la Figure 12, avec : $41,96 \leq h \leq 77,43$ $159,09 \leq i \leq 266,7$ $410,75 \leq j \leq 613,49$ **[arbre CART₂]** ; *cf.* exemple 8.

Par exemple, l'arbre CART est celui de la Figure 12, avec : $42,18 \leq h \leq 77,4$ $209,3 \leq i \leq 266,7$ $454,7 \leq j \leq 553,1$ **[arbre CART₃]** ; *cf.* exemples 7 et 8.

Par exemple, l'arbre CART est celui de la Figure 12, avec :
h = 47,29
i = 209,3
j = 503,4
**[arbre CART₄]** ; *cf.* exemples 7 et 8.

Un arbre CART de la demande, plus particulièrement CART₁, CART₂, CART₃ et CART₄, peut par exemple avoir un taux de bonne classification d'au moins 66%, plus particulièrement d'au moins 72%, plus particulièrement d'au moins 80%, plus particulièrement d'au moins 83%, plus particulièrement d'au moins 84%, plus particulièrement d'au moins 85%.

Un arbre CART de la demande, plus particulièrement CART₁, CART₂, CART₃ et CART₄, peut par exemple avoir plus une sensibilité d'au moins 70% et/ou une VPN d'au moins 70%, particulièrement une sensibilité d'au moins 75% et/ou une VPN d'au moins 75%, plus particulièrement une sensibilité d'au moins 76% et/ou une VPN d'au moins 75%, plus particulièrement une sensibilité d'au moins 77% et/ou une VPN d'au moins 77%, plus particulièrement une sensibilité d'au moins 78% et/ou une VPN d'au moins 78%, plus particulièrement une sensibilité d'au moins 82% et/ou une VPN d'au moins 80%.

Un arbre CART de la demande, plus particulièrement CART₁, CART₂, CART₃ et CART₄, peut par exemple avoir une spécificité d'au moins 59% et/ou une VPP d'au moins 54%, plus particulièrement une spécificité d'au moins 70% et/ou une VPP d'au moins 61%, plus particulièrement une spécificité d'au moins 85% et/ou une VPP d'au moins 85%, plus particulièrement une spécificité d'au moins 86% et/ou une VPP d'au moins 86%, plus particulièrement une spécificité d'au moins 89% et/ou une VPP d'au moins 90%, plus particulièrement une spécificité d'au moins 91% et/ou une VPP d'au moins 91%.

### Combinaison d'apprentissage automatique et de fonction mathématique

Alternativement ou complémentairement, ladite comparaison peut être faite en combinant apprentissage automatique et fonction mathématique, par exemple en combinant arbre de décision et fonction linéaire ou non linéaire.

Par exemple, cette comparaison peut être faite :
- par arbre de décision, plus particulièrement par arbre CART, pour un ou au moins un marqueur choisi parmi la Charge Virale, l'âge, l'IMC et la dureté du foie, plus particulièrement pour (au moins) le marqueur dureté du foie, puis
- par fonction linéaire ou affine, plus particulièrement par mROC ou régression logistique, pour des marqueurs choisis comme ci-dessus décrits, plus particulièrement pour au moins les marqueurs CXCL10 et HA.

Des illustrations sont présentées en exemple 6 ci-dessous et en Figures 9, 10, 11 : comparaison à une valeur seuil pour une des valeurs (en l'occurrence, la valeur de la dureté du foie), suivie par une comparaison à l'aide d'une fonction linéaire pour les marqueurs CXCL10 et HA (Figure 9), ou pour les marqueurs CXCL10, HA, âge, IMC et CV (Figure 10), ou pour les marqueurs CXCL10, HA et FS (Figure 11).

Ainsi, selon un mode de réalisation, la méthode commence par une étape de mesure de la dureté du foie dudit sujet, par exemple par FIBROSCAN™. Cette étape permet par exemple de déterminer si le foie dudit sujet est ou non atteint d'une cirrhose (stade Metavir F4). S'il n'est pas atteint d'une cirrhose, c'est-à-dire si le stade de fibrose hépatique dudit sujet est inférieur au score Metavir F4, un échantillon de fluide biologique peut être analysé pour quantifier les marqueurs biologiques choisis comme ci-dessus décrit, plus particulièrement pour au moins CXCL10 et HA, de sorte à déterminer plus avant son stade de fibrose hépatique (score Metavir de F0 ou F1, ou bien score Metavir de F2 ou F3). Si le foie dudit sujet est atteint d'une cirrhose (détectée par mesure de la dureté du foie), la mise en oeuvre d'une analyse d'un échantillon de fluide biologique apparaît redondante, et n'est donc pas nécessaire.

### Autres objets de la demande

Selon un aspect complémentaire, la demande est relative à des produits, réactifs ou ligands pour la détection et/ou la quantification des biomarqueurs sélectionnés, plus particulièrement à des réactifs ou ligands qui se lient de manière spécifique à des molécules circulantes sélectionnées à titre de biomarqueurs comme ci-dessus décrit, ainsi qu'à des articles manufacturés, des compositions, des compositions pharmaceutiques, des kits, des tubes, des supports solides comprenant de tels réactifs ou ligands, ainsi qu'à des systèmes informatiques (notamment, produit programme d'ordinateur et dispositif informatique), qui sont spécialement adaptés à la mise en oeuvre des méthodes ou produits de la demande.

La demande est notamment relative à un ligand qui se lie de manière spécifique à un biomarqueur qui est une molécule circulante telle que décrite ci-dessus, par exemple un ligand qui se lie de manière spécifique à HA ou un ligand qui se lie de manière spécifique à CXCL10 (plus particulièrement à la protéine CXCL10 humaine). Plus particulièrement, ce ligand se lie de manière spécifique à la forme circulante de cette molécule, ou s'il en existe plusieurs, à au moins une, à plusieurs, ou à toutes les formes circulantes de cette molécule.

De préférence, ce ligand permet non seulement de détecter spécifiquement le biomarqueur sélectionné, mais également de le quantifier.

Ce ligand peut notamment être une protéine, un polypeptide, un peptide, par exemple un anticorps (monoclonal ou polyclonal), un fragment d'anticorps, une protéine recombinante, un aptamère, un polysaccharide, un lipide, ou une combinaison de ces produits, plus particulièrement une protéine, un anticorps (monoclonal ou polyclonal), un fragment d'anticorps ou une protéine recombinante.

Des anticorps peuvent par exemple être produits par immunisation d'un mammifère non-humain (tel qu'un lapin) par une protéine codée par ledit gène choisi, ou par un fragment antigénique d'une telle protéine, éventuellement associée ou couplée à un adjuvant d'immunisation (tel qu'un adjuvant de Freund ou tel que KLH *-keyhole limpet hemocyanin*-)*,* par exemple par injection intrapéritonéale ou sous-cutanée, et par collecte des anticorps ainsi obtenus dans le sérum dudit mammifère.

Des anticorps monoclonaux peuvent être produits selon une technique d'hybridation lymphocytaire (hybridomes) telle que la technique de Köhler and Milstein 1975 (*cf.* également US 4 376 110), la technique d'hybridomes à cellules B humaines (Kosbor *et* al. 1983; Cole *et al.* 1983), ou la technique d'immortalisation des lymphocytes à l'aide du virus d'Epstein-Barr -EBV- (Cole *et al.* 1985). De tels anticorps peuvent par exemple être des IgG, IgM, IgE, IgA, IgD ou toute sous-classe de ces immunoglobulines.
Des anticorps modifiés par génie génétique peuvent être produits, tels que des anticorps recombinant, chimères, humanisés par greffage d'un ou plusieurs CDR *-Complementary Determining Region-.*

Les anticorps mis en oeuvre dans l'invention peuvent être des fragments d'anticorps ou des dérivés artificiels de tels fragments, dans la mesure où ces fragments ou dérivés présentent ladite propriété de liaison spécifique. De tels fragments peuvent par exemple être des fragments Fab, F(ab')2, Fv, Fab/c, scFv *(single chain Fragment variable*).

Des exemples de ligands comprennent les anticorps qui se lient de manière spécifique à CXCL10, tels que :
- l'anticorps monoclonal de souris anti-CXCL10 humaine commercialisé par R&D SYSTEMS, Inc. (614 McKinley Place NE ; Minneapolis, MN 55413 ; U.S.A.), sous la référence catalogue MAB266 (clone 33036, classe IgG1),
- l'anticorps polyclonal de chèvre anti-CXCL10 humaine disponible auprès de R&D SYSTEMS, Inc. (614 McKinley Place NE ; Minneapolis, MN 55413 ; U.S.A. ; Référence catalogue AF-266-NA pour une forme non couplée à la biotine),
- l'anticorps monoclonal de souris anti-CXCL10 humaine disponible auprès de R&D SYSTEMS, Inc. (614 McKinley Place NE ; Minneapolis, MN 55413 ; U.S.A. ; Référence catalogue MAB266).

Dans le cadre d'une mise en oeuvre en configuration « *sandwich* », chacun de ces anticorps peut être mis en oeuvre comme ligand de capture et/ou comme ligand de détection.

Des exemples de ligands comprennent les protéines qui se lient de manière spécifique à HA, telles que la protéine recombinante humaine aggrécane G1-IGD-G2 commercialisée par la société R&D SYSTEMS, Inc. (614 McKinley Place NE ; Minneapolis, MN 55413 ; U.S.A.), sous la référence catalogue 1220-PG-025, ou la protéine HABP (*HA binding protein* ; protéine issue du cartilage bovin) commercialisée par UNITED STATES BIOLOGICAL (4 Technology Way, Salem, MA 01970, U.S.A.) sous la référence catalogue H7980-30.

Dans le cadre d'une mise en oeuvre en configuration « *sandwich* », cette protéine recombinante peut être mise en oeuvre comme ligand de capture et/ou comme ligand de détection.

Chacun desdits ligands peut en outre comprendre au moins un marqueur pour leur détection, plus particulièrement au moins un marqueur de détection qui n'est pas naturellement présent dans la structure du ligand, par exemple au moins une entité choisie parmi les fluorophores (par exemple ATTO™ 550, ATTO™ 663 ; ATTO-TEC GmbH, Siegen, Germany), les chromophores, les enzymes (par exemple, la péroxydase de raifort, une phosphatase alcaline), les éléments radioactifs, les isotopes d'éléments chimiques.

La demande est également relative à un ensemble ou association d'au moins deux ligands, à savoir un premier ligand qui se lie de manière spécifique à une des molécules circulantes sélectionnées et un deuxième ligand qui se lie de manière spécifique à une autre des molécules circulantes sélectionnées, par exemple l'ensemble ou l'association d'un ligand spécifique de HA et d'un ligand spécifique de CXCL10.

Chacun desdits premier et deuxième ligands peut ou non porter un marqueur pour sa détection. Lesdits premier et deuxième ligands peuvent chacun porter un marqueur pour leur détection. Ils peuvent chacun porter le même marqueur, ou bien porter des marqueurs différents.

Lesdits ligands peuvent être en mélange, ou bien sous formes distinctes ou physiquement séparées l'une de l'autre, par exemple en préparation combinée pour une utilisation simultanée, séparée ou différée dans le temps.

Selon un mode réalisation, l'ensemble ou association ne contient pas de ligand qui se lierait à une protéine circulante qui ne ferait pas partie des molécules circulantes sélectionnées à titre de biomarqueurs comme ci-dessus décrit. Plus particulièrement, selon ce mode réalisation, l'ensemble ou association ne contient pas de ligand qui se lierait à une protéine circulante autre que HA ou CXCL10. Plus particulièrement, selon ce mode réalisation, l'ensemble ou association ne contient pas de ligand qui se lierait à A2M, GMCSF, IL12, IL2, MMP13, ALT, GGT, ICAM1, IL4, CXCL9, VCAM1, RBP4, TIMP1, VIM, SPP1, AST, ApoA1, IL6ST, p14ARF, MMP9, ANGPT2, CXCL11, MMP2, MMP7, S100A4, TIMP1, CHI3L1, COL1A1, CXCL1, CXCL6, IHH, IRF9 ou MMP1.

Cet ensemble ou association peut en outre comprendre des moyens pour la détection et/ou la mesure de la charge virale en virus de l'hépatite (par exemple, VHC et/ou VHB et/ou VHD).

La demande est également relative à un article manufacturé, une composition, une composition pharmaceutique, un kit qui chacun comprennent au moins un ligand ou au moins un ensemble ou association de ligands de la demande.

Plus particulièrement, la demande est relative à un article manufacturé, une composition, une composition pharmaceutique, un kit qui sont chacun adaptés à la détection en multiplex de molécules circulantes, c'est-à-dire de molécules contenues sous forme acellulaire dans un (échantillon de) fluide biologique, telles que CXCL10 et HA (*cf.* « Molécules circulantes » ci-dessus).

Plus particulièrement, la demande est relative à un article manufacturé, une composition, une composition pharmaceutique, un kit qui chacun comprennent :
- un premier ligand (par exemple, une première protéine), qui se lie de manière spécifique à une des molécules circulantes sélectionnées, par exemple un premier ligand (plus particulièrement, une première protéine, plus particulièrement un anticorps), qui se lie de manière spécifique à CXCL10, et
- un deuxième ligand (par exemple, une deuxième protéine), qui se lie de manière spécifique à une autre des molécules circulantes sélectionnées, par exemple un deuxième ligand (plus particulièrement, une deuxième protéine), qui se lie de manière spécifique à HA.

La demande est plus particulièrement relative à un article manufacturé, une composition, une composition pharmaceutique, un kit qui chacun comprennent ce premier ligand et ce deuxième ligand, comme produit de combinaison (ou sous forme combinée, ou en préparation combinée), notamment pour leur utilisation simultanée, séparée ou étalée dans le temps, plus particulièrement pour leur utilisation simultanée dans le temps.

Selon un mode réalisation, l'article manufacturé, la composition, la composition pharmaceutique ou le kit ne contient pas de ligand qui se lierait à une protéine circulante qui ne ferait pas partie des molécules circulantes sélectionnées à titre de biomarqueurs comme ci-dessus décrit. Plus particulièrement, selon ce mode réalisation, l'article manufacturé, la composition, la composition pharmaceutique ou le kit ne contient pas de ligand qui se lierait à une protéine circulante autre que HA ou CXCL10. Plus particulièrement, l'article manufacturé, la composition, la composition pharmaceutique ou le kit ne contient pas de ligand qui se lierait à A2M, GMCSF, IL12, IL2, MMP13, ALT, GGT, ICAM1, IL4, CXCL9, VCAM1, RBP4, TIMP1, VIM, SPP1, AST, ApoA1, IL6ST, p14ARF, MMP9, ANGPT2, CXCL11, MMP2, MMP7, S100A4, TIMP1, CHI3L1, COL1A1, CXCL1, CXCL6, IHH, IRF9 ou MMP1.

La demande est ainsi relative à un article manufacturé qui est adapté à la détection en multiplex de molécules contenues sous forme acellulaire dans un (échantillon de) fluide biologique, et qui comprend un support solide sur lequel sont fixés des ligands desdites molécules.

Ledit article manufacturé peut par exemple être :
- un ou des tubes,
- un kit, notamment un kit comprenant un ou des tubes,
- un support solide ou semi-solide, par exemple, en plastique, polystyrène, polypropylène, verre, silicium, nitrocellulose, poly(vinylidene fluoride) [PVDF] ou polymère, ou comprenant un matériau magnétique tel que de l'oxyde de fer, tel que :
   ∘ une plaque ou microplaque à puits, par exemple en polypropylène et/ou en polystyrène, plus particulièrement une plaque ou microplaque à puits adaptée à la titration et/ou au criblage à haut débit (*High Throughput Screening microplates*)*,*
   ∘ une puce ou un microréseau (à circuit intégré) en matériau conducteur ou semi-conducteur, plus particulièrement une puce (ou un microréseau) à galette (*wafer*) de silicium, plus particulièrement une puce (ou un microréseau) en silicium,
   ∘ un capillaire, plus particulièrement un capillaire en verre,
   ∘ une bille magnétique dont le diamètre moyen est inférieur au micromètre,
   ∘ une lame de verre,
   ∘ une membrane (par exemple, une membrane en nitrocellulose ou en PVDF).

Plus particulièrement, des microplaques pour criblage à haut débit peuvent être utilisées (*cf.* exemple 10). Les puits de ces microplaques ont la capacité d'adsorber les biomolécules, plus particulièrement les protéines. Elles possèdent généralement une excellente stabilité thermique et chimique ainsi que d'excellentes propriétés optiques. Elles sont généralement en polystyrène et/ou en polypropylène. Chaque microplaque contient une pluralité de puits, généralement 96 puits. Ces microplaques sont conçues pour recevoir des rangées de gouttes desdits premier et deuxième ligands, dont le volume est inférieur à 100 nL par goutte (généralement 50 nL par goutte). Un robot spotteur peut être utilisé pour déposer ces rangées de gouttes.

Lesdits premier et deuxième ligands peuvent être fixés, immobilisés ou greffés sur l'article manufacturé, ou y être liés par covalence.

L'article manufacturé peut par exemple sous la forme d'un article manufacturé qui est adapté à la détection en multiplex de molécules contenues sous forme acellulaire dans un (échantillon de) fluide biologique de volume inférieur ou égal à 500 µL (plus particulièrement inférieur ou égal à 400µL, plus particulièrement inférieur ou égal à 100 µL), dans lequel ledit support solide comprend une pluralité de zones de réception d'échantillons liquides, lesdites zones étant fluidiquement indépendantes les unes des autres et étant chacune adaptée à la réception d'un seul échantillon liquide par zone, le volume maximal d'échantillon liquide que chaque zone peut recevoir n'excédant pas 500 µL (plus particulièrement inférieur ou égal à 400µL, plus particulièrement inférieur ou égal à 100 µL), et dans lequel au moins l'une desdites de zones de réception d'échantillons liquides comprend à la fois ledit premier ligand et ledit deuxième ligand (plus particulièrement, ladite première protéine et ladite deuxième protéine). Lesdites zones de réception d'échantillons liquides peuvent par exemple être les puits d'une plaque ou microplaque de titration et/ou de criblage haut débit, les puits de réception de spots d'une puce en silicium, des portions cylindriques d'un capillaire en verre, une membrane, une lame de verre, un tube.

Dans l'article manufacturé, ledit premier ligand et ledit deuxième ligand (plus particulièrement, ladite première protéine et ladite deuxième protéine) peuvent être fixés (ou immobilisés, ou greffés, ou liés par covalence) audit support solide dans une configuration selon laquelle un seul et même échantillon liquide de volume inférieur ou égal à 500 µL (plus particulièrement inférieur ou égal à 400µL, plus particulièrement inférieur ou égal à 100 µL) peut contacter tant ladite première protéine que ladite deuxième protéine.

Dans l'article manufacturé, ledit premier ligand (plus particulièrement, ladite première protéine) peut être fixé (ou immobilisé, ou greffé, ou lié par covalence) audit support solide en un premier site de fixation, et ledit deuxième ligand (plus particulièrement, ladite deuxième protéine) peut être fixé (ou immobilisé, ou greffé, ou lié par covalence) audit support solide en un deuxième site de fixation, dans lequel ledit premier site de fixation est en contact fluidique avec ledit deuxième site de fixation.

Chacun de ces premiers et deuxième ligands peut être utilisé comme ligand de capture (par exemple, dans le cadre d'une détection en configuration sandwich mettant par ailleurs en oeuvre des ligands de détection qui portent au moins un marqueur de détection ; *cf.* composition ci-dessous, *cf.* exemple 10).

Selon un mode de réalisation, les ligands de molécules circulantes qui sont contenus dans un article manufacturé ne comprend pas de ligand qui se lierait à A2M, GMCSF, IL12, IL2, MMP13, ALT, GGT, ICAM1, IL4, CXCL9, VCAM1, RBP4, TIMP1, VIM, SPP1, AST, ApoA1, IL6ST, p14ARF, MMP9, ANGPT2, CXCL11, MMP2, MMP7, S100A4, TIMP1, CHI3L1, COL1A1, CXCL1, CXCL6, IHH, IRF9 ou MMP1.

Selon un mode de réalisation, les ligands de molécules circulantes qui sont contenus dans un article manufacturé sont constitués par :
- un premier ligand (par exemple, une première protéine), qui se lie de manière spécifique à HA, et
- un deuxième ligand (par exemple, une deuxième protéine, plus particulièrement un anticorps), qui se lie de manière spécifique à CXCL10.

Optionnellement, un article manufacturé comprend en outre des instructions (par exemple, une feuille d'instructions) pour quantifier les molécules auxquelles les ligands se lient spécifiquement, par exemple pour quantifier CXCL10 et HA, plus particulièrement des instructions (par exemple, une feuille d'instructions) pour quantifier ces molécules et déterminer un stade ou score de fibrose hépatique à partir des valeurs de quantification obtenues.

Ledit article manufacturé peut en outre comprendre un ou plusieurs des éléments suivants :
- un instrument pour le prélèvement dudit échantillon, notamment :
   ∘ une aiguille et/ou une seringue, plus particulièrement une aiguille et/ou une seringue pour le prélèvement d'un liquide intracorporel, tel que du sang, et/ou
   ∘ une aiguille adaptée à la cyto-ponction hépatique, par exemple une aiguille de diamètre 18 à 22G), et/ou
   ∘ une aiguille et/ou un cathéter et/ou un pistolet à biopsie adaptés à la PBH ;
- un produit programme d'ordinateur ou logiciel, notamment un produit programme d'ordinateur ou logiciel d'analyse statistique, par exemple un produit programme d'ordinateur utilisé dans l'invention tel que ci-dessous décrit.

La demande est également relative à une composition adaptée à la détection de molécules en multiplex, plus particulièrement à la détection en multiplex de molécules contenues sous forme acellulaire dans un fluide (telles que les molécules CXCL10 et HA), qui comprend en mélange :
- un premier ligand qui porte un premier marqueur de détection, et
- un deuxième ligand qui porte un deuxième marqueur de détection.

Ledit premier marqueur de détection peut être identique ou différent dudit deuxième marqueur de détection.

Le premier ligand et le deuxième ligand sont chacun spécifiques d'une molécule circulante différente (molécule circulante sélectionnée à titre de biomarqueur comme décrit ci-dessus).

Plus particulièrement, ledit premier ligand est un ligand (par exemple, une protéine) qui se lie de manière spécifique à HA et ledit deuxième ligand est un ligand (par exemple, une protéine, plus particulièrement un anticorps) qui se lie de manière spécifique à CXCL10 (humaine), plus particulièrement un ligand (par exemple, une protéine, plus particulièrement un anticorps) qui se lie de manière spécifique à une, au moins une, des ou toutes les formes circulantes de la protéine CXCL10 (humaine).

Ces premiers et deuxième ligands peuvent par exemple être utilisés comme ligands de détection (par exemple, dans le cadre d'une détection en configuration sandwich avec des ligands de capture ; *cf.* article manufacturé ci-dessus ; *cf.* exemple 10).

La demande est également relative à un kit, qui comprend :
- des acides nucléiques qui se lient de manière spécifique à un ou des virus de l'hépatite, et qui comprend en outre
- des ligands qui se lient à des molécules contenues sous forme acellulaire dans un fluide biologique, lesdits ligands étant contenus dans le kit en préparation combinée pour une utilisation séparée, différée ou simultanée dans le temps, plus particulièrement pour une utilisation simultanée dans le temps, plus particulièrement pour une utilisation en mélange.

Lesdits ligands qui se lient à des molécules contenues sous forme acellulaire dans un fluide biologique sont constitués par :
- un premier ligand qui porte un premier marqueur de détection, et
- un deuxième ligand qui porte un deuxième marqueur de détection.

Ledit premier marqueur de détection peut être identique ou différent dudit deuxième marqueur de détection.

Le premier ligand et le deuxième ligand sont chacun spécifiques d'une molécule circulante différente (molécule circulante sélectionnée à titre de biomarqueur comme décrit ci-dessus).

Plus particulièrement, ledit premier ligand est un ligand (par exemple, une protéine) qui se lie de manière spécifique à HA et ledit deuxième ligand est un ligand (par exemple, une protéine, plus particulièrement un anticorps) qui se lie de manière spécifique à CXCL10 (humaine), plus particulièrement un ligand (par exemple, une protéine, plus particulièrement un anticorps) qui se lie de manière spécifique à une, au moins une, des ou toutes les formes circulantes de la protéine CXCL10 (humaine).

Ces premiers et deuxième ligands peuvent par exemple être utilisés comme ligands de détection (par exemple, dans le cadre d'une détection en configuration sandwich avec des ligands de capture ; *cf.* article manufacturé ci-dessus ; *cf.* exemple 10).

Le kit peut en outre comprendre un article manufacturé tel que décrit ci-dessus.

La demande est également relative audit ligand, ensemble ou association de ligands, article manufacturé, composition, composition pharmaceutique, kit pour leur utilisation dans une méthode pour détecter ou diagnostiquer une hépatopathie qui comporte une atteinte tissulaire du foie, plus particulièrement une fibrose hépatique, plus particulièrement pour déterminer le score de fibrose hépatique d'un sujet, plus particulièrement une fibrose hépatique, plus particulièrement pour déterminer si le score de fibrose hépatique d'un sujet a ou non dépassé celui de la fibrose légère, plus particulièrement pour déterminer si la fibrose hépatique d'un sujet a un score de fibrose Metavir d'au plus F1 ou bien d'au moins F2.

La demande est également relative audit ligand, ensemble ou association de ligands, article manufacturé, composition, composition pharmaceutique, kit pour leur utilisation dans une méthode pour le traitement d'une hépatopathie qui comporte une atteinte tissulaire du foie, plus particulièrement une fibrose hépatique.

Cette utilisation peut notamment comprendre :
- la mise en oeuvre dudit(desdits) ligand(s) dans une méthode de l'invention pour déterminer si le score de fibrose hépatique d'un sujet a ou non dépassé celui de la fibrose légère, plus particulièrement pour déterminer si la fibrose hépatique d'un sujet a un score de fibrose Metavir d'au plus F1 ou bien d'au moins F2, et
- le fait d'administrer audit sujet un traitement visant à bloquer la progression de la fibrose hépatique (tel qu'un traitement comprenant interféron standard ou pégylé, en monothérapie, ou en plurithérapie associant ribavirine), si le sujet présente un score de fibrose hépatique qui a dépassé celui de la fibrose légère (score de fibrose Metavir d'au moins F2).

Cette méthode peut en outre comprendre le fait de ne pas administrer ce traitement si, ou tant que, ce score ne dépasse pas celui de la fibrose légère.

Ce traitement peut par exemple être :
- interféron alpha-2b pégylé (tel que PEG-INTRON®, Schering Plough Corporation, Kenilworth, NJ) à une dose d'environ 1,5 g/kg/semaine, et ribavirine (REBETOL® ; Schering Plough Corporation, Kenilworth, NJ) à une dose de 800 à 1200 mg/kg/jour (si l'hépatopathie implique un VHC de génotype 2 ou 3, une dose d'environ 800 mg/kg et par jour est généralement conseillée), ou
- interféron alpha-2a pégylé (tel que PEGASYS® ; Roche Corp., F. Hoffmann-La Roche Ltd. ; Basel, Suisse) à une concentration de 180 g/kg/semaine et ribavirine (COPEGUS®, Roche Corp. ; F. Hoffmann-La Roche Ltd. ; Basel, Suisse) à raison de 1000 à 1200 mg/kg/jour.

La durée du traitement peut par exemple être d'au moins 24 semaines, par exemple de 24 semaines pour une hépatopathie à VHC de génotype 2 ou 3, ou de 48 semaines pour une hépatopathie à VHC de génotype 1, 4 ou 5, ou pour un patient non-répondeur au traitement au bout de 24 semaines.

La demande est également relative à un médicament ou association médicamenteuse destiné au traitement d'une hépatopathie comportant une atteinte tissulaire du foie, plus particulièrement une fibrose hépatique (tel que interféron standard ou interféron pégylé, en monothérapie, ou en plurithérapie associant un ou plusieurs autres principes actifs, notamment de la ribavirine), pour son utilisation dans la méthode de traitement de la divulgation.

Dans la demande, le terme hépatopathie est entendu dans son sens ordinaire, à savoir une atteinte du foie, plus particulièrement une atteinte tissulaire du foie, plus particulièrement des lésions du foie, notamment une fibrose hépatique.

La demande s'adresse plus particulièrement aux hépatopathies chroniques (agressions chroniques du foie de 6 mois ou plus).

Différentes maladies causent et /ou conduisent à des lésions du foie, telles qu'une fibrose hépatique. Peuvent notamment être citées :
- les hépatites chroniques virales (notamment l'hépatite chronique B, l'hépatite chronique C, l'hépatite chronique D ;
- les stéatoses et stéato-hépatites (associées au syndrome métabolique, à l'obésité, au diabète).
- les hépatites alcooliques ;
- l'hématochromatose génétique et les surcharges en fer secondaire
- Les maladies auto-immunes ;
- les maladies biliaires (cirrhose biliaire primitive et cholangie sclérosante primitive) ;
- les intoxications par médicament ou substance toxique ;
- des maladies métaboliques ;
- des stéatohépatites non alcooliques (NASH).

La demande est plus particulièrement adaptée aux hépatites virales, notamment aux hépatites à virus C (VHC) et/ou à virus B (VHB) et/ou à virus D (VHD), notamment aux hépatites virales à au moins VHC (et optionnellement à VHB et/ou VHD).

La demande est également relative à un produit programme d'ordinateur, destiné à être stocké dans une mémoire d'une unité de traitement, ou sur un support mémoire amovible destiné à coopérer avec un lecteur de ladite unité de traitement. Le produit programme d'ordinateur comprend des instructions pour la mise en oeuvre d'une méthode ou d'un produit de la demande, notamment pour la mise en oeuvre d'une analyse statistique adaptée à la mise en oeuvre d'une méthode de l'invention [notamment adaptée à l'analyse statistique (multivariée) des biomarqueurs choisis] et/ou pour la construction d'un modèle de classification (multivariée) adapté à la mise en oeuvre d'une méthode ou de l'utilisation d'un produit de l'invention.

La demande est également relative à installation informatique, un dispositif informatique, ou ordinateur, comprenant une unité de traitement dans la mémoire de laquelle sont stockés ou enregistrés :
- un produit programme d'ordinateur de la demande, et, optionnellement,
- des valeurs de quantification des biomarqueurs sélectionnés.

Le terme "comprenant", avec lequel "incluant" ou "contenant" est synonyme, est un terme ouvert, et n'exclut pas la présence d'un ou plusieurs élément(s), ingrédient(s) ou étape(s) de méthode additionnel(s) qui ne serait(seraient) pas explicitement indiqué(s), tandis que le terme "consistant" ou "constitué" est un terme fermé, qui exclut la présence de tout autre élément additionnel, étape, ou ingrédient qui ne serait pas explicitement exposé. Le terme "consistant essentiellement" ou " essentiellement constitué" est un terme partiellement ouvert, qui n'exclut pas la présence d'un ou plusieurs élément(s), ingrédient(s) ou étape(s) additionnel(s), dans la mesure où cet(ces) élément(s), ingrédient(s) ou étape(s) additionnel(s) n'affecte(nt) pas matériellement les propriétés de base de l'invention.

Par conséquent, le terme "comprenant" (ou "comprend(comprennent)") inclut les termes "consistant", "constitué", aussi bien que les termes "consistant essentiellement" et " essentiellement constitué".

Dans le but de faciliter la lecture de la demande, la description a été séparée en divers paragraphes, sections et modes de réalisation. Il ne doit pas être considéré que ces séparations déconnectent la substance d'un paragraphe, section ou mode de réalisation, de celle d'un autre paragraphe, section ou mode réalisation. Au contraire, la description englobe toutes les combinaisons possibles des différents paragraphes, sections, phrases et modes de réalisations qu'elle contient.

Le contenu des références bibliographiques citées dans la demande est spécifiquement incorporé par référence dans le contenu de la demande.
Les exemples qui suivent sont donnés à titre purement illustratif. Ils ne limitent en aucune façon l'invention.

### EXEMPLES

### EXEMPLE 1 : établissement et application de combinaisons HA+CXCL10 à une population de patients dont le degré de fibrose est établi par Ponction Biopsie Hépatique (PBH)

La population de patients était formée de patients de l'Hôpital Beaujon (100, boulevard du Général Leclerc ; 92110 Clichy ; France), qui présentaient une hépatite chronique suite à une infection par le virus de l'Hépatite C (VHC), et couvrant tous, ou la plupart des génotypes de VHC.
Le score de fibrose hépatique de ces patients a été établi par Ponction Biopsie Hépatique (PBH). Cette population était constituée de 118 patients, parmi lesquels 73 présentaient un score de fibrose F1, et 45 présentaient un score de fibrose F2 (score établi par PBH selon le système de scores F Metavir, deux lectures indépendantes par un anatomo-pathologiste confirmé). Les études réalisées ont été approuvées par le Comité Éthique local, conformément à la Déclaration d'Helsinki. Tous les patients ont donné leur consentement éclairé par écrit.
Les caractéristiques de ces 118 patients sont présentées dans le Tableau 1 ci-dessous.

**Tableau 1 :**

| Variables | | Population totale | Patients F1 | Patients F2 |
|---|---|---|---|---|
| n | | 118 | 73 | 45 |
| Sexe [homme (%) / femme(%)] | | 54 (46) / 64 (54) | 27 (37) / 46 (63) | 27 (60) / 18 (40) |
| Âge à la date du prélèvement [moyenne ± SD (min-max)] | | 49,1 ± 11,2 (21 -71) | 46,7 ± 11,0 (21 - 71) | 53,1 ± 10,4 (32 - 71) |
| Alanine aminotransferase (ALT) UI/L [moyenne ± SD (min-max)] | | 95 ± 83 (22 - 647) | 77 ± 54 (22 - 308) | 122 ± 111 (36 - 647) |
| Génotype VHC [n(%)] | | | | |
| | 1 | 61 (52) | 34 (47) | 27 (60) |
| | 2 | 15 (13) | 11 (15) | 4 (9) |
| | 3 | 12 (10) | 7 (10) | 5 (11) |
| | 4 | 23(19) | 16 (22) | 7(16) |
| | 5 | 4 (3) | 3 (4) | 1 (2) |
| | 6 | 2 (2) | 1 (1) | 1 (2) |
| | inconnu | 1 (1) | 1 (1) | 0(0) |
| Charge virale à la date du prélèvement (CV): copies/mL [moyenne (min-max)] | | 5,6.10⁶ (1,4.10⁴ - 5,9.10⁷) | 4,8.10⁶ (3,7.10⁴-2,7.10⁷) | 7,0.10⁶ (1,4.10 ⁴-5,9.10⁷) |

| | | | | |
|---|---|---|---|---|
| SD = déviation standard ; UI = unité internationale | | | | |

Les concentrations sériques de la protéine CXCL10 et de l'acide hyaluronique (HA) ont été mesurées dans le sérum de ces patients à l'aide de kits ELISA disponibles dans le commerce (sandwich en phase solide).
Pour la concentration sérique de CXCL10 humaine, le kit qui a été utilisé était le kit QUANTIKINE® HUMAN CXCL10/IP-10 ELISA, commercialisé par R&D Systems, Inc. (614 McKinley Place NE, Minneapolis, MN 55413, U.S.A.), sous la référence catalogue DIP100.
Pour la concentration sérique de l'acide hyaluronique (HA), le kit qui a été utilisé était le kit HYALURONAN QUANTIKINE® ELISA, commercialisé par R&D Systems, Inc. (614 McKinley Place NE, Minneapolis, MN 55413, U.S.A.), sous la référence catalogue DHYALO.

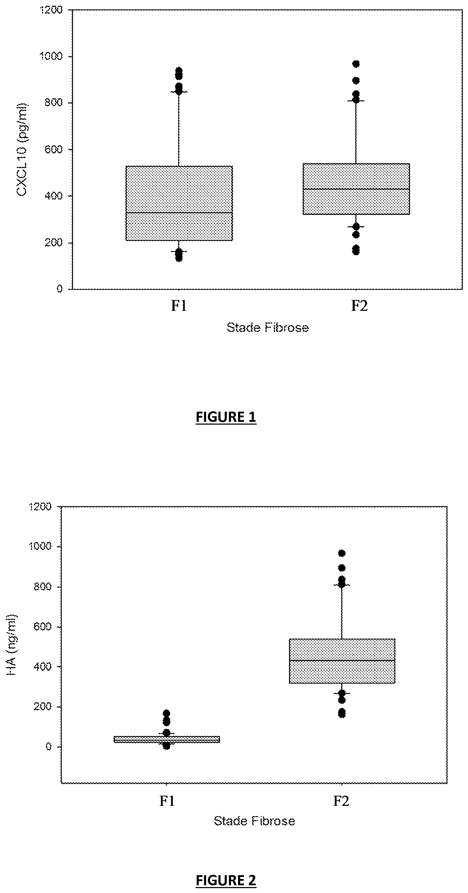

La distribution des concentrations des molécules CXCL10 et HA selon le score de fibrose hépatique est présentée en Figure 1 et Figure 2, respectivement.

Les valeurs de concentrations sériques de CXCL10 et HA ont été combinées selon la méthode mROC, afin d'établir une règle décisionnelle permettant de discriminer les patients présentant une fibrose significative (score Metavir de fibrose hépatique ≥ F2), de ceux présentant une fibrose non significative (score Metavir de fibrose hépatique < F2).
Les valeurs de concentrations sériques de CXCL10 et HA ont également été combinées selon la méthode mROC à des paramètres cliniques, tels que l'indice de masse corporelle (IMC), l'âge à la date du prélèvement (Âge) et la charge virale du patient à la date du prélèvement (CV), afin d'établir une règle décisionnelle permettant de discriminer les patients présentant une fibrose significative (score Metavir de fibrose hépatique ≥ F2), de ceux présentant une fibrose non significative (score Metavir de fibrose hépatique < F2).
La méthode mROC est la méthode *Receiver Operating Characteristic* multivariée, décrite notamment par Kramar *et al.* 1999 et Kramar *et al.* 2001.
La méthode mROC génère une fonction linéaire qui combine les différents biomarqueurs (BMQ) [Z = a(BMQ₁) + b(BMQ₂) + ...], ainsi que la valeur de référence (seuil maximisant l'indice de Youden, δ) qui confère les meilleures performances à cette fonction linéaire.

Les fonctions linéaires ainsi générées et les valeurs de référence qui leur sont respectivement associées sont indiquées dans le Tableau 2 ci-dessous.
Dans cet exemple, tout comme dans les autres exemples et partout ailleurs dans le texte de la demande, l'exposant t indiqué dans le libellé des fonctions « Z = » indique que la valeur à appliquer dans la fonction linéaire est la transformée Box-Cox (Box et Cox 1964) de la valeur mesurée pour le biomarqueur (BMQ), afin de normaliser cette valeur mesurée selon la formule suivante : BMQ^{t} = (BMQ^{λ}) - 1) / λ. Les valeurs de ces paramètres λ sont indiquées dans le Tableau 2 ci-dessous.

**Tableau 2 :**

| Biomarqueurs | **Règle** décisionnelle | | Exemple de seuil (seuil maximisant l'indice de Youden (δ)) |
|---|---|---|---|
| | Fonction linéaire (générée par mROC) | Paramètres de normalisation Box-Cox (λ) | |
| CXCL10 | Z = CXCL10^{t} | CXCL10 : -0,013 | 5,611 |
| HA | Z = HA^{t} | HA : 0,099 | 4,683 |
| **HA + CXCL10** | **Z** = (0,3686) x CXCL10^{t} + (0,3064) x HA^{t} | CXCL10 : -0,013 | 3,382 |
| | [fonction **Z1**] | HA : 0,099 | |
| **HA + CXCL10 + IMC + Âge** | **Z** = (0,3313) x CXCL10^{t} + (0,25154) x HA^{t} + (0,0143) x Âge^{t} + (- 9,8818) x IMC^{t} | CXCL10 : -0,013 | -6,263 |
| | | HA : 0,099 | |
| | | Âge: 1,086 | |
| | [fonction **Z2**] | IMC : -0,923 | |
| **HA + CXCL10 + IMC + Âge + CV** | **Z** = (0,2914) x CXCL10^{t} + (0,2569) x HA^{t} + (0,01419) x Âge^{t} + (- 9,3855) x IMC^{t} + (0,0140) x CV^{t} | CXCL10 : -0,013 | -5,730 |
| | | HA : 0,099 | |
| | | Âge: 1,086 | |
| | | IMC : -0,923 | |
| | [fonction **Z3**] | CV : 0,159 | |

Dans le Tableau 2 ci-dessus :
HA = concentration sérique en acide hyaluronique, exprimée en ng/mL
CXCL10 = concentration sérique en protéine CXCL10, exprimée en pg/mL
Âge = âge du patient à la date du prélèvement
IMC = Indice de Masse Corporelle du patient à la date du prélèvement (masse / taille²)
CV = Charge Virale du patient à la date du prélèvement, exprimée en 10³ copies par mL

Si l'on applique à un patient donné une fonction Z, alors :
- ce patient est affecté dans la classe des scores Metavir de fibrose hépatique supérieurs ou égaux à F2, si la valeur de cette fonction Z pour ce patient est supérieure ou égale à la valeur du seuil maximisant l'indice de Youden (δ) qui est associé à cette fonction Z ,
- inversement, ce patient est affecté dans la classe des scores Metavir de fibrose hépatique inférieurs à F2, si la valeur de cette fonction Z pour ce patient est inférieure à la valeur du seuil δ.

Par exemple, pour la fonction linéaire qui combine les biomarqueurs HA et CXCL10, sans les combiner à d'autres marqueurs [à savoir, la fonction **Z** = (0,3686) x CXCL10^{t} + (0,3064) x HA^{t}] :
- si la valeur de cette fonction Z pour un patient donné est supérieure ou égale la valeur de seuil de 3,382, ce patient est affecté dans la classe des scores Metavir de fibrose hépatique supérieurs ou égaux à F2,
- inversement, si la valeur de cette fonction Z pour un patient donné est inférieure à la valeur de seuil de 3,382, ce patient est affecté dans la classe des scores Metavir de fibrose hépatique inférieurs à F2.

Par exemple, pour la fonction linéaire qui combine les biomarqueurs HA et CXCL10 ainsi que les biomarqueurs IMC et Âge [à savoir, la fonction **Z** = (0,3313) x CXCL10^{t} + (0,25154) x HA^{t} + (0,0143) x Âge^{t} - (9,8818) x IMC^{t}] :
- si la valeur de cette fonction Z pour un patient donné est supérieure ou égale la valeur de seuil de -6,263, ce patient est affecté dans la classe des scores Metavir de fibrose hépatique supérieurs ou égaux à F2,
- inversement, si la valeur de cette fonction Z pour un patient donné est inférieure à la valeur de seuil de -6,263, ce patient est affecté dans la classe des scores Metavir de fibrose hépatique inférieurs à F2.

L'application de ces modèles de classification à la population de 118 patients afin de discriminer les patients présentant une fibrose significative (score Metavir de fibrose hépatique ≥ F2) de ceux présentant une fibrose non significative (score Metavir de fibrose hépatique < F2) a conduit aux résultats de sensibilité (Se), spécificité (Spé), valeur prédictive positive (VPP), valeur prédictive négative (VPN), taux de bonne classification (pourcentage de patients bien classés) et d'aire sous le courbe ROC (AUC), qui sont présentés dans le Tableau 3 ci-dessous.

**Tableau 3 :**

| Biomarqueurs | Taux de patients classés | Se | Spé | VPP | VPN | Taux de bonne classification | AUC |
|---|---|---|---|---|---|---|---|
| CXCL10 | 0% | 73% | 55% | 50% | 77% | 62% | 0,583 |
| HA | 0% | 62% | 73% | 58% | 76% | 69% | 0,681 |
| **HA + CXCL10** | 0% | **78%** | 59% | 54% | **81%** | 66% | **0,704** |
| **HA + CXCL10 + IMC + Âge** | 0% | 76% | 70% | 61% | 82% | 72% | 0,729 |
| **HA + CXCL10 + IMC + Âge + CV** | 0% | 76% | 71% | 62% | 83% | 73% | 0,731 |

Certaines performances se trouvent être améliorées par la combinaison des biomarqueurs HA et CXCL10, par rapport à ces mêmes biomarqueurs utilisés individuellement.

C'est notamment le cas de la valeur d'AUC, qui, pour la combinaison des biomarqueurs HA+CXCL10, est supérieure à 0,7, alors qu'elle est inférieure à 0,7 pour chacun des biomarqueurs utilisés individuellement :
AUC de 0,704 pour la combinaison HA+CXCL10 sans autre marqueur [AUC de 0,729 pour HA+CXCL10+IMC+Âge, et AUC de 0,731 pour HA+CXCL10+IMC+Age+CV], *versus*
AUC de 0,583 pour CXCL10 utilisé individuellement et de 0,681 pour HA utilisé individuellement,

C'est également le cas des performances de sensibilité :
sensibilité de 78% pour la combinaison HA+CXCL10 sans autre marqueur [sensibilité de 76% pour la combinaison HA+CXCL1 0+IMC+Âge ou HA+CXCL10+IMC+Âge+CV], *versus*
sensibilité de 73% pour CXCL10 utilisé individuellement et 62% pour HA utilisé individuellement.

C'est également le cas des performances de VPN :
VPN de 81% pour la combinaison HA+CXCL10 sans autre marqueur [sensibilité de 82% ou 83% pour la combinaison HA+CXCL10+IMC+Âge ou HA+CXCL10+IMC+Âge+CV], *versus*
VPN de 77% pour CXCL10 utilisé individuellement et 76% pour HA utilisé individuellement.

On constate ainsi que la combinaison des deux biomarqueurs HA et CXCL10 crée un effet synergique, allant au-delà de la simple addition des performances individuelles de ces biomarqueurs.
Cet effet synergique est inattendu, car la concentration de HA et l'expression de CXCL10 évoluent toutes deux dans le même sens. En effet, HA induit l'expression de CXCL10. Ainsi, si la concentration de HA augmente, l'expression de CXCL10 augmente également. Or, il n'était pas attendu que la combinaison de deux biomarqueurs, qui évoluent ensemble et dans le même sens, puisse apporter une information supplémentaire par rapport à celle que chacun apporte individuellement.

La combinaison de HA à CXCL10, optionnellement combinée à un ou plusieurs marqueurs additionnels (marqueur(s) clinique(s) ou anatomique(s) et/ou marqueur(s) virologique(s)), a présenté d'excellentes performances pour déterminer le stade de fibrose hépatique.

### EXEMPLE 2 : comparaison avec des tests non invasifs publiquement disponibles (même population de patients que l'exemple 1)

Les 118 patients de l'exemple 1 ont été testés à titre comparatif avec des tests de mesure du stade de fibrose hépatique qui sont par ailleurs publiquement disponibles (tests disponibles dans le commerce, ou décrits dans des articles scientifiques).
Cinq tests ont été mis en œuvre : le test HEPASCORE™, le test FIBROTEST™, le test APRI, le test FORNS et le test SHASTA.
Le test HEPASCORE™ est commercialisé par QUEST DIAGNOSTICS (3 Giralda Farms, Madison, NJ 07940, U.S.A.), et est décrit dans Adams *et al.* 2005.
Le test FIBROTEST™ est commercialisé par BIOPREDICTIVE (40, rue du Bac, 75007 Paris, France), et est décrit dans Imbert-Bismut *et al.* 2001.
Le test APRI est décrit dans Wai *et al.* 2003.
Le test FORNS est décrit dans Forns *et al.* 2002.
Le test SHASTA est décrit dans Kelleher *et al.* 2005.
Celui de ces tests qui est actuellement le plus utilisé est le test FIBROTEST™.
Chacun de ces tests a été mis en oeuvre conformément aux instructions du fabricant, ou, le cas échéant, conformément aux instructions des auteurs de l'article scientifique.
Les paramètres de ces cinq tests sont rapportés dans le Tableau 4 ci-dessous.
A2M = concentration en alpha 2 macroglobuline
GGT = concentration en gamma glutamyl transpeptidase
APOA1 = concentration en apolipoprotéine A1
Hapto = concentration en haptoglobine
Bilirubine = concentration en bilirubine totale
ASAT = concentration en aspartate aminotransférase
Plaquettes = concentration en plaquettes
Cholestérol = concentration en cholestérol total
Albumine = concentration en albumine
AST = concentration en aspartate aminotransférase
HA = concentration en acide hyaluronique
Âge = âge à la date du prélèvement
Sexe = sexe du patient (féminin ou masculin)

**Tableau 4 :**

| Nom du test | FIBROTEST™ | HEP ASCORE™ | APRI | FORNS | SHASTA | |
|---|---|---|---|---|---|---|
| Nombre de biomarqueurs impliqués dans le test | 7 | 6 | 2 | 4 | 3 | |
| | dont 5 biomarqueurs biochimiques nécessitant dosage | dont 4 biomarqueurs biochimiques nécessitant dosage | | dont 3 biomarqueurs biochimiques nécessitant dosage | | |
| Biomarqueurs biochimiques | A2M | A2M | ASAT | GGT | Albumine | |
| | GGT | GGT | Plaquettes | Plaquettes | AST | |
| | APOA1 | Bilirubine | | Cholestérol | HA | |
| | Hapto | HA | | | | |
| | Bilirubine | | | | | |
| Biomarqueurs cliniques | Âge | Âge | - | Âge | - | |
| | Sexe | Sexe | | | | |
| Seuils de référence préconisés par le test | 0,48 : ≥ F2 | ≥ 0,5 : ≥ F2 | > 1,5 : ≥ F2 | 6,9 : ≥ F2 | Seuil 1 | Seuil 2 |
| | 0,28 : < F2 | < 0,5 : < F2 | ≤ 0,5 : < F2 | <4,21 : < F2 | ≥ 0,8 : ≥ F2 | ≥ 0,3 : ≥ F2 |
| | | | | | < 0,8 : < F2 | < 0,3 : < F2 |
| | entre 0,28 et 0,47 : sujets indéterminés ne pouvant être classés | | entre 0,5 et 1,5 : sujets indéterminés ne pouvant être classés | entre 4,21 et 6,9 : sujets indéterminés ne pouvant être classés | | |

Ces cinq tests non invasifs ont été appliqués à la population de 118 patients de l'exemple 1, afin de discriminer les patients présentant une fibrose significative (score Metavir de fibrose hépatique ≥ F2), de ceux présentant une fibrose non significative (score Metavir de fibrose hépatique < F2).
Les résultats de sensibilité (Se), spécificité (Spé), valeur prédictive positive (VPP), valeur prédictive négative (VPN), taux de bonne classification et d'aire sous la courbe ROC (AUC) qui ont été obtenus, sont reportés dans le tableau 5 ci-dessous.
Le Tableau 5 ci-dessous présente en outre une comparaison de ces résultats avec ceux obtenus avec la combinaison des biomarqueurs HA+CXCL10 (combinaisons HA+CXCL10 de l'exemple 2 ci-dessus) pour la même population de patients.

**Tableau 5 :**

| | Taux de patients non classés | Se | Spé | VPP | VPN | Taux de bonne classification (*) | Taux global de bonne classification ($) |
|---|---|---|---|---|---|---|---|
| HEPASCORE™ | 0% | 67% | 75% | 63 | 79 | 72% | 72% |
| SHASTA [seuil 1] | 0% | 51% | 15% | 27 | 33 | 29% | 29% |
| SHASTA [seuil 2] | 0% | 67% | 14% | 32 | 40 | 34% | 34% |
| FIBROTEST™ | 22% | 74% | 68% | 59 | 81 | 71% | 55% |
| APRI | 42% | 26% | 96% | 71 | 77 | 76% | 44% |
| FORNS | 50% | 35% | 93% | 67 | 78 | 76% | 38% |

| Pour mémoire, les résultats de la combinaison HA+CXCL10 (combinaisons de l'exemple 1 conformes à la demande, *cf.* Tableau 3 ci-dessus) sont : | | | | | | | |
|---|---|---|---|---|---|---|---|
| HA + CXCL10 | **0%** | **78%** | 59% | 54% | **81%** | 66% | **66%** |
| HA + CXCL10 + IMC + Âge | **0%** | **76%** | 70% | 61% | **82%** | 72% | **72%** |
| HA + CXCL10 + IMC + Âge + CV | **0%** | **76%** | 71% | 62% | **83%** | 73% | **73%** |

| | | | | | | | |
|---|---|---|---|---|---|---|---|
| (*) : taux calculé sur les patients ayant pu être classés ($) : taux calculé sur la population totale (n = 118) | | | | | | | |

La combinaison de HA à CXCL10 permet d'atteindre des performances diagnostiques au moins comparables, voire supérieures, aux tests qui sont actuellement publiquement disponibles, notamment en termes de taux global de bonne classification, de taux de bonne classification, de sensibilité et de VPN.

Plus particulièrement, on observe que les performances atteintes par la combinaison de HA à CXCL10 (optionnellement combinée à un ou plusieurs marqueurs additionnels (marqueur(s) clinique(s) ou anatomique(s) et/ou marqueur(s) virologique(s))) sont très nettement supérieures à celles des tests SHASTA, APRI et FORNS.

Par rapport au test FIBROTEST™, la combinaison de HA à CXCL10 (optionnellement combinée à un ou plusieurs marqueurs additionnels (marqueur(s) clinique(s) ou anatomique(s) et/ou marqueur(s) virologique(s))) présente notamment :
- l'avantage de permettre une classification de tous les patients, alors qu'avec le test FIBROSTEST™, 22% des patients ne reçoivent pas de score de fibrose, et en outre
- l'avantage de ne requérir le dosage que de deux biomarqueurs (HA et CXCL10), alors que pour le test FIBROTEST™, cinq biomarqueurs doivent être dosés (A2M, GGT, APOA1, Hapto et Bilirubine).

Par rapport au test HEPASCORE™, la combinaison de HA à CXCL10 (optionnellement combinée à un ou plusieurs marqueurs additionnels (marqueur(s) clinique(s) ou anatomique(s) et/ou marqueur(s) virologique(s))) présente notamment l'avantage de ne requérir le dosage que de deux biomarqueurs (HA et CXCL10), alors que pour le test HEPASCORE™, quatre biomarqueurs doivent être dosés (A2M, GGT, Bilirubine et HA). On observe également que la combinaison de HA à CXCL10 (optionnellement combinée à un ou plusieurs marqueurs additionnels (marqueur(s) clinique(s) ou anatomique(s) et/ou marqueur(s) virologique(s))) présente une performance de sensibilité améliorée par rapport au test HEPASCORE™.

La combinaison de HA à CXCL10 (optionnellement combinée à un ou plusieurs marqueurs additionnels (marqueur(s) clinique(s) ou anatomique(s) et/ou marqueur(s) virologique(s))) a donc des performances diagnostiques au moins aussi satisfaisantes que celles des deux tests qui sont actuellement les plus utilisés, à savoir HEPASCORE™ et FIBROTEST™, tout en étant beaucoup plus simple (et donc plus rapide, moins coûteuse, et plus sûre) à mettre en oeuvre.

### EXEMPLE 3 : établissement et application de combinaisons HA+CXCL10 à une population de patients indépendante de celle de l'exemple 1

Une population de patients indépendante de celle de l'exemple 1 a été constituée. Cette population indépendante était formée de 310 patients de l'Hôpital Haut-Lévêque (1, avenue Magellan ; 33600 Pessac ; France) présentant une hépatite chronique suite à une infection par le virus de l'Hépatite C (VHC), et couvrant tous, ou la plupart des génotypes de VHC.
Les études réalisées ont été approuvées par le Comité Éthique local, conformément à la Déclaration d'Helsinki. Tous les patients ont donné leur consentement éclairé par écrit.

La Ponction Biopsie Hépatique (PBH) n'étant quasiment plus utilisée à l'heure actuelle, le stade de fibrose a été déterminé en utilisant une combinaison de tests non invasifs basés sur des principes différents (tests sanguins et imagerie), tel que recommandé par la communauté scientifique (*cf.* Castera 2012).
Pour ce faire, une modification de l'algorithme de classification multivariée de Castera *et al.* a été mise au point.

L'algorithme de classification multivariée de Castera *et al.* est celui décrit dans Castera *et al.* 2010 et Castera *et al.* 2014. Il est basé sur la concordance entre les résultats du FIBROTEST™ (test sanguin) et du FIBROSCAN™ (imagerie).
Cet algorithme permet la classification des patients en deux classes (absence ou présence d'une fibrose significative) lorsque les tests non invasifs FIBROTEST™ et FIBROSCAN™ sont concordants.
Lorsque les résultats des tests FIBROTEST™ et FIBROSCAN™ sont discordants, l'algorithme de Castera *et al.* recommande de pratiquer une BPH.

Pour déterminer le stade de fibrose, sans disposer et sans mettre en oeuvre une PBH, l'algorithme de Castera *et al.* a été modifié par ajout d'un deuxième niveau d'analyse. Ce deuxième niveau d'analyse est destiné aux échantillons qui donnent lieu à des résultats discordants par les tests FIBROTEST™ et FIBROSCAN™. Ce deuxième niveau d'analyse met en oeuvre plusieurs tests non invasifs, en l'occurrence FIBROTEST™ (FT), FIBROSCAN™ (FS), HEPASCORE™ (HS), APRI et FORNS.
Les tests FIBROTEST™, HEPASCORE™, APRI et FORNS ont été décrits à l'exemple 2 ci-dessus.
Le test FIBROSCAN™ est une technique d'imagerie mettant en oeuvre l'élastographie impulsionnelle, qui permet de déterminer la dureté du foie (exprimée en kPa équivalent de fibrose) ; *cf.* Castera *et al.* 2005. Le dispositif FIBROSCAN™ est commercialisé par la société ECHOSENS (30 place d'Italie, 75013 Paris, France).
Lors du deuxième niveau d'analyse, au moins trois de ces tests (au moins trois parmi FIBROTEST™ (FT), FIBROSCAN™ (FS), HEPASCORE™ (HS), APRI et FORNS) sont appliqués aux échantillons qui ont donné des résultats discordants à l'issue du premier niveau d'analyse. Le stade de fibrose obtenu avec chacun de ces tests a ainsi été déterminé. Le stade majoritairement identifié par l'ensemble de ces tests a été alloué à l'échantillon analysé. A défaut de majorité, l'algorithme modifié prévoit d'allouer à l'échantillon l'étiquette « stade de fibrose non déterminé ».

L'algorithme (ou le modèle) de classification ainsi modifié est présenté en Figure 3.
Il permet de classer les patients selon leur stade de fibrose en deux classes : fibrose significative (score Metavir de fibrose hépatique ≥ F2) et fibrose non significative (score Metavir de fibrose hépatique < F2), et cela sans recourir à une PBH.

L'application de cet algorithme modifié à la population de 310 patients formée a permis d'identifier 141 patients présentant une fibrose non significative (score Metavir de fibrose hépatique < F2) et 169 patients présentant une fibrose significative (score Metavir de fibrose hépatique ≥ F2).
Les caractéristiques des 310 patients sont présentées dans le Tableau 6 ci-dessous.

**Tableau 6 :**

| Variables | | Population totale | Patients dont le score Metavir de fibrose hépatique est < F2 | Patients dont le score Metavir de fibrose hépatique est ≥ F2 |
|---|---|---|---|---|
| n | | 310 | 141 | 169 |
| Sexe [homme (%) / femme (%)] | | 140 (45) / 170 (55) | 55 (39) / 86 (61) | 85 (50) / 84 (50) |
| Âge à la date du prélèvement [moyenne ± SD (min-max)] | | 57,9 ± 11,0 (27 - 88) | 55,2 ± 10,0 (27 - 88) | 60,2 ± 11,2 (35 - 88) |
| Alanine aminotransferase (ALT) UI/L [moyenne ± SD (min-max)] | | 87 ± 83 (16 - 795) | 54 ± 34 (16 - 271) | 113 ± 101 (21 - 795) |
| Genotype HCV [n (%)] | | | | |
| | 1 | 213 (69) | 95 (67) | 118 (70) |
| | 2 | 32 (10) | 19 (13) | 13 (8) |
| | 3 | 26 (8) | 10 (7) | 16 (9) |
| | 4 | 33 (11) | 13 (9) | 20 (12) |
| | 5 | 3 (1) | 2 (1) | 1 (1) |
| | 7 | 1 (0) | 1 (0) | 0 (0) |
| | inconnu | 2 (1) | 1 (0) | 1 (1) |
| Charge virale à la date du prélèvement (CV) : UI/mL [moyenne (min-max)] | | 1,5.10⁶ (14 - 1,1.10⁷) | 1,5.10⁶ (14-8,9.10⁷) | 1,5.10⁶ (39- 1,1.10⁷) |

| | | | | |
|---|---|---|---|---|
| SD = déviation standard ; UI = unité internationale | | | | |

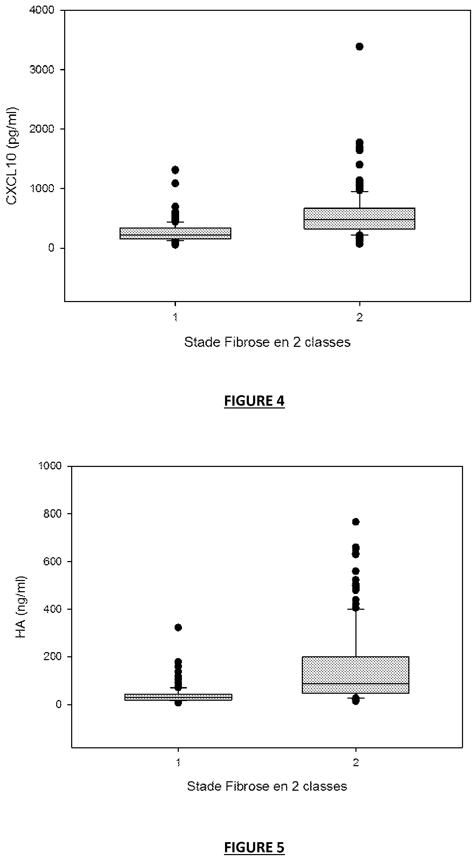

Pour chacun des 310 patients de la population, les concentrations sériques en HA et CXCL10 ont été mesurées comme décrit dans l'exemple 1 ci-dessus. La distribution de ces concentrations selon le degré de fibrose hépatique (score Metavir < F2 ou ≥ F2) est présentée en Figure 4 (CXCL10) et en Figure 5 (HA).

Les valeurs de concentrations sériques en CXCL10 et HA ont été combinées selon la méthode mROC afin d'établir une règle décisionnelle permettant de discriminer les patients présentant une fibrose significative (score Metavir ≥ F2), de ceux présentant une fibrose non significative (score Metavir < F2).
Les valeurs de concentrations sériques en CXCL10 et HA ont également été combinées selon la méthode mROC à des paramètres cliniques, tels que l'indice de masse corporelle à la date du prélèvement (IMC), et la charge virale du patient à la date du prélèvement (CV), afin d'établir une règle décisionnelle permettant de discriminer les patients présentant une fibrose significative (score Metavir ≥ F2), de ceux présentant une fibrose non significative (score Metavir < F2).
Pour une description de la méthode mROC, *cf*. notamment l'exemple 1 ci-dessus.
La fonction linéaire ainsi générée par la méthode mROC, ainsi que la valeur de référence qui confère les meilleures performances à cette fonction linéaire (seuil maximisant l'indice de Youden, δ), sont indiquées dans le Tableau 7 ci-dessous. Les valeurs des paramètres λ de la transformée Box-Cox [BMQ^{t} = (BMQ^{λ} - 1) / λ] sont également indiquées dans le Tableau 7 ci-dessous.

**Tableau 7 :**

| Biomarqueurs | **Règle** décisionnelle | | Exemple de seuil (seuil maximisant l'indice de Youden (δ)) |
|---|---|---|---|
| | Fonction linéaire (générée par mROC) | Paramètres de normalisation Box-Cox (λ) | |
| **HA** | **Z** = HA^{t} | HA : -0,288 | 2,330 |
| **CXCL10** | **Z** = CXCL10^{t} | CXCL10 : -0,116 | 4,242 |
| **HA** + **CXCL10** | **Z** = (1,999) x CXCL10^{t} + (2,852) x HA¹ | CXCL10 : -0,116 | 15,170 |
| | [fonction **Z4**] | HA : -0,288 | |
| **HA** + **CXCL10 + IMC + Âge + CV** | **Z** = (1,999) x CXCL10^{t} + (2,958) x HA^{t} + (0,616) x IMC^{t} + (- 0,053) x Âge^{t} - (0,00024) x CV^{t} | CXCL10 : -0,116 | 16,543 |
| | | HA : -0,288 | |
| | | Âge : 0,433 | |
| | [fonction **Z5**] | IMC : -0,039 | |
| | | CV : 0,279 | |

Dans le Tableau 7 ci-dessus :
HA = concentration sérique en acide hyaluronique, exprimée en ng/mL
CXCL10 = concentration sérique en protéine CXCL10, exprimée en pg/mL
Âge = âge du patient à la date du prélèvement
IMC = Indice de Masse Corporelle à la date du prélèvement (masse / taille²)
CV = Charge Virale du patient à la date du prélèvement, en UI/mL
L'exposant t indiqué dans le libellé des fonctions « Z = » indique que la valeur à appliquer dans la fonction linéaire est la transformée Box-Cox (Box et Cox 1964) de la valeur mesurée pour le biomarqueur (BMQ), afin de normaliser cette valeur mesurée selon la formule suivante : BMQ^{t} = (BMQ^{λ} - 1) / λ.

Pour l'application d'une fonction Z à un patient donné et la comparaison de la valeur de Z ainsi obtenue au seuil maximisant l'indice de Youden, *cf*. exemple 1 ci-dessus.

L'application de chacun de ces modèles de classification à la population de 310 patients afin de discriminer les patients présentant une fibrose significative (score Metavir ≥ F2), de ceux présentant une fibrose non significative (score Metavir < F2) a conduit aux résultats de sensibilité (Se), spécificité (Spé), valeur prédictive positive (VPP), valeur prédictive négative (VPN), taux de bonne classification et d'aire sous la courbe ROC (AUC), qui sont présentés dans le Tableau 8 ci-dessous.

**Tableau 8 :**

| Biomarqueurs | Taux de patients non classés | Se | Spé | VPP | VPN | Taux de bonne classification | AUC |
|---|---|---|---|---|---|---|---|
| **HA** | 0% | 76% | 79% | 82% | 74% | 78% | 0,840 |
| **CXCL10** | 0% | 72% | 76% | 78% | 69% | 74% | 0,810 |
| **HA + CXCL10** | **0%** | **78%** | **90%** | **90%** | **77%** | **83%** | **0,898** |
| **HA + CXCL10 + IMC + Âge + CV** | 0% | 80% | 87% | 88% | 78% | 83% | 0,899 |

La combinaison de HA à CXCL10 (optionnellement combiné à un ou plusieurs marqueurs additionnels (marqueur(s) clinique(s) ou anatomique(s) et/ou marqueur(s) virologique(s))) permet d'obtenir d'excellents résultats de classification (taux de patients non classés, sensibilité, spécificité, VPP et VPN), et cela avec seulement deux biomarqueurs et sans PBH.

Plus particulièrement, on observe que les performances de Se, Spé, VPP, VPN, taux de bonne classification et AUC de la combinaison HA+CXCL10 (optionnellement combiné à un ou plusieurs marqueurs additionnels (marqueur(s) clinique(s) ou anatomique(s) et/ou marqueur(s) virologique(s))) sont supérieures aux performances du marqueur HA seul, ainsi qu'à celles du marqueur CXCL10 seul.
Les performances de la combinaison HA+CXCL10 (optionnellement combiné à un ou plusieurs marqueurs additionnels (marqueur(s) clinique(s) ou anatomique(s) et/ou marqueur(s) virologique(s))) vont donc au-delà de la simple juxtaposition des performances individuelles de chacun des deux marqueurs de la combinaison.
On constate ainsi que la combinaison de HA à CXCL10 (optionnellement combinée à un ou plusieurs marqueurs cliniques, anatomiques, virologiques) crée un effet synergique inattendu, allant au-delà de la simple addition des performances individuelles des biomarqueurs HA et CXCL10 (synergie démontrée sur une population de 310 patients).

Pour plus avant démontrer que les performances de la combinaison de HA à CXCL10 vont au-delà de ce qui serait attendu de la simple addition de leurs performances respectives, un test qui ne combine pas HA à CXCL10, mais qui se contente de les juxtaposer, a été réalisé sur la population de 310 patients de l'exemple.
Ce test de juxtaposition repose sur le fait que la simple addition des performances respectives des biomarqueurs HA et CXCL10 correspond à l'addition de l'information apportée par HA à celle apportée par CXCL10 : selon ce test de juxtaposition, le test est positif (score de fibrose supérieur ou égal à F2) lorsqu'au moins un des deux biomarqueurs indique que le test est positif (score de fibrose supérieur ou égal à F2).
La classification obtenue avec le test de juxtaposition est alors la suivante :

**Tableau 9 :**

| Score de fibrose donné par le test HA seul (*cf*. exemple 3) | Score de fibrose donné par le test CXCL10 seul (*cf*. exemple 3) | Score de fibrose donné par le test qui juxtapose HA à CXCL10 |
|---|---|---|
| ≥F2 | ≥F2 | ≥F2 |
| ≥F2 | <F2 | ≥F2 |
| <F2 | ≥F2 | ≥F2 |
| <F2 | <F2 | <F2 |

Les performances de cet test de juxtaposition appliqué à la population de 310 patients de l'exemple 3 sont les suivantes : Taux de patients non classés = 0% ; sensibilité (Se) = 90% ; spécificité (Spé) = 57% ; Valeur de Prédiction Positive (VPP) = 72% ; Valeur de Prédiction Négative (VPN) = 83% ; taux de bonne classification = 75%.
La présentation de ces résultats comparés à ceux obtenus pour chacun des deux marqueurs individuellement, et comparés à ceux obtenus avec la combinaison des deux marqueurs est présentée ci-dessous.

**Tableau 10 :**

| Biomarqueurs | Taux de patients non classés | Se | Spé | VPP | VPN | Taux de bonne classification | AUC |
|---|---|---|---|---|---|---|---|
| HA seul (*cf*. Tableau 8 ci-dessus) | 0% | 76% | **79%** | **82%** | 74% | 78% | 0,840 |
| CXCL10 seul (*cf*. Tableau 8 ci-dessus) | 0% | 72% | **76%** | **78%** | 69% | 74% | 0,810 |
| HA/CXCL10 Test qui **juxtapose** HA à CXCL10 | 0% | 90% | **57%** | **72%** | 83% | 75% | NA |
| HA + CXCL10 Test qui **combine** HA à CXCL10 (*cf*. Tableau 8 ci-dessus) | **0%** | **78%** | **90%** | **90%** | **77%** | **83%** | **0,898** |

| | | | | | | | |
|---|---|---|---|---|---|---|---|
| NA = non applicable | | | | | | | |

Par rapport à chaque marqueur pris individuellement :
- la juxtaposition de HA à CXCL10 permet d'augmenter fortement la sensibilité et la VPN par rapport à chaque marqueur pris séparément ;
- par contre, la spécificité et la VPP sont diminuées, ce qui conduit à un taux de bonne classification de 75% (situé entre le taux obtenu pour HA seul et pour CXCL10 seul).
En revanche, lorsque l'on combine HA à CXCL10, on obtient non pas une diminution de la spécificité et de la VPP, mais une très forte augmentation (90% *versus* 57% pour Spé et 90% *versus* 72% pour VPP).
Cet effet synergique ne pouvait être attendu ni au vu des performances de spécificité et de VPP obtenues avec la juxtaposition des deux marqueurs, ni au vu de celles obtenues avec chacun des deux biomarqueurs pris individuellement.

En outre, le taux de bonne classification, qui permet d'évaluer globalement les performances des biomarqueurs, est très nettement supérieur avec la combinaison des deux biomarqueurs (83%) par rapport à leur juxtaposition (75%).

Un effet synergique a par ailleurs également été observé en exemple 1 ci-dessus (population de 118 patients).
Cet effet synergique n'est donc pas lié à la règle décisionnelle utilisée, mais bien de manière plus générale, à la combinaison HA+CXCL10.

Pour illustrer l'effet synergique de la combinaison HA+CXCL10 par rapport à HA seul ou CXCL10 seul, la méthode de ré-échantillonnage connue sous le nom de *Bootstrap* (*cf*. Efron 1979) a été mise en œuvre de sorte à évaluer le gain apporté par la combinaison HA+CXCL10.

À partir de la population de 310 patients, 1000 sous-populations de même taille ont été tirées aléatoirement (tirage avec remise, de sorte qu'un même patient peut être présent plusieurs fois au sein d'une même sous-population).
Les valeurs d'AUC et de Taux de bonne classification ont été mesurées sur chacune de ces 1000 sous-populations:
- d'une part, lorsque la règle décisionnelle mROC appliquée a pour coefficients ceux initialement établis sur la population de 310 patients (« coefficients fixes » ou « coef fix »), et
- d'autre part, lorsque la règle décisionnelle mROC appliquée a pour coefficients ceux établis pour chacune des 1000 sous-populations (« coefficients optimisés » ou « coef optim »).
La valeur d'AUC est la valeur de l'aire sous la courbe ROC.
La valeur du Taux de bonne classification est la valeur du pourcentage de patients bien classés.

Les résultats sont présentés en Figures 13A, 13B, 14A et 14B.

Les Figures 13A et 13B présentent sous forme d'histogrammes les valeurs des différences d'AUC et de précision qui ont été mesurées entre d'une part la règle décisionnelle mROC à « coefficients fixes » (ou « coef fix ») et d'autre part la règle décisionnelle mROC à « coefficients optimisés » (« coef optim »).
En Figures 13A et 13B, la règle décisionnelle mROC mettait en œuvre la combinaison HA+CXCL10.
La Figure 13A présente l'histogramme des différences d'AUC entre les coefficients fixes et les coefficients optimisés pour la combinaison HA+CXCL10 en *Bootstrap* (B = 1 000) [axe des abscisses : 0,000 ; 0,005 ; 0,010].
La Figure 13B présente l'histogramme des différences de taux de bonne classification (pourcentage de patients bien classés) avec les coefficients fixes ou optimisés pour la combinaison HA+CXCL10 en *Bootstrap* (B = 1 000).

Comme illustré en Figures 13A et 13B, il a été constaté :
- qu'en terme d'AUC, la moyenne de la différence est de 0,001 (avec un intervalle de confiance à 95% de [-0,002 ; 0,008]) ; et
- qu'en terme de taux de bonne classification, la moyenne de la différence est de 1,04 (avec un intervalle de confiance à 95% de [-0,33 ; 3,55]).

Les Figures 13A et 13B montrent qu'il y a peu d'écart de performances que l'on applique des coefficients fixes ou des coefficients qui sont optimisés pour la population étudiée.

Ainsi, les Figures 13A et 13B démontrent que les performances ne sont pas dues aux coefficients mais aux marqueurs eux-mêmes, puisque quels que soient les coefficients mis en œuvre (coefficients optimisés ou coefficients fixes), les performances obtenues sont comparables.

Les Figures 14A et 14B présentent sous forme d'histogrammes les valeurs des différences d'AUC et de taux de bonne classification qui ont été mesurées pour la règle décisionnelle mROC entre d'une part la combinaison HA+CXCL10 et d'autre part le marqueur HA seul.
La Figure 14A présente l'histogramme des différences d'AUC entre la combinaison HA+CXCL10 d'une part et le marqueur HA seul d'autre part, en *Bootstrap* (B = 1 000) avec les coefficients fixes (« coef fix ») [axe des abscisses : 0,02 ; 0,04 ; 0,06 ; 0,08 ; 0,10; 0,12].
La Figure 14B présente l'histogramme des différences de taux de bonne classification (pourcentage de patients bien classés) entre la combinaison HA+CXCL10 d'une part et le marqueur HA seul d'autre part, en *Bootstrap* (B = 1 000) avec les coefficients fixes (« coef fix »).

Comme illustré en Figures 14A et 14B, il a été constaté :
- qu'en terme d'AUC, le gain de la combinaison HA+CXCL10 par rapport au marqueur HA seul est toujours supérieur à zéro, la moyenne de cette différence étant de 0,06 (avec un intervalle de confiance à 95% de [0,027 ; 0,092]), et
- qu'en terme de taux de bonne classification, le gain de la combinaison HA+CXCL10 par rapport au marqueur HA seul est supérieur à zéro dans 99% des cas, la moyenne de la différence étant de 5,5% (avec un intervalle de confiance à 95% de [1,29 ; 10]).

Ces gains viennent confirmer que la combinaison HA+CXCL10 (optionnellement combinée à un ou plusieurs marqueurs cliniques, anatomiques, virologiques) crée un effet synergique inattendu, allant au-delà de la simple addition des performances individuelles des biomarqueurs HA et CXCL10, et que cet effet synergique est indépendant du modèle de classification utilisé.

### EXEMPLE 4 : comparaison avec des tests non invasifs publiquement disponibles (même population de patients que l'exemple 3)

Les 310 patients de l'exemple 3 ont été testés à titre comparatif avec des tests de mesure du stade de fibrose hépatique qui sont par ailleurs publiquement disponibles (tests disponibles dans le commerce, ou décrits dans des articles scientifiques).
Cinq tests ont été mis en œuvre : le test HEPASCORE™, le test FIBROTEST™, le test APRI, le test FORNS et le test FIBROSCAN™.
Le test HEPASCORE™ est commercialisé par QUEST DIAGNOSTICS (3 Giralda Farms, Madison, NJ 07940, U.S.A.), et est décrit dans Adams *et al.* 2005.
Le test FIBROTEST™ est commercialisé par BIOPREDICTIVE (40, rue du Bac, 75007 Paris, France), et est décrit dans Imbert-Bismut *et al.* 2001.
Le test APRI est décrit dans Wai *et al.* 2003.
Le test FORNS est décrit dans Forns *et al.* 2002.
Le test FIBROSCAN™ est une technique d'imagerie mettant en œuvre l'élastographie impulsionnelle, qui permet de déterminer la dureté du foie (exprimée en kPa équivalent de fibrose) ; *cf*. Castera *et al.* 2005. Le dispositif FIBROSCAN™ est commercialisé par la société ECHOSENS (30 place d'Italie, 75013 Paris, France).
Chacun de ces tests a été mis en œuvre conformément aux instructions du fabricant, ou, le cas échéant, conformément aux instructions des auteurs de l'article scientifique.
Les paramètres de ces cinq tests sont rapportés dans le Tableau 11 ci-dessous.
A2M = concentration en alpha 2 macroglobuline
GGT = concentration en gamma glutamyl transpeptidase
APOA1 = concentration en apolipoprotéine A1
Hapto = concentration en haptoglobine
Bilirubine = concentration en bilirubine totale
ASAT = concentration en aspartate aminotransférase
Plaquettes = concentration en plaquettes
Cholestérol = concentration en cholestérol total
HA = concentration en acide hyaluronique
Âge = âge à la date du prélèvement
Sexe = sexe du patient (féminin ou masculin)

**Tableau 11 :**

| Nom du test | FIBROTEST™ | HEPASCORE™ | APRI | FORNS | FIBROSCAN™ |
|---|---|---|---|---|---|
| Nombre de biomarqueurs impliqués dans le test | 7 | 6 | 2 | 4 | 0 |
| | dont 5 biomarqueurs biochimiques nécessitant dosage | dont 4 biomarqueurs biochimiques nécessitant dosage | | dont 3 biomarqueurs biochimiques nécessitant dosage | (mesure de l'élastométrie en kPa) |
| Biomarqueurs biochimiques | A2M | A2M | ASAT | GGT | - |
| | GGT | GGT | Plaquettes | Plaquettes | |
| | APOA1 | Bilirubine | | Cholestérol | |
| | Hapto | HA | | | |
| | Bilirubine | | | | |
| Biomarqueurs | Âge | Âge | - | Âge | - |
| cliniques | Sexe | Sexe | | | |
| Seuils de référence préconisés par le test | 0,48 : ≥ F2 | ≥ 0,5 : ≥ F2 | > 1,5 : ≥ F2 | > 6,9 : ≥ F2 | < 7.1 : < F2 |
| | 0,28 : < F2 | < 0,5 : < F2 | ≤ 0,5 : < F2 | < 4,21 : < F2 | ≥ 7.1 : ≥ F2 |
| | | | | | ≥ 9.5 : ≥ F3 |
| | entre 0,28 et 0,47 : sujets indéterminés ne pouvant être classés | | entre 0,5 et 1,5 : sujets indéterminés ne pouvant être classés | entre 4,21 et 6,9 : sujets indéterminés ne pouvant être classés | ≥12.5 : F4 |

Ces cinq tests non invasifs ont été appliqués à la population de 310 patients de l'exemple 3, afin de discriminer les patients présentant une fibrose significative (score Metavir de fibrose hépatique ≥ F2), de ceux présentant une fibrose non significative (score Metavir de fibrose hépatique < F2).
Les résultats de sensibilité (Se), spécificité (Spé), valeur prédictive positive (VPP), valeur prédictive négative (VPN), taux de bonne classification et d'aire sous la courbe ROC (AUC), qui ont été obtenus sont reportés dans le Tableau 12 ci-dessous.
Le Tableau 12 ci-dessous présente en outre une comparaison de ces résultats avec ceux obtenus avec la combinaison des biomarqueurs HA+CXCL10 (combinaisons HA+CXCL10 de l'exemple 3 ci-dessus) pour la même population de patients.

**Tableau 12 :**

| Mesures faites et traitées conformément aux indications du fabricant ou des auteurs | Taux de patients non classés | Se | Spé | VPP | VPN | Taux de bonne classification (*) | Taux global de bonne classification ($) |
|---|---|---|---|---|---|---|---|
| **HEPASCORE™** | 0% | 95% | 90% | 94% | 90% | 93% | 93% |
| **FIBROSCAN™** | 0% | 62% | 94% | 95% | 58% | 74% | 74% |
| **FIBROTEST™** | 15% | 100% | 58% | 81% | 100% | 85% | 72% |
| **FORNS** | 54% | 49% | 44% | 100% | 100% | 47% | 45% |
| **APRI** | 45% | 36% | 64% | 98% | 81% | 46% | 50% |

| Pour mémoire, les résultats de la combinaison HA+CXCL10 (combinaisons de l'exemple 3 conformes à la demande, *cf*. Tableau 8 ci-dessus) sont : | | | | | | | |
|---|---|---|---|---|---|---|---|
| HA + CXCL10 | **0%** | **78%** | **90%** | **90%** | **77%** | 83% | 83% |
| HA + CXCL10 + IMC + Âge + CV | **0%** | **80%** | **87%** | **88%** | **78%** | 83% | 83% |

| | | | | | | | |
|---|---|---|---|---|---|---|---|
| (*) : taux calculé sur les patients ayant pu être classés ($) : taux calculé sur la population totale (n = 310) | | | | | | | |

On observe que les performances atteintes par la combinaison de HA à CXCL10 (optionnellement combinée à un ou plusieurs marqueurs additionnels (marqueur(s) clinique(s) ou anatomique(s) et/ou marqueur(s) virologique(s))) sont très nettement supérieures à celles des tests APRI et FORNS.

Par rapport au test FIBROTEST™, la combinaison de HA à CXCL10 (optionnellement combinée à un ou plusieurs marqueurs additionnels (marqueur(s) clinique(s) ou anatomique(s) et/ou marqueur(s) virologique(s))) présente notamment :
- l'avantage de permettre une classification de tous les patients, alors qu'avec le test FIBROSTEST™, 15% des patients de la population ne peuvent recevoir de score de fibrose,
- l'avantage de présenter de meilleures performances de spécificité et VPP (en sus du meilleur taux de bonne classification), et en outre
- l'avantage de ne requérir le dosage que de deux biomarqueurs (HA et CXCL10), alors que pour le test FIBROTEST™, cinq biomarqueurs doivent être dosés (A2M, GGT, APOA1, Hapto et Bilirubine).

Par rapport au test HEPASCORE™, la combinaison de HA à CXCL10 (optionnellement combinée à un ou plusieurs marqueurs additionnels (marqueur(s) clinique(s) ou anatomique(s) et/ou marqueur(s) virologique(s))) présente notamment l'avantage de ne requérir le dosage que de deux biomarqueurs (HA et CXCL10), alors que pour le test HEPASCORE™, quatre biomarqueurs doivent être dosés (A2M, GGT, Bilirubine et HA).

Il est à noter que les performances des différents tests non invasifs ici mesurées avec les tests HEPASCORE™, FIBROTEST™, FIBROSCAN™, APRI et FORNS (exemple 4) sont probablement favorisées, étant donné que ces tests font partie du référentiel qui a été utilisé pour déterminer le stade de fibrose des patients (*cf*. exemple 3 ci-dessus ; *cf*. Figure 3). Ce n'était pas le cas pour les mesures comparatives de l'exemple 2 ci-dessus (stade de fibrose déterminé par PBH).

La combinaison de HA à CXCL10 (optionnellement combinée à un ou plusieurs marqueurs additionnels (marqueur(s) clinique(s) ou anatomique(s) et/ou marqueur(s) virologique(s))) a donc des performances diagnostiques très satisfaisantes, tout en étant beaucoup plus simple (et donc plus rapide, moins coûteuse, et plus sûre) à mettre en œuvre que les tests non invasifs qui sont actuellement disponibles.

### EXEMPLE 5 : combinaison des biomarqueurs HA et CXCL10 et de FIBROSCAN™

Les résultats du test FIBROSCAN™ des 310 patients de l'exemple 4 ont été traités et analysés par la méthode mROC, seuls ou en combinaison avec les résultats du test HA+CXCL10 de ces mêmes patients (résultats HA+CXCL10 décrits en exemple 3 ci-dessus).
Pour une description de la méthode mROC, *cf*. notamment l'exemple 1 ci-dessus.
La fonction linéaire ainsi générée par la méthode mROC et la valeur de référence (seuil maximisant l'indice de Youden, δ), qui confère les meilleures performances à cette règle décisionnelle, sont indiquées dans le Tableau 13 ci-dessous. Les valeurs des paramètres λ de la transformée Box-Cox [BMQ^{t} = (BMQ^{λ} - 1) / λ] sont également indiquées dans le Tableau 13 ci-dessous.

**Tableau 13 :**

| Biomarqueurs | **Règle** décisionnelle | | Exemple de seuil (seuil maximisant l'indice de Youden (δ)) |
|---|---|---|---|
| | Fonction linéaire (générée par mROC) | Paramètres de normalisation Box-Cox (λ) | |
| **FS** | **Z** = FS^{t} | HA : -0,888 | 0,893 |
| **HA + CXCL10** | **Z** = (1,999) x CXCL10^{t} + (2,852) x HA^{t} | CXCL10 : -0,116 | 15,170 |
| | [fonction **Z4**] | HA : -0,288 | |
| **HA + CXCL10 + FS** | **Z** = (1,686) x CXCL10^{t} + (2,216) x HA^{t} + (6,947) x FS^{t} | CXCL10 : -0,116 | 18,375 |
| | | HA : -0,288 | |
| | [fonction **Z6**] | FS : -0,888 | |

Dans le Tableau 13 ci-dessus :
HA = concentration sérique en acide hyaluronique, exprimée en ng/mL
CXCL10 = concentration sérique en protéine CXCL10, exprimée en pg/mL
FS = mesure de dureté du foie exprimée en kPa équivalent fibrose (FIBROSCAN™)

Pour l'application d'une fonction Z à un patient donné et la comparaison de la valeur de Z ainsi obtenue au seuil maximisant l'indice de Youden, *cf*. exemple 1 ci-dessus.

L'application des fonctions ci-dessus aux 310 patients de la population a permis leur classification en stade de fibrose < F2 and ≥ F2 (score Metavir). Les résultats de sensibilité (Se), spécificité (Spé), valeur de prédiction négative (VPN), valeur de prédiction positive (VPP=, taux de bonne classification et d'aire sous la courbe ROC (AUC) sont présentés dans le Tableau 14 ci-dessous.

**Tableau 14 :**

| | Se | Spé | VPP | VPN | Taux de bonne classification | AUC |
|---|---|---|---|---|---|---|
| FS | 76% | 82% | 83% | 74% | 78% | 0,858 |
| HA+CXCL10 | **78%** | **90%** | **90%** | **77%** | **83%** | **0,898** |
| HA+CXCL10+FS | **83%** | **89%** | **90%** | **81%** | **86%** | **0,931** |

| Pour mémoire : | | | | | | |
|---|---|---|---|---|---|---|
| HA | 76% | 79% | 82% | 74% | 78% | 0,840 |
| (*cf*. exemple 3, Tableau 8 ci-dessus) | | | | | | |
| CXCL10 | 72% | 76% | 78% | 69% | 74% | 0,810 |
| (*cf*. exemple 3, Tableau 8 ci-dessus) | | | | | | |

La combinaison de HA à CXCL10 donne de meilleures performances diagnostiques que le test FIBROSCAN™ seul.
La combinaison de HA à CXCL10 et en outre à FS donne encore de meilleures performances.

### EXEMPLE 6 : établissement et application de combinaisons HA+CXCL10 pour une classification fine des stades de fibrose (F0-F1/F2-F3/F4) (même population de patients que l'exemple 3)

Pour chacun des 310 patients de la population de l'exemple 3, une détermination fine du stade de fibrose a été conduite à l'aide d'un algorithme de classification multivariée.
Cet algorithme a été mis au point par modification de l'algorithme de Boursier *et al.* 2012 (*cf*. Figure 2C de Boursier *et al.* 2012).
L'algorithme de Boursier *et al.* 2012 a été modifié de sorte à permettre la classification des patients qui présentent des résultats discordants au FIBROSCAN™ et au FIBROSTEST™, sans avoir à recourir à la PBH, tout en permettant une classification fine du stade de fibrose (F0-F1/F2-F3/F4).
L'algorithme ainsi modifié est présenté en Figure 6.
L'algorithme modifié comprend un deuxième niveau d'analyse (*cf*. Figure 6), qui met en œuvre la combinaison de plusieurs tests non invasifs, en l'occurrence FIBROSCAN™ (FS), HEPASCORE™ (HS), APRI et FORNS.
Le test FIBROSCAN™ a été décrit à l'exemple 3 ci-dessus.
Les tests HEPASCORE™, APRI et FORNS ont été décrits à l'exemple 2 ci-dessus.
Lors du deuxième niveau d'analyse, au moins trois de ces tests sont appliqués aux échantillons qui ont donné des résultats discordants à l'issue du premier niveau d'analyse. Le stade de fibrose obtenu avec chacun de ces tests a ainsi été déterminé. Le stade majoritairement identifié par l'ensemble de ces tests a été alloué à l'échantillon analysé. À défaut de majorité, l'algorithme modifié prévoit d'allouer à l'échantillon l'étiquette « stade de fibrose non déterminé ».

Les caractéristiques des patients 310 patients sont présentées dans le Tableau 15 ci-dessous.

**Tableau 15 :**

| Variables | | Population totale | Patients F0-F1 | Patients F2-F3 | Patients F4 |
|---|---|---|---|---|---|
| n | | 310 | 141 | 128 | 41 |
| Sexe [homme (%) / femme(%)] | | 140 (45) / 170 (55) | 55 (39) / 86 (61) | 57 (45) / 71 (55) | 28 (68) / 13 (32) |
| Âge à la date du prélèvement [moyenne ± SD (min-max)] | | 57,9 ± 11,0 (27 -88) | 55,2 ± 10,0 (27 - 88) | 60,7 ± 11,3 (35 - 88) | 58,9 ± 11,0 (41 - 85) |
| Alanine aminotransferase (ALT) UI/L [moyenne ± SD (min-max)] | | 87 ± 83 (16 - 795) | 54 ± 34 (16 - 271) | 103 ± 91 (21 - 795) | 149 ± 121 (23 - 741) |
| Génotype HCV [n (%)] | | | | | |
| | 1 | 213 (69) | 95(67) | 90 (70) | 28 (68) |
| | 2 | 32 (10) | 19 (13) | 9 (7) | 4 (10) |
| | 3 | 26 (8) | 10 (7) | 12 (9) | 4 (10) |
| | 4 | 33 (11) | 13 (9) | 15 (12) | 5 (12) |
| | 5 | 3 (1) | 2 (1) | 1 (0) | 0(0) |
| | 7 | 1 (0) | 1 (0) | 0 (0) | 0 (0) |
| | inconnu | 2 (1) | 1 (0) | 1 (0) | 0(0) |
| Charge virale à la date du prélèvement (CV): UI/mL [moyenne (min-max)] | | 1,5.10⁶ (14 - 1,1.10⁷) | 1,5.10⁶ (14 - 8,9.10⁷) | 1,7.10⁶ (39 - 1,1.10⁷) | 1,0.10⁶ (1,3.10³-4,2.10⁶) |

| | | | | | |
|---|---|---|---|---|---|
| SD = déviation standard ; UI = unité internationale | | | | | |

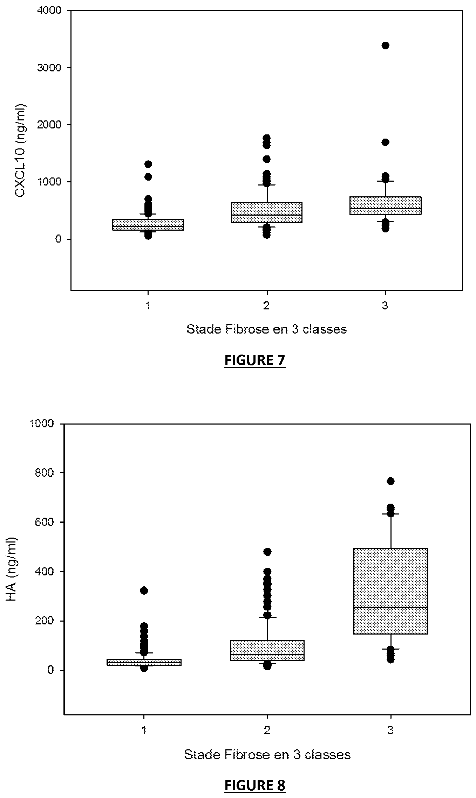

Pour chacun des 310 patients de la population, les concentrations sériques en HA et CXCL10 ont été mesurées comme décrit dans l'exemple 1 ci-dessus. La distribution de ces concentrations selon le degré de fibrose hépatique (score Metavir F0-F1 ou F2-F3 ou F4) est présentée en Figure 7 (CXCL10) et en Figure 8 (HA). Les mesures de FIBROSCAN™ (dureté du foie exprimée en kPa équivalent fibrose) ont été comparées à la valeur seuil préconisée par ce test pour classer le stade de fibrose hépatique par rapport au score Metavir F4, à savoir le seuil de 12,5 kPa. Conformément aux instructions du fabricant :
- ceux des patients dont la valeur de mesure par FIBROSCAN™ (FS) était inférieure à 12,5 kPa ont été affectés à la classe « score Metavir de fibrose hépatique inférieur à F4 », et
- ceux des patients dont la valeur de mesure par FIBROSCAN™ (FS) était supérieure ou égale à 12,5 kPa ont été affectés à la classe « score Metavir de fibrose hépatique égal à F4 ».
Les patients dont le score Metavir de fibrose hépatique a ainsi été déterminé comme étant inférieur à F4 ont été plus avant classifiés à l'aide de la combinaison de marqueurs HA+CXCL10 (2^{ème} niveau d'analyse). À cet effet, les valeurs de concentrations sériques en CXCL10 et HA ont été combinées selon la méthode mROC afin d'établir une règle décisionnelle permettant de déterminer le stade de fibrose (score Metavir F0-F1 ou F2-F3). Les valeurs de concentrations sériques en CXCL10 et HA ont également été combinées :
- à des paramètres cliniques, tels que l'indice de masse corporelle (IMC), l'âge et la charge virale du patient (CV),
- aux valeurs mesurées par FIBROSCAN™,
conformément à la méthode mROC, afin d'établir une règle décisionnelle permettant de déterminer le stade de fibrose (score Metavir F0-F1 ou F2-F3).
Pour une description de la méthode mROC, *cf*. notamment l'exemple 1 ci-dessus. La fonction linéaire ainsi générée par la méthode mROC et la valeur de référence (seuil maximisant l'indice de Youden, δ), qui confère les meilleures performances à cette règle décisionnelle, sont indiquées dans le Tableau 16 ci-dessous. Les valeurs des paramètres λ de la transformée Box-Cox [BMQ^{t} = (BMQ^{λ} - 1) / λ] sont également indiquées dans le Tableau 16 ci-dessous. Les figures 9, 10 et 11 présentent une illustration de l'algorithme de classification multivariée qui a ainsi été mis au point et appliqué.

**Tableau 16 :**

| Biomarqueurs | **Règle** décisionnelle | | Exemple de seuil (seuil maximisant l'indice de Youden (δ)) | Algorithme de classification multivariée |
|---|---|---|---|---|
| | Fonction linéaire (générée par mROC) | Paramètres de normalisation Box-Cox (λ) | | |
| HA+CXCL10 | **Z** = (1,849) x CXCL10^{t} + (2,368) x HA^{t} | HA : -0,27 | 13,5 | Figure 11 |
| | [fonction **Z7**] | CXCL10 : - 0,116 | | |
| HA+CXCL10+IMC+Âge+CV | **Z** = (1,853) x CXCL10^{t} + (2,511) x HA^{t} + (-0,00027) x CV^{t} + (- 0,0343) x Âge^{t} + (0,4246) x IMC^{t} | HA : -0,27 | 14,7 | Figure 12 |
| | | CXCL10 : - 0,116 | | |
| | | CV : 0,288 | | |
| | [fonction **Z8**] | Âge : 0,536 | | |
| | | IMC : 0,056 | | |
| HA+CXCL10+FS | **Z** = (1,585) x CXCL10^{t} + (2,181) x HA^{t} + (2,910) x FS^{t} | HA : -0,27 | 15,7 | Figure 13 |
| | | CXCL10 : - 0,116 | | |
| | [fonction **Z9**] | FS : -0,27 | | |

| | | | | |
|---|---|---|---|---|
| FS = mesure de dureté du foie exprimée en kPa équivalent fibrose (FIBROSCAN™) | | | | |

Dans le Tableau 16 ci-dessus :
HA = concentration sérique en acide hyaluronique, exprimée en ng/mL
CXCL10 = concentration sérique en protéine CXCL10, exprimée en pg/mL
FS = FIBROSCAN™ = mesure de dureté du foie exprimée en kPa équivalent de fibrose Âge = âge du patient à la date du prélèvement
IMC = Indice de Masse Corporelle à la date du prélèvement (masse / taille²)
CV = Charge Virale du patient à la date du prélèvement, en UI/mL
L'exposant t indiqué dans le libellé des fonctions « Z = » indique que la valeur à appliquer dans la fonction linéaire est la transformée Box-Cox (Box et Cox 1964) de la valeur mesurée pour le biomarqueur (BMQ), afin de normaliser cette valeur mesurée selon la formule suivante : BMQ^{t} = (BMQ^{λ} - 1) / λ.

Pour l'application d'une fonction Z à un patient donné et la comparaison de la valeur de Z ainsi obtenue au seuil maximisant l'indice de Youden, *cf*. exemple 1 ci-dessus.

Chacune des fonctions Z indiquées dans le Tableau 16 ci-dessus peut être directement appliquée à un patient dont le score Metavir de fibrose hépatique est inférieur à F4.
Si le score de fibrose hépatique du patient est connu, et si ce score est inférieur au score Metavir F4, un examen par FIBROSCAN™ (ni aucun autre examen) n'est préalablement pas nécessaire : une formule Z du Tableau 16 peut être directement appliquée aux données de ce patient.
Si, par contre, le score de fibrose hépatique du patient n'est pas connu, un examen doit être mené de sorte à déterminer si ce score est inférieur à F4, par exemple par FIBROSCAN™ pour alors procéder comme illustré en Figure 9, 10 ou 11 selon la formule Z choisie.

L'application de cette classification aux 310 patients de la population a permis de classer les patients en 3 stades de Fibrose (score Metavir de fibrose hépatique F0-F1 ou F2-F3 ou F4) avec les taux de bonne classification qui sont présentés dans le Tableau 17 ci-dessous.

**Tableau 17 (taux de bonne classification) :**

| Combinaison appliquée à ceux des patients dont le score Metavir de fibrose hépatique est inférieur à F4 | Taux de bonne classification | | | |
|---|---|---|---|---|
| | Score Metavir de fibrose hépatique | | | Population totale |
| | F0-F1 | F2-F3 | F4 (score connu ou déterminé par FIBROSCAN™) | |
| Combinaison HA+CXCL10 | **89%** | **69%** | 100% | 82% |
| Combinaison HA+CXCL 10+C V+Âge+IMC | **84%** | **73%** | 100% | 82% |
| Combinaison HA+CXCL10+FS | **82%** | **84%** | 100% | 85% |

La combinaison de HA à CXCL10, optionnellement combinée à un ou plusieurs marqueurs additionnels (marqueur(s) clinique(s) ou anatomique(s) et/ou marqueur(s) virologique(s)), permet d'atteindre d'excellents taux de bonne classification parmi la population des patients dont le score Metavir de fibrose hépatique est inférieur à F4.

### EXEMPLE 7 :

- **les méthodes qui ne sont pas basées sur une fonction linéaire peuvent être utilisées ; et**
- **démonstration expérimentale d'une formulation mROC générale**

La méthode mROC est une méthode de classification multivariée basée sur une fonction linéaire. Pour une description de la méthode mROC, *cf*. exemple 1. Pour des exemples de fonctions linéaires mROC, *cf*. exemples 1 à 6 ci-dessus.
Les méthodes de classification mutlivariée, qui comme la méthode mROC sont basées sur une fonction linéaire ne sont pas les seules méthodes pouvant être mises en œuvre pour établir un modèle de classification des patients à partir de la combinaison des biomarqueurs CXCL10 et HA.
En effet, des méthodes de classification multivariée qui ne sont pas basées sur une fonction linéaire peuvent être utilisées. Par exemple, peuvent être utilisées :
- les méthodes de classification multivariée qui sont basées sur une fonction qui n'est pas une fonction linéaire, par exemple les méthodes de classification multivariée qui sont basées sur une fonction affine, telle que la méthode de Régression Logistique (LR), ou
- les méthodes de classification multivariée qui ne sont pas basées sur une fonction (mathématique), telles que les méthodes basées sur un arbre de décision, par exemple la méthode CART (*Classification And Regression Tree*).
Le modèle de Régression Logistique a été décrit par Berkson 1944.
Le modèle CART a été décrit par Breiman *et al.* 1984.

La population de patients est identique à celle présentée en exemple 3 ci-dessus. Cette population était constituée de 310 patients dont le stade de fibrose a été déterminé selon l'algorithme de classification présenté en Figure 3. Les caractéristiques de ces patients sont présentées dans le Tableau 6 ci-dessus.

Un groupe d'apprentissage et un groupe de validation ont été tirés aléatoirement à 10 reprises parmi la population de 310 patients.
Le groupe d'apprentissage était constitué de 2/3 des patients, soit 207 patients, et le groupe de validation était constitué de 1/3 des patients, soit 103 patients.

Trois méthodes ont été comparées :
- la méthode mROC (à fonction linéaire),
- la méthode LR (à fonction affine), et
- la méthode CART (pas de fonction mathématique ; classification par arbre de décsion).

Les fonctions mROC utilisées sont celles de l'exemple 3 ci-dessus (*cf*. Tableau 7 ci-dessus). Pour mémoire :

**Tableau 18 :**

| Biomarqueurs | **Règle** décisionnelle | | Exemple de seuil (seuil maximisant l'indice de Youden (δ)) |
|---|---|---|---|
| | Fonction linéaire (générée par mROC) | Paramètres de normalisation Box-Cox (λ) | |
| **HA** | **Z** = HA^{t} | HA : -0,288 | 2,330 |
| **CXCL10** | **Z** = CXCL10^{t} | CXCL10 : -0,116 | 4,242 |
| **HA + CXCL10** | **Z** = (1,999) x CXCL10^{t} + (2,852) x HA^{t} | CXCL10 : -0,116 | 15,170 |
| | [fonction Z4] | HA : -0,288 | |

L'exposant t indiqué dans le libellé des fonctions « Z = » indique que la valeur à appliquer dans la fonction linéaire est la transformée Box-Cox (Box et Cox 1964) de la valeur mesurée pour le biomarqueur (BMQ), afin de normaliser cette valeur mesurée selon la formule suivante : BMQ^{t} = (BMQ^{λ} - 1) / λ.

Les modèles de classifications CART et LR ont été établis sur les groupes d'apprentissage (*cf*. Tableau 19 ci-dessous).

**Tableau 19 :**

| Méthode | Marqueur(s) | Règle décisionnelle | | Exemple de seuil(s) |
|---|---|---|---|---|
| | | Fonction ou arbre de décision | Paramètres | |
| CART | HA+CXC110 | *cf*. Figure 12 | NA | h ∈ [42,18 ; 77,4] |
| | | | | i ∈ [209,3 ; 266,7] |
| | | | | j ∈ [454,7 ; 553,1] |
| | | | | [arbre **CART₃**] |
| | HA | si HA < seuil, score est < F2 | NA | seuil ∈ [42,18 ; 77,4] |
| | | si HA ≥ seuil, score est ≥ F2 | | |
| | CXCL10 | si CXCL10 < seuil, score est < F2 | NA | seuil ∈ [220,8 ; 440,3] |
| | | si CXCL10 ≥ seuil, score est ≥ F2 | | |
| LR | HA+CXCL10 | LOGIT = Intercept + k(CXCL10) + l(HA) | Intercept ∈ [-3,57 ; -2,67] | Logit = 0,5 |
| | | | k ∈ [0,003 ; 0,007] | |
| | | [fonction **LOGIT₃**] | l ∈ [0,02 ; 0,04] | |
| | HA | LOGIT = Intercept + 1(HA) | Intercept ∈ [-2,13 ; -1,36] | |
| | | | l ∈ [0,02 ; 0,04] | |
| | CXCL10 | LOGIT = Intercept + k(CXCL10) | Intercept ∈ [-2.41 ; -1.55] | |
| | | | k ∈ [0.005 ; 0.007] | |

Les valeurs des paramètres indiquées dans le Tableau 19 ci-dessus (méthode LR) correspondent aux bornes inférieure et supérieure des valeurs des paramètres des 10 populations tirées au hasard.

Les performances ont été validées sur le groupe de validation (score Metavir de fibrose hépatique inférieur à F2, ou score Metavir de fibrose hépatique supérieur ou égal à F2). La valeur moyenne des résultats de sensibilité (Se), de spécificité (Spé), de valeur de prédiction négative (VPN), de valeur de prédiction positive (VPP), d'aire sous la courbe ROC (AUC) et de taux de bonne classification, qui ont été obtenus sur l'ensemble des 10 réplicats pour le groupe d'apprentissage et pour le groupe de validation sont présentés dans le Tableau 20 et dans le Tableau 21 ci-dessous, respectivement.

**Tableau 20 :**

| | | Groupe d'apprentissage n = 207 | | | | | |
|---|---|---|---|---|---|---|---|
| Combinaison | Méthode | Se | Spé | VPP | VPN | Taux de bonne classification | AUC |
| **HA + CXCL10** | **mROC** | **76** | **92** | **92** | **76** | **83** | **0,898** |
| HA | mROC | 78 | 79 | 82 | 75 | 78 | 0,845 |
| CXCL10 | mROC | 72 | 75 | 78 | 70 | 74 | 0,802 |
| **HA + CXCL10** | **CART** | **77** | **91** | **91** | **77** | **83** | **NA** |
| HA | CART | 78 | 79 | 82 | 75 | 78 | NA |
| CXCL10 | CART | 72 | 75 | 78 | 70 | 74 | NA |
| **HA + CXCL10** | **LR** | **76** | **86** | **86** | **75** | **80** | **0,878** |
| HA | LR | 68 | 84 | 84 | 68 | 75 | 0,845 |
| CXCL10 | LR | 72 | 73 | 76 | 68 | 72 | 0,802 |

**Tableau 21 :**

| | | Groupe de validation n = 103 | | | | | |
|---|---|---|---|---|---|---|---|
| Marqueurs | Méthode | Se | Spé | VPP | VPN | Taux de bonne classification | AUC |
| **HA + CXCL10** | **mROC** | **76** | **88** | **88** | **76** | **81** | **0,898** |
| HA | mROC | 76 | 73 | 78 | 73 | 75 | 0,831 |
| CXCL10 | mROC | 73 | 71 | 77 | 70 | 72 | 0,832 |
| **HA + CXCL10** | **CART** | **78** | **91** | **91** | **78** | **84** | **NA** |
| HA | CART | 76 | 73 | 78 | 73 | 75 | NA |
| CXCL10 | CART | 73 | 71 | 77 | 70 | 72 | NA |
| **HA + CXCL10** | **LR** | **78** | **86** | **87** | **77** | **82** | **0,888** |
| HA | LR | 68 | 83 | 83 | 68 | 75 | 0,831 |
| CXCL10 | LR | 77 | 72 | 77 | 72 | 75 | 0,832 |

| | | | | | | | |
|---|---|---|---|---|---|---|---|
| HA = concentration sérique en acide hyaluronique (HA), en ng/mL CXCL10 = concentration sérique en protéine CXCL10 (CXCL10), en pg/mL NA = non applicable | | | | | | | |

Il a ainsi pu être constaté que les performances de la combinaison HA+CXCL10 sont supérieures à celles de HA seul et à celles de CXCL10 seul. C'est plus particulièrement le cas des performances de spécificité, VPP, VPN, Taux de bonne classification et d'AUC.
Le fait que la combinaison de HA à CXCL10 atteigne de meilleures performances que le marqueur HA seul et que le marqueur CXCL10 seul indique que la combinaison HA+CXCL10 a un effet synergique allant au-delà de la simple juxtaposition des deux marqueurs (au sujet de la synergie, *cf*. également exemples 1 et 3 ci-dessus).
On constate également que les performances sont similaires quelle que soit la méthode de classification multivariée utilisée.
Les performances, plus particulièrement l'effet synergique, de la combinaison de HA et de CXCL10 ne sont pas liés à la nature de la méthode de classification multivariée utilisée.
Ceci prouve qu'une méthode de classification multivariée autre que mROC peut être utilisée.
Plus particulièrement, alternativement aux méthodes de classification multivariée qui sont basées sur une fonction linéaire telles que mROC, peuvent être utilisées :
- aussi bien les méthodes de classification multivariée qui sont basées sur une fonction qui n'est pas une fonction linéaire, par exemple les méthodes de classification multivariée qui sont basées sur une fonction affine, telles que la méthode LR,
- que les méthodes de classification qui ne sont pas basées sur une fonction (mathématique), telles que les méthodes basées sur un arbre de décision par exemple la méthode CART.
Les méthodes mROC, CART et LR ont également été appliquées sur la population totale des 310 patients de l'exemple 3. Des exemples de règles décisionnelles obtenues sur la population des 310 patients pour les méthodes CART et LR sont présentés dans le Tableau 22 ci-dessous (score Metavir de fibrose hépatique inférieur à F2, ou score Metavir de fibrose hépatique supérieur ou égal à F2). Les fonctions mROC utilisées sont celles de l'exemple 3 ci-dessus (*cf*. Tableau 7 ci-dessus).

**Tableau 22 :**

| Méthode | Règle décisionnelle | | Exemple de seuil(s) |
|---|---|---|---|
| | Fonction ou arbre de décision | Paramètres | |
| mROC | Z = a(CXCL10^{t}) + b(HA^{t}) | a = 1,999 | δ₁ = 15,170 |
| | | b = 2,852 | |
| | [fonction **Z₄**] | λ_{CXCL10} = -0,116 | |
| | | λ_{HA} = -0,288 | |
| mROC | Z = a(CXCL10^{t}) + b(HA^{t}) + c(IMC^{t}) + d(Âge^{t}) + e(CV^{t}) | a = 1,999 | δ₂ = 16,543 |
| | | b = 2,958 | |
| | | c = 0,616 | |
| | | d = -0,053 | |
| | | e = -0,00024 | |
| | | λ_{CXCL10} = -0,116 | |
| | [fonction **Z₅**] | λ_{HA} = -0,288 | |
| | | λ_{IMC} = -0,039 | |
| | | λ_{âge} = 0,433 | |
| | | λ_{CV} = 0,279 | |
| CART | *cf*. Figure 12 | NA | h = 47,29 |
| | | | i = 209,3 |
| | | | j = 503,4 |
| | | | [arbre **CART₄**] |
| LR | LOGIT = Intercept + k(CXCL10) + 1(HA) | Intercept = -3,164 | Logit = 0,5 |
| | | k = 0,005 | |
| | [fonction **LOGIT₄**] | l = 0,024 | |

| | | | |
|---|---|---|---|
| HA = concentration sérique en acide hyaluronique, en ng/mL CXCL10 = concentration sérique en protéine CXCL10, en pg/mL | | | |

Conformément à la méthode mROC, lorsque la valeur de la fonction Z₄ pour un patient donné était supérieure ou égale à 15,170 (combinaison HA+CXCL10), on a attribué à ce patient un score Metavir de fibrose hépatique supérieur ou égal à F2. Inversement, lorsque la valeur de la fonction Z₄ pour un patient donné était inférieure à 15,170, on a attribué à ce patient un score Metavir de fibrose hépatique inférieur à F2.
De même, lorsque la valeur de la fonction Z₅ pour un patient donné était supérieure ou égale à 16,543 (combinaison HA+CXCL10+IMC+Âge+CV), on a attribué à ce patient un score Metavir de fibrose hépatique supérieur ou égal à F2. Inversement, lorsque la valeur Z₅ pour un patient donné était inférieure à 16,543, on a attribué à ce patient un score Metavir de fibrose hépatique inférieur à F2.
Conformément à la méthode CART (*cf*. Figure 12), lorsque la concentration sérique en acide hyaluronique (HA) en ng/mL d'un patient était inférieure à la valeur du paramètre h (47,29), on lui a affecté :
- un score Metavir de fibrose hépatique inférieur à F2, lorsque sa concentration sérique en protéine CXCL10 en pg/mL était inférieure à la valeur du paramètre j (503,4), et
- un score Metavir de fibrose hépatique supérieur ou égal à F2, lorsque sa concentration sérique en protéine CXCL10 en pg/mL était supérieure ou égale à la valeur du paramètre j (503,4).
De même, conformément à la méthode CART (*cf*. Figure 12), lorsque la concentration sérique en acide hyaluronique (HA) en ng/mL d'un patient était supérieure à la valeur du paramètre h (47,29), on lui a affecté :
- un score Metavir de fibrose hépatique inférieur à F2, lorsque sa concentration sérique en protéine CXCL10 en pg/mL était inférieure à la valeur du paramètre i (209,3), et
- un score Metavir de fibrose hépatique supérieur ou égal à F2, lorsque sa concentration sérique en protéine CXCL10 en pg/mL était supérieure ou égale à la valeur du paramètre i (209,3).
Conformément à la méthode LR, lorsque la valeur de la fonction LOGIT d'un patient donné était supérieure ou égale à la valeur du seuil logit (0,5), on a affecté à ce patient un score Metavir de fibrose hépatique supérieur ou égal à F2. Inversement, lorsque la valeur de la fonction LOGIT d'un patient donné était inférieure à la valeur du seuil logit (0,5), on a affecté à ce patient un score Metavir de fibrose hépatique inférieur à F2.

Les résultats de sensibilité (Se), de spécificité (Spé), de valeur de prédiction négative (VPN), de valeur de prédiction positive (VPP), d'aire sous la courbe ROC (AUC) et de taux de bonne classification, qui ont été obtenus pour ces règles décisionnelles sur la population de 310 patients sont présentés dans le Tableau 23 ci-dessous.

**Tableau 23 :**

| | Population totale n = 310 | | | | | | |
|---|---|---|---|---|---|---|---|
| Combinaison | Méthode | Se | Spé | VPP | VPN | Taux de bonne classification | AUC |
| HA + CXCL10 | mROC | 78 | 90 | 90 | 77 | 83 | 0,898 |
| HA + CXCL10 + IMC + Âge + CV | mROC | 80 | 87 | 88 | 78 | 83 | 0,899 |
| HA + CXCL10 | CART | 82 | 89 | 90 | 80 | 85 | NA |
| HA + CXCL10 | LR | 77 | 87 | 87 | 76 | 81 | 0,882 |

| | | | | | | | |
|---|---|---|---|---|---|---|---|
| HA = concentration sérique en acide hyaluronique (HA), en ng/mL CXCL10 = concentration sérique en protéine CXCL10 (CXCL10), en pg/mL Âge = âge du patient à la date du prélèvement IMC = Indice de Masse Corporelle à la date du prélèvement (masse / taille²) CV = Charge Virale du patient à la date du prélèvement, en UI/mL NA = non applicable | | | | | | | |

Il a ainsi pu être constaté que les performances de la combinaison du biomarqueur HA au biomarqueur CXCL10 sont totalement indépendantes de la nature de la méthode de classification multivariée utilisée.

### EXEMPLE 8 : formulation générale

Les gammes de valeurs des paramètres et seuils mis en œuvre par les méthodes de classification peuvent être calculées, en utilisant la méthode de *Bootstrap* (*cf.* Efron 1979). Conformément à cette méthode, une population aléatoire de 310 patients a été tirée aléatoirement 1000 fois de suite.
Les résultats de ces calculs pour les méthodes mROC, CART et LR sont présentés dans le Tableau 24 ci-dessous.

**Tableau 24 :**

| Méthode classification multivariée Règle décisionnelle | | Paramètres | Exemple de seuil(s) | Performance |
|---|---|---|---|---|
| mROC **Z = a(CXCL10^{t}) + b(HA^{t})** | | 0,812 ≤ a ≤ 5,089 | 11,68 ≤ δ ≤ 23,71 | **AUC ≥ 0,865** |
| | | 2,033 ≤ b ≤ 4,462 | | [0,865 ≤ AUC ≤ 0,931] |
| | | -0,262 ≤ λ_{CXL10} ≤ | | **Taux BC ≥ 80%** |
| | | 0,030 | | [80% ≤ TauxBC ≤ 88%] |
| [fonction **Z₁₀]** | | -0,382 ≤ λ_{HA} ≤ -0,219 | | |
| mROC | | 0,748 ≤ a ≤ 5,357 | 11 ≤ δ ≤ 25,87 | **AUC ≥ 0,868** |
| | | 2,075 ≤ b ≤ 4,690 | | |
| **Z** = **a(CXCL10^{t})** + **b(HA^{t}) + c(IMC^{t}) + d(Âge^{t}) + e(CV^{t})** | | -0,848≤ c ≤ 3,697 | | |
| | | -0,746 ≤ d ≤ 0,147 | | [0,868 ≤ AUC ≤ 0,933] |
| | | -0,003 ≤ e ≤ 0,002 | | **Taux BC ≥ 80%** |
| [fonction **Z₁₁]** | | -0,262 ≤ λ_{CXCL10} ≤ 0,047 | | [80% ≤ TauxBC ≤ 88%] |
| | | -0,882 ≤ λ_{HA} ≤ -0,219 | | |
| | | -5,545 ≤ λ_{IMC} ≤ 0,485 | | |
| | | -0,116 ≤ λ_{âge} ≤ 0,828 | | |
| | | 0,236 ≤ λ_{CV} ≤ 0,305 | | |
| ***Méthode de classification multivariée non basée sur une fonction linéaire*** | ***CART*** (*Classification And Régression Tree*) | NA | 41,96 ≤ h ≤ 77,43 | **Taux BC ≥ 81%** |
| | | | 159,0 ≤ i ≤ 266,7 | [81% ≤ TauxBC ≤ 89%] |
| | *cf.* Figure 12 (marqueurs HA et CXCL10) | | 410,7 ≤ j ≤ 613,5 | |
| | | | [arbre **CART₂]** | |
| | **LR (régression logistique)** | -4,481 ≤ Intercept ≤ - 2,398 | Logit = 0,5 par exemple | **AUC ≥ 0,844** |
| | | | | [0,844 ≤ AUC ≤ 0,921] |
| | LOGIT = Intercept + k(CXCL10) + 1(HA) | 0,003 ≤ k ≤ 0,008 | | **Taux BC ≥ 77%** |
| | | 0,013 ≤ 1 ≤ 0,045 | | [77% ≤ TauxBC ≤ 86%] |
| | [fonction **LOGIT₂]** | | | |

| | | | | |
|---|---|---|---|---|
| TauxBC = taux de bonne classification ; NA = non applicable. | | | | |

Dans le Tableau 24, a, b, c, d, e, λ_{CXCL10}, λ_{HA}, λ_{IMC}, λ_{CV}, Intercept, k et 1 sont chacun différents de zéro.

L'ensemble des différentes fonctions mROC peut ainsi être formulé comme suit : $Z = a \left(CXCL{10}^{t}\right) + b \left({HA}^{t}\right) + c \left({IMC}^{t}\right) + d \left({Âge}^{t}\right) + e \left({CV}^{t}\right) + f \left({FS}^{t}\right)$ avec :
a et b étant chacun, indépendamment l'un de l'autre, un nombre réel positif allant de +0,1 à +6,0, plus particulièrement de +0,3 à +5,5,
c étant un nombre réel allant de -10,0 à +4,0,
d étant un nombre réel allant de -0,8 à +0,2,
e étant un nombre réel allant de -0,003 à +0,002,
f étant un nombre réel allant de +0,0 à +10,0,
λ_{CXCL10}, λ_{HA}, λ_{IMC}, λ_{Âge}, λ_{CV} et λ_{FS} étant chacun, indépendamment les uns des autres, un nombre réel allant de -6,0 à 1,2 mais différent de zéro.

L'ensemble des différentes fonctions mROC peut ainsi être formulée comme suit : $Z = a \left(CXCL{10}^{t}\right) + b \left({HA}^{t}\right) + c \left({IMC}^{t}\right) + d \left({Âge}^{t}\right) + e \left({CV}^{t}\right) + f \left({FS}^{t}\right)$ avec :
a et b étant chacun, indépendamment l'un de l'autre, un nombre réel positif allant de +0,1 à +6,0,
c, d, e, et f étant chacun, indépendamment les uns des autres, un nombre réel allant de -10,0 à +10,0,
λ_{CXCL10}, λ_{HA}, λ_{IMC}, λ_{Âge}, λ_{CV} et λ_{FS} étant chacun, indépendamment les uns des autres, un nombre réel allant de -6,0 à 1,2 mais différent de zéro.

L'ensemble des fonctions LOGIT peut ainsi être formulé comme suit : $LOGIT = Intercept + k \left(CXCL10\right) + l \left(AH\right)$ avec $- 5 \leq Intercept \leq - 1$ $0,001 \leq k \leq 0,010$ $0,010 \leq l \leq 0,050 .$

L'ensemble des différents arbres CART peut être formulé comme suit :
arbre décisionnel de la Figure 12, avec $40 \leq h \leq 80$ $150 \leq i \leq 300$ $400 \leq j \leq 620$ [arbre **CART₁].**

Pour chaque fonction mROC ou fonction LR ou arbre CART, les valeurs des paramètres peuvent être choisies conformément aux plages de valeurs indiquées.
Une fois la valeur de chacun des paramètres choisis, la fonction mROC ou la fonction LR ou l'arbre CART qui en résulte peut être testée sur une population de référence, par exemple sur la population de 310 patients décrite en exemple 3. Ainsi, on peut par exemple tester qu'avec les paramètres choisis, la fonction mROC ou la fonction LR ou l'arbre CART conduit bien à la(les) performance(s) attendue(s) ou souhaitée(s), notamment à la valeur d'AUC et/ou à la valeur de taux de bonne classification attendue(s) ou souhaitée(s).

Les performances peuvent être mesurées sur une population de patients VHC positifs, présentant un stade de fibrose hépatique couvrant au moins les stades F1 à F3 (score Metavir), telle que la population de 118 patients de l'exemple 1 ou la population de 310 patients de l'exemple 3.
Le seuil associé peut par exemple être le seuil maximisant l'indice de Youden (δ), dont la valeur est un nombre réel allant de -7 à 25.

### EXEMPLE 9 : robustesse de la combinaison HA+CXCL10

Pour illustrer la robustesse de la combinaison HA+CXCL10, la méthode de ré-échantillonnage connue sous le nom de *Bootstrap* (*cf.* Efron 1979) a été mise en œuvre sur la population de 310 patients de l'exemple 3 ci-dessus.

À partir de cette population de 310 patients, 1000 sous-populations de même taille ont été tirées aléatoirement (tirage avec remise, de sorte qu'un même patient peut être présent plusieurs fois au sein d'une même sous-population).

Les valeurs d'AUC et de taux de bonne classification ont été mesurées sur chacune de ces 1000 sous-populations:
- d'une part, lorsque la règle décisionnelle mROC appliquée a pour coefficients et seuil ceux initialement établis sur la population de 310 patients (« coefficients fixes » ou « coef fix »), et
- d'autre part, lorsque la règle décisionnelle mROC appliquée a pour coefficients et seuil ceux établis pour chacune des 1000 sous-populations («coefficients optimisés» ou « coef optim ») ;
*cf.* exemple 3 ci-dessus.
La valeur du taux de bonne classification est la valeur du pourcentage de patients bien classés.
La valeur d'AUC est la valeur de l'aire sous la courbe ROC.

La variabilité entre les AUC obtenues avec la règle mROC à « coefficients fixes » (« coef fix ») et les AUC obtenues avec la règle mROC à « coefficients optimisés » (« coef optim ») a été déterminée (Figure 15).
La Figure 15 présente l'histogramme des AUC pour la combinaison HA+CXCL10, en *Bootstrap* (B = 1 000) avec les coefficients fixes (« coef fixes ») [axe des abscisses : 0,84 ; 0,86 ; 0,88 ; 0,90 ; 0,92 ; 0,94 ; 0,96]. La Figure 17 démontre que sur 1000 populations différentes, les performances de la combinaison HA+CXCL10 sont robustes (valeurs d'AUC comprises entre 0,84 et 0,96, ce qui sont des performances élevées).
Il a par ailleurs été constaté que les performances en terme d'AUC sont similaires que l'on utilise la règle mROC à « coefficients fixes » (« coef fix ») [coefficients fixés à partir de la population initiale de 310 patients et appliqués à chacune des sous-populations *bootstrap*] ou bien que l'on utilise la règle mROC à « coefficients optimisés » (« coef optim ») [coefficients de la règle mROC optimisés pour chacune des 1000 sous-populations *bootstrap*]*.*

Les performances de la fonction mROC obtenue sur la population de 118 patients de l'exemple 1 (marqueurs HA+CXCL10 ; fonction Z₁ ; *cf.* Tableau 2 ci-dessus) ont été comparées à celles de la fonction mROC obtenue sur la population de 310 patients de l'exemple 3 (marqueurs HA+CXCL10 ; fonction Z₄ ; *cf.* Tableau 7 ci-dessus), lorsqu'elles sont toutes deux appliquées à la population de 118 patients.
Les résultats de cette comparaison sont illustrés en Figures 16A et 16B.
Figure 16A : en ordonnée, fonction Z1 de l'exemple 1 [Z = (0,3686) x CXCL10^{t} + (0,3064) x HA^{t}, avec λCXCL10 = -0,013 et λHA = 0,099] ; en abscisse, fonction Z4 de l'exemple 3 [Z = (1,999) x CXCL10^{t} + (2,852) x HA^{t}, avec λCXCL10 = -0,116 et λHA = -0,288].
Figure 16B : en ordonnée, sensibilité ; en abscisse, spécificité ; courbes de Z1 et Z4.
La transposition de la règle obtenue à partir de la population de 310 patients (fonction Z₄), sur la population de 118 patients, permet d'obtenir des AUC similaires à celles obtenues avec les coefficients optimisés sur cette population (fonction Z₁). On a ainsi pu constater qu'en dépit d'une grande différence de coefficients, les deux scores sont très corrélés et les courbes ROC sont proches. Les différences de sensibilité et spécificité s'expliquent essentiellement par le seuil qui lui aussi provient de la population de 310 patients. On pourrait parfaitement déterminer un seuil permettant d'obtenir des performances similaires.

### EXEMPLE 10 : combinaison de HA à CXCL10 en multiplex

Des microplaques à puits pour criblage à haut débit (*High Throughput Screening microplates*) sont utilisées. Il s'agit de microplaques en polystyrène à 96 puits LUMITRAC™ 600 (fond F, « *high binding* », cheminée noire, volume maximal théorique d'un puits = 392 µL) disponibles auprès de GREINER BIO-ONE GmbH ; Maybachstrasse 2 ; DE 72636 Frickenhausen ; Allemagne (Référence catalogue 655 097).
À l'aide d'un robot spotteur, on dépose des rangées de spots sur le fond de chaque puits (spot = goutte d'environ 50nL). Chaque spot contient un ligand de capture, à savoir soit un ligand de capture anti-CXCL10 humaine, soit un ligand de capture anti-HA humain. Le ligand de capture se fixe sur la surface du puits.
Le ligand de capture anti-CXCL10 humaine est un anticorps monoclonal de souris anti-CXCL10 humaine, commercialisé par R&D SYSTEMS, Inc. (614 McKinley Place NE ; Minneapolis, MN 55413 ; U.S.A.), sous la référence catalogue MAB266 (clone 33036, classe IgG1).

Le ligand de capture anti-HA humain est une protéine recombinante humaine aggrécane G1-IGD-G2 commercialisée par la société R&D SYSTEMS, Inc. (614 McKinley Place NE ; Minneapolis, MN 55413 ; U.S.A.), sous la référence catalogue 1220-PG-025.
Les échantillons sont des échantillons de sérum ou de plasma de patients infectés par le VHC. Ils proviennent de l'Hôpital Haut-Levêque (1, avenue Magellan; 33600 Pessac ; France) ou de l'Hôpital Beaujon (100, boulevard du Général Leclerc ; 92110 Clichy ; France). Il s'agit par exemple de ou des patients de l'exemple 3 ou de l'exemple 1.
Une gamme étalon de CXCL10 a été préparée en diluant une protéine CXCL10 recombinante (PEPROTECH ; Princeton Business Park ; 5 Crescent Avenue ; P.O. Box 275 ; Rocky Hill, NJ 08553 ; U.S.A. ; Référence catalogue 300-12) dans une solution tampon PBS (*Phosphate Buffer Saline*) à pH 7,4 contenant de l'albumine de sérum bovin à 5%, du glycérol à 20% et un conservateur à 0,1% (PROCLIN® 300 ; produit SUPELCO commercialisé par SIGMA-ALDRICH CHIMIE ; 38297 Saint-Quentin-Fallavier CEDEX ; France).
Une gamme étalon de HA a été préparée en diluant un acide hyaluronique humain recombinant (R&D SYSTEMS ; Minneapolis ; U.S.A.) dans la solution tampon PBS à pH 7,4 qui contient de l'albumine de sérum bovin à 5%, du glycérol à 20% et le conservateur PROCLIN® 300 à 0,1%.

Dans chaque puits de la microplaque, sont distribués successivement (sur chaque spot) :
40 µL de la solution tampon PBS à pH 7,4 qui contient de l'albumine de sérum bovin à 5%, du glycérol à 20% et le conservateur PROCLIN® 300 à 0,1%, et
40 µL d'échantillon à analyser, ou d'une solution de la gamme étalon CXCL10, ou d'une solution de la gamme étalon HA.
Le mélange est mis en incubation pendant 40 min à 37°C sous agitation.
Trois lavages successifs sont réalisés, chacun avec au moins 400 µL d'une solution de lavage (solution tampon TRIS 10 mM à pH 7,4 contenant NaCl 218 mM, TWEEN® 20 (SIGMA-ALDRICH CHIMIE; 38297 Saint-Quentin-Fallavier CEDEX; France; Référence catalogue 2287) à 0,1%, et le conservateur PROCLIN® 300 à 0,002%.

Dans chaque puits réactionnels, sont ensuite distribués (sur chaque spot) 50 µL d'une solution tampon PBS 10 mM à pH 7,4 contenant du NaCl à 150 mM, du glycérol à 10%, de l'IgG de souris (MERIDIAN LIFE SCIENCE, Inc. ; 5171 Wilfong Road ; Memphis TN 38134 ; U.S.A. ; Référence catalogue A66185M) à 50 g/L, le conservateur PROCLIN® 300 à 0,1%, ainsi qu'un ligand de détection de la CXCL10 humaine à une concentration de 0,2 µg/mL et un ligand de l'HA humain à une concentration de 0,2 à 0,5 µg/mL.
Le ligand pour la détection de CXCL10 est un anticorps polyclonal de chèvre anti-CXCL10 humaine couplé à la biotine, disponible auprès de R&D SYSTEMS, Inc. (614 McKinley Place NE ; Minneapolis, MN 55413 ; U.S.A. ; Référence catalogue BAF266). Le ligand pour la détection de l'HA humain est la protéine recombinante aggrécane G1-IGD-G2 couplée à la biotine, disponible auprès de R&D SYSTEMS, Inc. (614 McKinley Place NE ; Minneapolis, MN 55413 ; U.S.A.), sous la référence catalogue 1220-PG-025. Le mélange est mis en incubation pendant 15 min à 37°C sous agitation.
Trois lavages successifs sont réalisés, chacun avec au moins 400 µL d'une solution de lavage (solution tampon TRIS 10 mM à pH 7,4 contenant NaCl 218 mM, TWEEN® 20 (SIGMA-ALDRICH CHIMIE; 38297 Saint-Quentin-Fallavier CEDEX; France; Référence catalogue 2287) à 0,1%, et le conservateur PROCLIN® 300 à 0,002%.

Dans chaque puits réactionnel, est ensuite distribué le rapporteur, à savoir (sur chaque spot) 50 µL d'une solution tampon citrate 50 mM, pH 6,7, contenant NaCl 150 mM, EDTA 5,6 mM, TRITON® 2%, sérum de mouton 10%, IgG de souris 500 µg/mL, le conservateur PROCLIN® 300 0,5%, lait de vache (100% écrémé) 15%, glycérol 10%, NaN₃ 0,095% et contenant en outre de la streptavidine couplée à la péroxydase de raifort (streptavidin-POD, disponible auprès de ROCHE DIAGNOSTICS GmbH ; Roche Applied Science ; 68298 Mannheim ; Allemagne ; Référence catalogue 11089153001) (*cf.* Nakane et Kawaoi 1974) à 3 µg/mL.
Le mélange est mis en incubation pendant 15 min à 37°C sous agitation.

Dans chaque puits réactionnel, sont ensuite distribués le substrat pour la révélation des réactions de chimioluminescence, à savoir (sur chaque spot) 25µL de solution *enhancer*/luminol XLSE024L et 25 µM de solution de péroxyde XLSE024P [25 µL de la solution (A) et 25 µL de la solution (B) de l'ELISTAR ETA C Ultra ELISA, commercialisé par CYANAGEN ; Via degli Stradelli Guelfi 40/C ; 40138 Bologna ; Italie ; Référence catalogue XLSE024,0020].
Le mélange est mis en incubation pendant 1 min à 37°C sous agitation.

L'acquisition du signal de luminescence est réalisée pendant 180 secondes.

Les résultats des lectures sont directement traités par un système d'analyse d'image et enregistrés en Unités Relatives de Luminescence ou Relative light Unit (RLU).
Pour l'interprétation des résultats, pour chaque échantillon la concentration des biomarqueurs CXCL10 et HA est recalculée à l'aide de la gamme étalon de CXCL10 recombinant pour CXCL10 en pg/mL et de la gamme étalon de HA recombinant pour HA en ng/mL.

Alternativement, le ligand de détection de la CXCL10 humaine et le ligand de l'HA humain peuvent être placés non pas chacun dans des spots distincts, mais au contraire tous deux dans un même spot. Dans ce cas, la détection sera faite de sorte à différencier les CXCL10 liées des HA liés, par exemple non pas en chimioluminescence, mais en fluorescence (à l'aide de deux fluorophores différents, l'un porté par le ligand de détection de CXCL10, l'autre porté par le ligand de détection de HA).

### RÉFÉRENCES BIBLIOGRAPHIQUES

Adams et al. 2005. Clinical Chemistry 51(10): 1867-1873.
Anastasiadis et al. 2005 ; New globally convergent training scheme based on the resilient propagation algorithm. Neurocomputing 64: 253-270.
Berkson 1944. Application of the Logistic Function to Bio-Essay. Journal of the American Statistical Association, volume 39, pages 357-365.
Berkson 1951. Why I Prefer Logits to Probits. Biometrics, vol. 7, 1951, p. 327-329. Booth et al. 2002. Biochemistry 41(33) : 10418-10425.
Boursier et al. 2012. Hepatology 55(1): 58-67.
Box et Cox 1964. An analysis of transformations. Journal of the Royal Statistical Society, Series B 26: 211-243.
Breiman Friedman, Stone et Olshen 1984. Classification and regression trees. Monterey, CA: Wadsworth & Brooks/Cole Advanced Books & Software. ISBN 978-0-412-04841-8.
Breiman 2001 ; Random Forests. Machine Learning 45 : 5-32.
Castera et al. 2005. Gastroenterology 128: 343-350.
Castera et al. 2010. Journal of Hepatology 52: 191-198.
Castera et al. 2014. Comparison of transient elastography (Fibroscan), Fibrotest, APRI and two algorithms combining these non-invasive tests for liver fibrosis staging in HIV/HCV coinfected patients: ANRS CO13 HEPAVIH and FIBROSTIC collaboration. HIV Medicine 15(1): 30-39.
Castera 2012. Gastroenterology 142: 1293-1302.
Chambers 2008 ; Software for data analysis : programming with R. Springer, New York, ISBN 978-0-387-75935-7.
Efron 1979. Bootstrap Methods: Another Look at the Jacknife. Annals of Statistics, volume n°7, n°1, pages 1-26.
Falissard 2005 ; Comprendre et utiliser les statistiques dans les sciences de la vie, Masson*.*
Forns et al. 2002. Hepatology 36: 986-992.
Goodman 2007. Journal of Hepatology 47: 598-607.
Hastie, Tibishirani et Friedman, 2009 ; "The Elements of Statistical Learning: Data Mining, Inference and Prediction", 2nd Edition, Springer.
Imbert-Bismut et al. 2001. The Lancet 357: 1069-1075.
Intrator et Intrator 1993 ; Using Neural Nets for Interprétation of Nonlinear Models. Proceedings of the Statistical Computing Section, San Francisco: American Statistical Society (eds), pages 244-249.
Kelleher et al. 2005. Journal of Hepatology 43: 78-84.
Kramar et al. 1999 ; Critères ROC généralisés pour l'évaluation de plusieurs marqueurs tumoraux. Revue d'Epidémiologie et Santé Publique 47: 376-383.
Kramar et al. 2001. mROC: a computer program for combining tumour markers in predicting disease states. Computer methods and programs in biomedicine 66: 199-207.
Liaw et Wiener 2002 ; Classification and regression by Random Forest. R. News 2.3: 18-22.
Nakane et Kawaoi 1974. J. Histochem. Cytochem. 22(12) : 1084-1091.
Reiser et Faraggi 1997 ; Confidence intervals for the generalized ROC criterion. Biometrics 53: 644-652.
Riedmiller 1994 ; Rprop - Description and Implementation Details. Technical Report. University of Karlsruhe.
Riedmiller et Braun 1993 ; A direct adaptive method for faster backpropagation learning: the RPROP algorithm. Proceedings of the IEEE International Conference on Neural Networks (ICNN), San Francisco, pages 586-591.
Shapiro 1999 ; The interpretation of diagnostic tests. Statistical Methods in Medical Research, 8: 113-134.
Su et Liu 1993 ; Linear combinations of multiple diagnostic markers. Journal of the American Statistical Association 88: 1350-1355.
Swaminathan et al. 2003. Structure 11(5) : 521-532.
Theodoridis et Koutroumbos 2009 ; Pattern Recognition. Academic Press, Elsevier. Wai et al. 2003. Hepatology 38: 518-526.
Zeremski et al. 2009. The Journal of Infectious Diseases 200: 1774-1780.
US 7 141 380 B2
US 2007/0225919 A1
US 6 986 995 B2
US 8 489 335 B2

## Revendications

1. Une méthode in vitro pour déterminer si le stade de fibrose hépatique d'un sujet infecté par un ou plusieurs virus de l'hépatite a ou non dépassé celui des stades de fibrose hépatique qui, selon le système de scores de fibrose Metavir, est de score F1, ladite méthode comprenant les étapes suivantes :
i) sélectionner différents marqueurs biologiques, lesdits différents marqueurs biologiques sélectionnés consistant en :
a) l'acide hyaluronique ainsi que la protéine CXCL10, et
b) zéro, un, deux, trois ou quatre marqueur(s) additionnel(s) choisi(s) parmi la liste de marqueurs constituée par l'âge, l'Indice de Masse Corporelle, la charge virale et la dureté du foie,
ii) quantifier les différents marqueurs biologiques sélectionnés à l'étape i)
en mesurant in vitro la concentration d'acide hyaluronique et la concentration de protéine CXCL10 dans un échantillon de fluide biologique préalablement obtenu à partir dudit sujet, et
lorsqu'un, deux, trois ou quatre marqueurs additionnel(s) est(sont) choisi(s) parmi ladite liste de l'étape i)b) ci-dessus, et lorsque ce (ou ces) marqueur(s) additionnel(s) est(sont) ou comprennent un ou plusieurs marqueur(s) parmi l'âge, l'Indice de Masse Corporelle et la dureté du foie : en collectant la valeur de quantification de ce ou chacun de ces marqueur(s) additionnel(s) qui a été préalablement déterminée pour ou sur ledit sujet,
lorsqu'un, deux, trois ou quatre marqueurs additionnel(s) est(sont) choisi(s) parmi ladite liste de l'étape i)b) ci-dessus, et lorsque ce (ou ces) marqueur(s) additionnel(s) est(sont) ou comprennent la charge virale : en mesurant cette charge virale in vitro dans un échantillon de fluide biologique préalablement obtenu à partir dudit sujet, ou en collectant la valeur de cette charge virale qui a été préalablement déterminée pour ledit sujet, et
iii) comparer les valeurs de quantification obtenues à l'étape ii) à leurs valeurs, ou à la distribution de leurs valeurs, dans des cohortes de référence préétablies selon le stade de fibrose hépatique, pour classer ledit sujet dans celle de ces cohortes de référence vis-à-vis de laquelle il a la plus forte probabilité d'appartenance, lesdites cohortes de référence étant :
une première cohorte de référence dans laquelle le stade de fibrose hépatique des individus ne dépasse pas celui des stades de fibrose hépatique qui, selon le système de scores de fibrose Metavir, est de score F1, et
une deuxième cohorte de référence dans laquelle le stade de fibrose hépatique des individus dépasse celui des stades de fibrose hépatique qui, selon le système de scores de fibrose Metavir, est de score F1,
le classement dans ladite première cohorte indiquant que le stade de fibrose hépatique dudit sujet n'a pas dépassé celui des stades de fibrose hépatique qui, selon le système de scores de fibrose Metavir, est de score F1,
le classement dans ladite deuxième cohorte indiquant que le stade de fibrose hépatique dudit sujet a dépassé celui des stades de fibrose hépatique qui, selon le système de scores de fibrose Metavir, est de score F1,
dans laquelle ladite comparaison de l'étape iii) est faite :
par apprentissage automatique, ou par régression logistique, ou par mROC, et
dans laquelle ladite comparaison de l'étape iii) est faite :
par apprentissage automatique en suivant l'arbre décisionnel de la Figure 12 avec 40 ≤ h ≤ 80, 150 ≤ i ≤ 300, et 400 ≤ j ≤ 620, ou
par régression logistique à l'aide de la fonction LOGIT1, ladite fonction LOGIT1 étant : $LOGIT = Intercept + k \left(CXCL10\right) + l \left(HA\right) ,$ avec
-5 ≤ Intercept ≤ -1, 0,001 ≤ k ≤ 0,010, et 0,010 ≤ l ≤ 0,050, ou
par mROC à l'aide de la fonction Z13, ladite fonction Z13 étant $Z = a \left(CXCL10t\right) + b \left(HAt\right) + c \left(IMCt\right) + d \left(Âget\right) + e \left(CVt\right) + f \left(FSt\right) ,$ avec
a et b étant chacun, indépendamment l'un de l'autre, un nombre réel positif allant de +0,1 à +6,0 mais différent de zéro,
c, d, e, et f étant chacun, indépendamment les uns des autres, un nombre réel allant de -10,0 à +10,0,
l'exposant t indiqué indiquant que la valeur à appliquer dans la fonction est la transformée Box-Cox de la valeur mesurée pour le marqueur considéré (BMQ),
afin de normaliser cette valeur mesurée selon la formule suivante : BMQt = (BMQλ, - 1) / λ, et
la valeur de λ pour chacun des marqueurs CXCL10 (λCXCL10,) HA (λHA), IMC (λIMC), Âge (λÂge), CV (λCV) et FS (λFS) étant chacun, indépendamment les uns des autres, un nombre réel allant de -6,0 à 1,2 mais différent de zéro, et
dans laquelle la comparaison de l'étape iii) est faite en combinant les valeurs de quantification obtenues pour ledit sujet dans un modèle de classification préalablement construit comme suit :
a) pour une population d'individus qui sont de la même espèce que ledit sujet, et qui sont infectés par le ou les mêmes virus de l'hépatite que ledit sujet, déterminer le stade de fibrose hépatique de chacun desdits individus de la population, et les classer en sous-populations selon leur stade de fibrose hépatique, constituant ainsi des cohortes de référence établies selon leur stade de fibrose hépatique, lesdites cohortes de référence comprenant ou étant :
une première cohorte de référence dans laquelle le stade de fibrose hépatique des individus ne dépasse pas celui des stades de fibrose hépatique qui, selon le système de scores de fibrose Metavir, est de score F1, et
une deuxième cohorte de référence dans laquelle le stade de fibrose hépatique des individus dépasse celui des stades de fibrose hépatique qui, selon le système de scores de fibrose Metavir, est de score F1 ;
β) pour chacun desdits individus, quantifier les différents marqueurs biologiques sélectionnés à l'étape i), et
γ) faire une comparaison inter-cohorte des valeurs de quantification obtenues à l'étape β), ou de la distribution de ces valeurs, pour construire un modèle de classification, qui, à partir de valeurs de quantification desdits marqueurs biologiques sélectionnés, induit un classement dans l'une desdites cohortes de référence.

2. La méthode de la revendication 1, dans laquelle lesdits différents marqueurs biologiques sélectionnés à l'étape i) consistent en :
a) l'acide hyaluronique ainsi que la protéine CXCL10, et
b) zéro, un, deux ou trois marqueur(s) additionnel(s) choisi(s) parmi la liste de marqueurs constituée par l'âge, l'Indice de Masse Corporelle, la charge virale et la dureté du foie.

3. La méthode de la revendication 1 ou 2, dans laquelle lesdits différents marqueurs biologiques sélectionnés à l'étape i) consistent en :
- l'acide hyaluronique et la protéine CXCL10, ou
- l'acide hyaluronique, la protéine CXCL10, l'âge et l'Indice de Masse Corporelle, ou
- l'acide hyaluronique, la protéine CXCL10, l'âge, l'Indice de Masse Corporelle et la charge virale, ou
- l'acide hyaluronique, la protéine CXCL10 et la dureté du foie.

4. La méthode selon l'une quelconque des revendications 1 à 3, dans laquelle ladite comparaison de l'étape iii) de la revendication 1 est faite en classant ledit sujet dans celle desdites cohortes de référence vis-à-vis de laquelle il a la plus forte probabilité d'appartenance avec une sensibilité d'au moins 75% et/ou avec une Valeur de Prédiction Négative d'au moins 75%.

5. La méthode selon l'une quelconque des revendications 1 à 4, dans laquelle ladite comparaison de l'étape iii) de la revendication 1 est faite en classant ledit sujet dans celle desdites cohortes de référence vis-à-vis de laquelle il a la plus forte probabilité d'appartenance avec au moins l'une des deux performances 1/ et 2/ ci-dessous :
1/ une spécificité d'au moins 85% et/ou une Valeur de Prédiction Positive d'au moins 85%,
2/ un taux de bonne classification d'au moins 80% et/ou une aire sous la courbe ROC d'au moins 0,800.

6. La méthode selon l'une quelconque des revendications 1 à 5, dans laquelle, à l'étape ii) de la revendication 1, la mesure de la concentration d'acide hyaluronique et la mesure de la concentration de protéine CXCL10 sont faites en multiplex.

7. L'utilisation d'un article manufacturé pour effectuer la méthode selon l'une quelconque des revendications 1 à 6, dans laquelle ledit article manufacturé est adapté à la détection en multiplex de molécules contenues sous forme acellulaire dans un échantillon de fluide biologique, et qui comprend un support solide sur lequel sont fixés des ligands desdites molécules, dans lequel les ligands desdites molécules sont constitués par :
- une première protéine, qui est une protéine qui se lie de manière spécifique à HA et qui est un anticorps ou la protéine humaine recombinante aggrécane, et
- une deuxième protéine, qui est une protéine qui se lie de manière spécifique à la protéine CXCL10 et qui est un anticorps.

8. L'utilisation de la revendication 7, dans laquelle ledit support solide est une plaque ou microplaque à puits, une puce en silicium, un capillaire en verre, une lame de verre, des billes magnétiques, une membrane.

9. L'utilisation d'une composition pour effectuer la méthode selon l'une quelconque des revendications 1 à 6, dans laquelle ladite composition est adaptée à la détection en multiplex de molécules contenues sous forme acellulaire dans un échantillon de fluide biologique, et comprend en mélange des ligands desdites molécules, dans laquelle les ligands desdites molécules sont constitués par :
- une première protéine, qui est une protéine qui se lie de manière spécifique à HA, qui est un anticorps ou la protéine humaine recombinante aggrécane, et qui porte un premier marqueur de détection, et
- une deuxième protéine, qui est une protéine qui se lie de manière spécifique à la protéine CXCL10, qui est un anticorps et qui porte un deuxième marqueur de détection,
dans laquelle ledit premier marqueur de détection est différent dudit deuxième marqueur de détection.

10. L'utilisation d'un kit pour effectuer la méthode selon l'une quelconque des revendications 1 à 6, dans laquelle ledit kit comprend des acides nucléiques qui se lient de manière spécifique à un ou des virus de l'hépatite, et qui comprend en outre des ligands qui se lient à des molécules contenues sous forme acellulaire dans un fluide biologique, lesdits ligands étant contenus dans le kit en préparation combinée pour une utilisation simultanée, lesdits ligands étant constitués par :
- une première protéine qui se lie de manière spécifique à HA, qui est un anticorps ou la protéine humaine recombinante aggrécane, et qui porte un premier marqueur de détection, et
- une deuxième protéine qui se lie de manière spécifique à CXCL10, qui est un anticorps et qui porte un deuxième marqueur de détection,
dans lequel ledit deuxième marqueur de détection est différent dudit premier marqueur de détection.

11. L'utilisation de la revendication 10, dans laquelle ledit kit comprend en outre l'article manufacturé de la revendication 7 ou 8.

12. L'utilisation d'un produit programme d'ordinateur pour effectuer la méthode selon l'une quelconque des revendications 1 à 6, dans laquelle ledit produit programme d'ordinatuer est destiné à être stocké dans une mémoire d'une unité de traitement, ou sur un support mémoire amovible destiné à coopérer avec un lecteur de ladite unité de traitement, **caractérisé en ce qu'**il comprend des instructions pour la mise en œuvre d'une méthode selon l'une quelconque des revendications 1 à 6.

## Patentansprüche

1. In-vitro-Verfahren zur Bestimmung, ob das Leberfibrosestadium eines durch ein oder mehrere Hepatitisviren infizierten Patienten das der Leberfibrosestadien, die nach dem Metavir-Fibrose-Score-System dem Score F1 entsprechen, überschritten hat oder nicht, wobei das Verfahren die folgenden Schritte umfasst:
i) Auswählen verschiedener biologischer Marker, wobei die verschiedenen ausgewählten biologischen Marker aus
a) Hyaluronsäure sowie dem Protein CXCL10 und
b) null, einem, zwei, drei oder vier zusätzlichen Markern, die aus der Liste von Markern, welche aus dem Alter, dem Body Mass Index, der Viruslast und der Härte der Leber besteht, ausgewählt sind,
bestehen;
ii) Quantifizieren der verschiedenen, in Schritt i) ausgewählten biologischen Marker durch in-vitro-Messung der Konzentration von Hyaluronsäure und der Konzentration des Proteins CXCL10 in einer Probe biologischer Flüssigkeit, die vorher von dem Patienten erhalten wurde; und
wenn ein, zwei, drei oder vier zusätzliche Marker aus der obigen Liste von Schritt i)b) ausgewählt sind und wenn dieser oder diese zusätzlichen Marker einen oder mehrere Marker aus dem Alter, dem Body Mass Index und der Härte der Leber sind oder umfassen: indem man den Quantifizierungswert dieses oder jedes dieser zusätzlichen Marker, der vorher für oder an dem Patienten bestimmt wurde, erfasst;
wenn ein, zwei, drei oder vier zusätzliche Marker aus der obigen Liste von Schritt i)b) ausgewählt sind und wenn dieser oder diese zusätzlichen Marker die Viruslast sind oder umfassen: indem man diese Viruslast in vitro in einer Probe biologischer Flüssigkeit, die vorher von dem Patienten erhalten wurde, misst oder indem man den Wert dieser Viruslast, der vorher für den Patienten bestimmt wurde, erfasst; und
iii) Vergleichen der in Schritt ii) erhaltenen Quantifizierungswerte mit ihren Werten oder der Verteilung ihrer Werte in gemäß dem Stadium der Leberfibrose vorher bestimmten Referenzkohorten, um den Patienten in diejenige dieser Referenzkohorten zu klassifizieren, gegenüber der er die höchste Wahrscheinlichkeit der Zugehörigkeit hat, wobei die Referenzkohorten sind:
eine erste Referenzkohorte, in der das Leberfibrosestadium der Individuen das der Leberfibrosestadien, die nach dem Metavir-Fibrose-Score-System dem Score F1 entsprechen, nicht überschreiten; und
eine zweite Referenzkohorte, in der das Leberfibrosestadium der Individuen das der Leberfibrosestadien, die nach dem Metavir-Fibrose-Score-System dem Score F1 entsprechen, überschreiten;
wobei die Klassifizierung in der ersten Kohorte anzeigt, dass das Leberfibrosestadium des Patienten das der Leberfibrosestadien, die nach dem Metavir-Fibrose-Score-System dem Score F1 entsprechen, nicht überschritten hat;
wobei die Klassifizierung in der zweiten Kohorte anzeigt, dass das Leberfibrosestadium des Patienten das der Leberfibrosestadien, die nach dem Metavir-Fibrose-Score-System dem Score F1 entsprechen, überschritten hat;
wobei der Vergleich von Schritt iii) durchgeführt wird:
durch automatisches Lernen oder durch logistische Regression oder durch mROC und wobei der Vergleich von Schritt iii) durchgeführt wird:
durch automatisches Lernen unter Folgen des Entscheidungsbaums der Figur 12 mit 40 ≤ h ≤ 80, 150 ≤ i ≤ 300 und 400 ≤ j ≤ 620 oder
durch logistische Regression mit Hilfe der Funktion LOGIT1, wobei die Funktion LOGIT1 lautet: $LOGIT = Ordinatenabscnitt + k \left(CXCL10\right) + l \left(HA\right)$ mit $- 5 \leq Ordinatenabschnitt \leq - 1 ,$ $0,001 \leq k \leq 0,010 ,$ und $0,010 \leq l \leq 0,050 ;$ oder
durch mROC mit Hilfe der Funktion Z13, wobei die Funktion Z13 lautet: $Z = a \left(CXCL10t\right) + b \left(HAt\right) + c \left(IMCt\right) + d \left(Altert\right) + e \left(CVt\right) + f \left(FSt\right) ,$ wobei
a und b jeweils unabhängig voneinander eine positive reelle Zahl von +0,1 bis +6,0 sind, aber von null verschieden,
wobei c, d, e und f jeweils unabhängig voneinander eine reelle Zahl von -10,0 bis +10,0 sind;
wobei der angegebene Exponent t angibt, dass der in der Funktion anzuwendende Wert die Box-Cox-Transformierte des für den betreffenden Marker gemessenen Werts (BMQ) ist, um diesen gemessenen Wert gemäß der folgenden Formel zu normieren: BMQt = (BMQλ, - 1)/λ, und
der Wert von λ für jeden der Marker CXCL10 (λCXCL10), HA (λHA), IMC (λIMC), Alter (λAlter), CV (λCV) und FS (λFS) jeweils unabhängig voneinander eine reelle Zahl von -6,0 bis 1,2 sind, aber von null verschieden, und
wobei der Vergleich von Schritt iii) dadurch erfolgt, dass man die für den Patienten erhaltenen Quantifizierungswerte in einem vorher konstruierten Klassifikationsmodell wie folgt kombiniert:
a) für eine Population von Individuen, die derselben Art wie der Patient angehören und mit dem- oder denselben Hepatitisviren wie der Patient infiziert sind: Bestimmen des Leberfibrosestadiums jedes dieser Individuen der Population und Klassifizieren derselben in Unterpopulationen gemäß ihrem Leberfibrosestadium, wodurch gemäß ihrem Leberfibrosestadium festgelegte Referenzkohorten gebildet werden, wobei die Referenzkohorten umfassen oder sind:
eine erste Referenzkohorte, in der das Leberfibrosestadium der Individuen das der Leberfibrosestadien, die nach dem Metavir-Fibrose-Score-System dem Score F1 entsprechen, nicht überschreiten; und
eine zweite Referenzkohorte, in der das Leberfibrosestadium der Individuen das der Leberfibrosestadien, die nach dem Metavir-Fibrose-Score-System dem Score F1 entsprechen, überschreiten;
β) für jedes der Individuen: Quantifizieren der verschiedenen biologischen Marker, die in Schritt i) ausgewählt wurden; und
γ) Durchführen eines Inter-Kohorten-Vergleichs der in Schritt β) erhaltenen Quantifizierungswerte oder der Verteilung dieser Werte, um ein Klassifizierungsmodell zu konstruieren, das ausgehend von Quantifizierungswerten der ausgewählten biologischen Marker eine Klassifizierung in einer der Referenzkohorten induziert.

2. Verfahren gemäß Anspruch 1, wobei die verschiedenen, in Schritt i) ausgewählten biologischen Marker aus
a) Hyaluronsäure sowie dem Protein CXCL10 und
b) null, einem, zwei oder drei zusätzlichen Markern, die aus der Liste von Markern, welche aus dem Alter, dem Body Mass Index, der Viruslast und der Härte der Leber besteht, ausgewählt sind,
bestehen.

3. Verfahren gemäß Anspruch 1 oder 2, wobei die verschiedenen, in Schritt i) ausgewählten biologischen Marker aus
- Hyaluronsäure und dem Protein CXCL10 oder
- Hyaluronsäure, dem Protein CXCL10, dem Alter und dem Body Mass Index oder
- Hyaluronsäure, dem Protein CXCL10, dem Alter, dem Body Mass Index und der Viruslast oder
- Hyaluronsäure, dem Protein CXCL10 und der Härte der Leber
bestehen.

4. Verfahren gemäß einem der Ansprüche 1 bis 3, wobei der Vergleich von Schritt iii) von Anspruch 1 dadurch erfolgt, dass man den Patienten in diejenige der Referenzkohorten klassifiziert, gegenüber der er die höchste Wahrscheinlichkeit der Zugehörigkeit mit einer Empfindlichkeit von wenigstens 75% und/oder mit einem negativen Vorhersagewert von wenigstens 75% hat.

5. Verfahren gemäß einem der Ansprüche 1 bis 4, wobei der Vergleich von Schritt iii) von Anspruch 1 dadurch erfolgt, dass man den Patienten in diejenige der Referenzkohorten klassifiziert, gegenüber der er die höchste Wahrscheinlichkeit der Zugehörigkeit mit wenigstens einer der folgenden zwei Leistungsfähigkeiten 1/ und 2/ hat:
1/ eine Spezifität von wenigstens 85% und/oder einen positiven Vorhersagewert von wenigstens 85%;
2/ einen Grad der guten Klassifikation von wenigstens 80% und/oder eine Fläche unter der ROC-Kurve von wenigstens 0,800.

6. Verfahren gemäß einem der Ansprüche 1 bis 5, wobei in Schritt ii) von Anspruch 1 die Messung der Konzentration von Hyaluronsäure und die Messung der Konzentration des Proteins CXCL10 in einem Multiplexverfahren erfolgen.

7. Verwendung eines Fertigungsprodukts zur Durchführung des Verfahrens gemäß einem der Ansprüche 1 bis 6, wobei das Fertigungsprodukt für den Multiplex-Nachweis von Molekülen, die in azellulärer Form in einer Probe biologischer Flüssigkeit enthalten sind, geeignet ist und einen festen Träger umfasst, auf dem Liganden der Moleküle fixiert sind, wobei die Liganden der Moleküle aus
- einem ersten Protein, das ein Protein ist, das spezifisch an HA bindet und ein Antikörper ist, oder dem rekombinanten humanen Protein Aggrecan; und
- einem zweiten Protein, das ein Protein ist, das spezifisch an das Protein CXCL10 bindet und ein Antikörper ist,
bestehen.

8. Verwendung gemäß Anspruch 7, wobei es sich bei dem festen Träger um eine Well-Platte oder -Mikroplatte, einen Siliciumchip, eine Glaskapillare, einen Objektträger, magnetische Kügelchen oder eine Membran handelt.

9. Verwendung einer Zusammensetzung zur Durchführung des Verfahrens gemäß einem der Ansprüche 1 bis 6, wobei die Zusammensetzung für den Multiplex-Nachweis von Molekülen, die in azellulärer Form in einer Probe biologischer Flüssigkeit enthalten sind, geeignet ist und ein Gemisch der Liganden der Moleküle umfasst, wobei die Liganden der Moleküle aus
- einem ersten Protein, das ein Protein ist, das spezifisch an HA bindet und ein Antikörper ist, oder dem rekombinanten humanen Protein Aggrecan, der bzw. das einen ersten Nachweismarker trägt; und
- einem zweiten Protein, das ein Protein ist, das spezifisch an das Protein CXCL10 bindet, ein Antikörper ist und einen zweiten Nachweismarker trägt;
wobei der erste Nachweismarker von dem zweiten Nachweismarker verschieden ist.

10. Verwendung eines Kits zur Durchführung des Verfahrens gemäß einem der Ansprüche 1 bis 6, wobei der Kit Nucleinsäuren umfasst, die spezifisch an ein oder mehrere Hepatitisviren binden, und weiterhin Liganden umfasst, die an Moleküle binden, welche in azellulärer Form in einer biologischen Flüssigkeit enthalten sind, wobei die Liganden in dem Kit in einem Kombinationspräparat für eine gleichzeitige Verwendung enthalten sind, wobei die Liganden aus
- einem ersten Protein, das ein Protein ist, das spezifisch an HA bindet und ein Antikörper ist, oder dem rekombinanten humanen Protein Aggrecan, der bzw. das einen ersten Nachweismarker trägt; und
- einem zweiten Protein, das ein Protein ist, das spezifisch an das Protein CXCL10 bindet, ein Antikörper ist und einen zweiten Nachweismarker trägt;
wobei der erste Nachweismarker von dem zweiten Nachweismarker verschieden ist.

11. Verwendung gemäß Anspruch 10, wobei der Kit weiterhin das Fertigungsprodukt gemäß Anspruch 7 oder 8 umfasst.

12. Verwendung eines Computerprogrammprodukts zur Durchführung des Verfahrens gemäß einem der Ansprüche 1 bis 6, wobei das Computerprogrammprodukt zur Speicherung in einem Speicher einer Behandlungseinheit oder auf einem unbeweglichen Speichermedium, das mit einem Lesegerät der Behandlungseinheit zusammenwirken soll, vorgesehen ist, **dadurch gekennzeichnet, dass** es Anweisungen für die Ausführung eines Verfahrens gemäß einem der Ansprüche 1 bis 6 umfasst.

## Claims

1. An in vitro method for determining whether or not the liver fibrosis state of a subject infected by one or more hepatitis viruses has exceeded the one of liver fibrosis states that is of score F1 according to the Metavir fibrosis scores system, said method comprising the following steps:
i) selecting different biological markers, said selected different biological markers consisting of:
a) hyaluronic acid and the protein CXCL10, and
b) zero, one, two, three or four additional marker(s) selected from the list of markers consisting of the age, the body mass index, the viral load, and the stiffness of the liver,
ii) quantifying the different biological markers selected in step i) by measuring in vitro the concentration of hyaluronic acid and the concentration of the protein CXCL10 in a sample of biological fluid obtained in advance from the subject, and
when one, two, three or four additional marker(s) are selected from the list of step i)b) above, and when such additional marker(s) are or comprise one or more markers among the age, the body mass index and the stiffness of the liver: by collecting the quantification value of the or each of these additional marker(s), which was determined in advance for or on said subject,
when one, two, three or four additional marker(s) are selected from the list of step i)b) above, and when such additional marker(s) are or comprise the viral load: by measuring this viral load in vitro in a sample of biological fluid obtained in advance from the subject, or by collecting the value of this viral load, which was determined in advance for said subject, and
iii) comparing the quantification values obtained in step ii) to their values or to the distribution of their values in reference cohorts established in advance according to the liver fibrosis state, to classify said subject into the one of these reference cohorts to which he has the highest probability of belonging, said reference cohorts being:
a first reference cohort in which the liver fibrosis state of the individuals does not exceed beyond the one of the liver fibrosis states that is of score F1 according to the Metavir fibrosis scores system, and
a second reference cohort in which the liver fibrosis state of the individuals exceeds beyond the one of the liver fibrosis states that is of score F1 according to the Metavir fibrosis scores system,
being classified into said first cohort indicating that the liver fibrosis state of said subject has not exceeded beyond the one of the liver fibrosis states that is of score F1 according to the Metavir fibrosis scores system,
being classified into said second cohort indicating that the liver fibrosis state of said subject has exceeded beyond the one of the liver fibrosis states that is of score F1 according to the Metavir fibrosis scores system,
wherein said comparison of step iii) is effected:
by automatic learning, or by logistic regression, or by mROC, and wherein said comparison of step iii) is effected:
by automatic learning by following the decision tree of Figure 12 with 40 ≤ h ≤ 80, 150 ≤ i ≤ 300, and 400 ≤ j ≤ 620, or
by logistic regression using the function LOGIT1, said function LOGIT1 being: $LOGIT = Intercept + k \left(CXCL10\right) + I \left(HA\right) ,$ with $- 5 \leq Intercept \leq - 1 ,$ $0.001 \leq k \leq 0.010 ,$ and $0.010 \leq l \leq 0.050 ;$ or
by mROC using the function Z13, said function Z13 being: $Z = a \left(CXCL10t\right) + b \left(HAt\right) + c \left(IMCt\right) + d \left(aget\right) + e \left(CVt\right) + f \left(FSt\right) ,$ with
a and b each being, independently of one another, a positive real number from +0.1 to +6.0, but different from zero,
c, d, e and f each being, independently of one another, a real number from -10.0 to +10.0;
the stated exponent t indicating that the value to be applied in the function is the Box-Cox-transformed measured value for the marker in question (BMQ), in order to normalize this measured value according to the following formula: BMQt = (BMQλ - 1)/λ, and
the value of λ for each of the markers CXCL10 (λCXCL10), HA (λHA), IMC (λIMC), age (λage), CV (λCV) and FS (λFS) each being, independently of one another, a real number from -6.0 to 1.2, but different from zero, and
wherein the comparison of step iii) is effected by combining the quantification values obtained for said subject in a classification model constructed in advance as follows:
α) for a population of individuals being of the same species as said subject, and being infected by the same hepatitis virus or viruses as said subject, determining the liver fibrosis state of each of these individuals of the population, and classifying them into subpopulations according to their liver fibrosis state, thus forming reference cohorts established according to their liver fibrosis state, said reference cohorts comprising or being:
a first reference cohort in which the liver fibrosis state of the individuals does not exceed beyond the one of the liver fibrosis states that is of score F1 according to the Metavir fibrosis scores system, and
a second reference cohort in which the liver fibrosis state of the individuals exceeds beyond the one of the liver fibrosis states that is of score F1 according to the Metavir fibrosis scores system,
β) for each of said individuals, quantifying the different biological markers selected in step i), and
γ) performing an inter-cohort comparison of the quantification values obtained in step β), or of the distribution of such values, to construct a classification model, which induces a classification into one of said reference cohorts from the quantification values of said selected biological markers.

2. The method according to claim 1, wherein said different biological markers selected in step i) consist of:
a) hyaluronic acid and the protein CXCL10, and
b) zero, one, two or three additional marker(s) selected from the list of markers consisting of the age, the body mass index, the viral load, and the stiffness of the liver.

3. The method according to claim 1 or 2, wherein said different biological markers selected in step i) consist of:
- hyaluronic acid and the protein CXCL10, or
- hyaluronic acid, the protein CXCL10, the age, and the body mass index, or
- hyaluronic acid, the protein CXCL10, the age, the body mass index, and the viral load, or
- hyaluronic acid, the protein CXCL10, and the stiffness of the liver.

4. The method according to any of claims 1 to 3, wherein said comparison of step iii) of claim 1 is effected by classifying said subject into the one of these reference cohorts to which he has the highest probability of belonging with a sensitivity of at least 75% and/or with a negative prediction value of at least 75%.

5. The method according to any of claims 1 to 4, wherein said comparison of step iii) of claim 1 is effected by classifying said subject into the one of these reference cohorts to which he has the highest probability of belonging with at least one of the two performances 1/ and 2/ below:
1/ a specificity of at least 85% and/or a positive prediction value of at least 85%,
2/ a good classification rate of at least 80% and/or an area under the ROC curve of at least 0.800.

6. The method according to any of claims 1 to 5, wherein the measurement of the concentration of hyaluronic acid and the measurement of the concentration of the protein CXCL10 in step ii) of claim 1 are performed in multiplex mode.

7. Use of a fabricated article for performing the method according to any of claims 1 to 6, wherein said fabricated article is adapted to the detection in multiplex mode of molecules contained in an acellular form in a sample of biological fluid, and comprises a solid support on which ligands of said molecules are attached, wherein the ligands of said molecules are represented by:
- a first protein, which is a protein that binds specifically to HA and which is an antibody, or the recombinant human aggrecan protein, and
- a second protein, which is a protein that binds specifically to the protein CXCL10 and which is an antibody.

8. The use according to claim 7, wherein said solid support is a multiwell plate or microplate, a silicon chip, a glass capillary, a slide, magnetic beads, a membrane.

9. Use of a composition for performing the method according to any of claims 1 to 6, wherein said composition is adapted to the detection in multiplex mode of molecules contained in an acellular form in a sample of biological fluid, and comprises ligands of said molecules in admixture, wherein the ligands of said molecules are represented by:
- a first protein, which is a protein that binds specifically to HA and which is an antibody, or the recombinant human aggrecan protein, and which bears a first detection marker, and
- a second protein, which is a protein that binds specifically to the protein CXCL10 and which is an antibody, and which bears a second detection marker,
wherein said first detection marker is different from said second detection marker.

10. Use of a kit for performing the method according to any of claims 1 to 6, wherein said kit comprises nucleic acids that bind specifically to a hepatitis virus or viruses, further comprising ligands that bind to molecules contained in an acellular form in a biological fluid, said ligands being contained in the kit in a combined preparation for simultaneous use, said ligands being represented by:
- a first protein, which is a protein that binds specifically to HA and which is an antibody, or the recombinant human aggrecan protein, and which bears a first detection marker, and
- a second protein, which is a protein that binds specifically to the protein CXCL10 and which is an antibody, and which bears a second detection marker,
wherein said first detection marker is different from said second detection marker.

11. The use according to claim 10, wherein said kit further comprises the fabricated article according to claim 7 or 8.

12. Use of a computer program product for performing the method according to any of claims 1 to 6, wherein said computer program product is designated to be stored in a memory of a treatment unit, or on an immobile storage medium designated to cooperate with a reader of said treatment unit, **characterized in that** it comprises instructions for performing a method according to any of claims 1 to 6.
